# EUROPEAN PATENT APPLICATION

(11) **EP 3 915 576 A1**
(43) Date of publication of application: **01.12.2021**
(21) Application number: 20382457.8
(22) Date of filing: 28.05.2020
(51) Int. Cl.: A61K 39/00, A61P 35/00, C07K 14/725, C07K 14/705, C07K 16/32

(54) **CHIMERIC ANTIGEN RECEPTORS SPECIFIC FOR P95HER2 AND USES THEREOF**

(71) Applicant: Fundació Privada Institut d'Investigació Oncològica de Vall-Hebron, 08035 Barcelona (ES); Institució Catalana de Recerca i Estudis Avançats, 08010 Barcelona (ES)
(72) Inventor: ARRIBAS LÓPEZ, Joaquín, E-08035 Barcelona (ES); RIUS RUIZ, Irene, E-08035 Barcelona (ES); ROMÁN ALONSO, Macarena, E-08035 Barcelona (ES); GROS VIDAL, Alena, E-08035 Barcelona (ES); FAJARDO CALDERÓN, Carlos Alberto, E-08035 Barcelona (ES); KLEIN, Christian, 8952 Schlieren (CH); MOESSNER, Ekkehard, 8952 Schlieren (CH)
(74) Representative: ABG Intellectual Property Law, S.L.

(57) **Abstract**

The present invention relates to a chimeric antigen receptor (CAR) capable of targeting p95HER2-expressing cells. The invention also relates to a single-chain variable fragment (ScFv), to an antigen-binding domain and to an antibody or antibody fragment thereof capable of binding to the p95HER2 antigen. In addition, the present invention relates to an antibody-drug conjugate (ADC) comprising a therapeutic agent and an antibody or antigen-binding domain specific for p95HER2. The invention also relates to a method of cancer diagnosis and to a pharmaceutical composition for use in a method of preventing or treating cancer comprising the CAR and/or the antigen-binding domain or the antibody of the invention.

SEQUENCE LISTING

<110>  FUNDACIÓ PRIVADA INSTITUT D'INVESTIGACIÓ ONCOLÒGICA DE VALL
        HEBRON
        INSTITUCIÓ CATALANA DE RECERCA I ESTUDIS AVANÇATS

<120>  CHIMERIC ANTIGEN RECEPTORS SPECIFIC FOR P95HER2 AND USES THEREOF

<130>  P18520EP00

<160>  195

<170>  PatentIn version 3.5

<210>  1
<211>  5
<212>  PRT
<213>  Artificial Sequence

<220>
<223>  CDR1 VH m32H2

<400>  1

Asp Phe Gly Met Ser
1               5


<210>  2
<211>  17
<212>  PRT
<213>  Artificial Sequence

<220>
<223>  CDR2 VH m32H2

<400>  2

Thr Ile Asn Thr Asn Gly Gly Thr Thr His Tyr Pro Asp Asn Val Lys
1               5                   10                  15


Gly




<210>  3
<211>  5
<212>  PRT
<213>  Artificial Sequence

<220>
<223>  CDR3 VH m32H2

<400>  3

Glu Gly Leu Asp Tyr
1               5


<210>  4
<211>  11
<212>  PRT

<213>  Artificial Sequence

<220>
<223>  CDR1 VL m32H2

<400>  4

Lys Ala Ser Gln Ser Val Gly Thr Ala Val Ala
1               5                   10


<210>  5
<211>  7
<212>  PRT
<213>  Artificial Sequence

<220>
<223>  CDR2 VL m32H2

<400>  5

Ser Ala Ser Asn Arg Phe Thr
1               5


<210>  6
<211>  9
<212>  PRT
<213>  Artificial Sequence

<220>
<223>  CDR3 VL m32H2

<400>  6

Gln Gln Tyr Ser Thr Tyr Pro Leu Ala
1               5


<210>  7
<211>  5
<212>  PRT
<213>  Artificial Sequence

<220>
<223>  CDR1 VH 214D8

<400>  7

Thr Tyr Gly Met Ala
1               5


<210>  8
<211>  17
<212>  PRT
<213>  Artificial Sequence

<220>
<223>  CDR2 VH 214D8

<400>  8

Thr Ile Asn Ser Asn Gly Gly Lys Thr Tyr His Pro Asp Ser Val Lys
1               5                   10                  15

Gly

<210>  9
<211>  5
<212>  PRT
<213>  Artificial Sequence

<220>
<223>  CDR3 VH 214D8

<400>  9

Glu Gly Phe Asp Tyr
1               5

<210>  10
<211>  11
<212>  PRT
<213>  Artificial Sequence

<220>
<223>  CDR1 VL 214D8

<400>  10

Lys Ala Ser Gln Asn Val Gly Thr Ala Val Ala
1               5                   10

<210>  11
<211>  7
<212>  PRT
<213>  Artificial Sequence

<220>
<223>  CDR2 VL 214VL

<400>  11

Ser Ala Ser Asn Arg Tyr Thr
1               5

<210>  12
<211>  9
<212>  PRT
<213>  Artificial Sequence

<220>
<223>  CDR3 VL 214D8

<400>  12

Gln Gln Tyr Ser Thr Tyr Pro Leu Thr
1               5

<210>  13
<211>  5
<212>  PRT
<213>  Artificial Sequence

<220>
<223>  CDR1 VH 215C2

<400>  13

Asp Tyr Gly Met Ser
1               5


<210>  14
<211>  17
<212>  PRT
<213>  Artificial Sequence

<220>
<223>  CDR2 VH 215C2

<400>  14

Thr Ile Asn Gly Asn Gly Val Lys Ile Tyr Tyr Val Asp Ser Val Lys
1               5                   10                  15


Gly




<210>  15
<211>  5
<212>  PRT
<213>  Artificial Sequence

<220>
<223>  CDR3 VH 215C2

<400>  15

Glu Gly Phe Asp Tyr
1               5


<210>  16
<211>  11
<212>  PRT
<213>  Artificial Sequence

<220>
<223>  CDR1 VL 215C2

<400>  16

Lys Ala Ser Gln Asn Val Gly Thr Ala Val Ala
1               5                   10


<210>  17
<211>  7

<212>  PRT
<213>  Artificial Sequence

<220>
<223>  CDR2 VL 215C2

<400>  17

Ser Ala Ser Asn Arg Tyr Thr
1               5


<210>  18
<211>  9
<212>  PRT
<213>  Artificial Sequence

<220>
<223>  CDR3 VL 215C2

<400>  18

Gln Gln Tyr Ser Ser Tyr Pro Leu Thr
1               5


<210>  19
<211>  30
<212>  PRT
<213>  Artificial Sequence

<220>
<223>  FR1 VH  H1-32H2

<400>  19

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Asn
            20                  25                  30


<210>  20
<211>  14
<212>  PRT
<213>  Artificial Sequence

<220>
<223>  FR2 VH  H1-32H2

<400>  20

Trp Val Arg Gln Ala Pro Gly Lys Gly Leu Glu Trp Val Ser
1               5                   10


<210>  21
<211>  32
<212>  PRT
<213>  Artificial Sequence

<220>
<223>  FR3 VH  H1-32H2

<400>  21

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Ala Lys
            20                  25                  30

<210>  22
<211>  11
<212>  PRT
<213>  Artificial Sequence

<220>
<223>  FR4 VH  H1-32H2

<400>  22

Trp Gly Gln Gly Thr Leu Val Thr Val Ser Ser
1               5                   10

<210>  23
<211>  23
<212>  PRT
<213>  Artificial Sequence

<220>
<223>  FR1 VL  H1-32H2

<400>  23

Glu Ile Val Leu Thr Gln Ser Pro Ala Thr Leu Ser Leu Ser Pro Gly
1               5                   10                  15

Glu Arg Ala Thr Leu Ser Cys
            20

<210>  24
<211>  15
<212>  PRT
<213>  Artificial Sequence

<220>
<223>  FR2 VH  H1-32H2

<400>  24

Trp Tyr Gln Gln Lys Pro Gly Gln Ala Pro Arg Leu Leu Ile Tyr
1               5                   10                  15

<210>  25
<211>  32
<212>  PRT
<213>  Artificial Sequence

<220>
<223>  FR3 VL  H1-32H2

<400>  25

Gly Ile Pro Ala Arg Phe Ser Gly Ser Gly Ser Gly Thr Asp Phe Thr
1               5                   10                  15

Leu Thr Ile Ser Ser Leu Glu Pro Glu Asp Phe Ala Val Tyr Tyr Cys
            20                  25                  30

<210>  26
<211>  10
<212>  PRT
<213>  Artificial Sequence

<220>
<223>  FR4 VL  H1-32H2

<400>  26

Phe Gly Gln Gly Thr Lys Leu Glu Ile Lys
1               5                   10

<210>  27
<211>  107
<212>  PRT
<213>  Artificial Sequence

<220>
<223>  VL  (FRs H1-32H2 and CDRs m32H2)

<400>  27

Glu Ile Val Leu Thr Gln Ser Pro Ala Thr Leu Ser Leu Ser Pro Gly
1               5                   10                  15

Glu Arg Ala Thr Leu Ser Cys Lys Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys

100                 105

<210>  28
<211>  114
<212>  PRT
<213>  Artificial Sequence

<220>
<223>  VH  (FRs H1 32H2 and CDRs m32H2)

<400>  28

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Gly Met Ser Trp Val Arg Gln Ala Pro Gly Lys Gly Leu Glu Trp Val
        35                  40                  45

Ser Thr Ile Asn Thr Asn Gly Gly Thr Thr His Tyr Pro Asp Asn Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Glu Gly Leu Asp Tyr Trp Gly Gln Gly Thr Leu Val Thr Val
            100                 105                 110

Ser Ser

<210>  29
<211>  16
<212>  PRT
<213>  Artificial Sequence

<220>
<223>  linker coneting VH and VL regions

<400>  29

Thr Gly Ser Thr Ser Gly Ser Gly Lys Pro Gly Ser Gly Glu Gly Ser
1               5                   10                  15

<210>  30
<211>  237
<212>  PRT
<213>  Artificial Sequence

<220>
<223>  VL-VH (CDRs m32H2 and FRs H1 32H2)

<400>  30

Glu Ile Val Leu Thr Gln Ser Pro Ala Thr Leu Ser Leu Ser Pro Gly
1               5                   10                  15

Glu Arg Ala Thr Leu Ser Cys Lys Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Asn Asp Phe Gly Met Ser Trp Val
145                 150                 155                 160

Arg Gln Ala Pro Gly Lys Gly Leu Glu Trp Val Ser Thr Ile Asn Thr
                165                 170                 175

Asn Gly Gly Thr Thr His Tyr Pro Asp Asn Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Ala Lys Glu Gly Leu
    210                 215                 220

Asp Tyr Trp Gly Gln Gly Thr Leu Val Thr Val Ser Ser

225                 230                 235

<210>  31
<211>  30
<212>  PRT
<213>  Artificial Sequence

<220>
<223>  FR1 VH 214D8

<400>  31

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser
            20                  25                  30

<210>  32
<211>  14
<212>  PRT
<213>  Artificial Sequence

<220>
<223>  FR2 VH 214D8

<400>  32

Trp Val Arg Gln Thr Pro Asp Arg Arg Leu Glu Leu Val Ala
1               5                   10

<210>  33
<211>  32
<212>  PRT
<213>  Artificial Sequence

<220>
<223>  FR3 VH 214D8

<400>  33

Arg Phe Thr Ile Ser Arg Asp Asn Ala Lys Asn Thr Leu Tyr Leu Gln
1               5                   10                  15

Met Ser Ser Leu Lys Ser Glu Asp Thr Ala Met Tyr Tyr Cys Thr Arg
            20                  25                  30

<210>  34
<211>  11
<212>  PRT
<213>  Artificial Sequence

<220>
<223>  FR4 VH 214D8

<400>  34

Trp Gly Gln Gly Thr Thr Leu Thr Val Ser Ser
1               5                   10

<210>  35
<211>  23
<212>  PRT
<213>  Artificial Sequence

<220>
<223>  FR1 VL 214D8

<400>  35

Asp Ile Val Met Thr Gln Ser Gln Lys Phe Met Ser Thr Ser Val Gly
1               5                   10                  15

Asp Arg Val Ser Ile Thr Cys
            20

<210>  36
<211>  15
<212>  PRT
<213>  Artificial Sequence

<220>
<223>  FR2 VL 214D8

<400>  36

Trp Tyr Gln Gln Lys Pro Gly Gln Ser Pro Lys Ile Leu Ile Tyr
1               5                   10                  15

<210>  37
<211>  32
<212>  PRT
<213>  Artificial Sequence

<220>
<223>  FR3 VL 214C8

<400>  37

Gly Val Pro Asp Arg Phe Thr Gly Ser Gly Ser Gly Thr Asp Phe Thr
1               5                   10                  15

Leu Thr Ile Ser Asn Met Lys Ser Glu Asp Leu Ala Asp Tyr Phe Cys
            20                  25                  30

<210>  38
<211>  10
<212>  PRT
<213>  Artificial Sequence

<220>
<223>  FR4 VL 214D8

<400>  38

Phe Gly Ala Gly Thr Lys Leu Glu Leu Lys
1               5                   10

<210>  39
<211>  107
<212>  PRT
<213>  Artificial Sequence

<220>
<223>  VL 214D8

<400>  39

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Ile Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Lys Ser
65                  70                  75                  80

Glu Asp Leu Ala Asp Tyr Phe Cys Gln Gln Tyr Ser Thr Tyr Pro Leu
                85                  90                  95

Thr Phe Gly Ala Gly Thr Lys Leu Glu Leu Lys
            100                 105

<210>  40
<211>  114
<212>  PRT
<213>  Artificial Sequence

<220>
<223>  VH 214D8

<400>  40

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Thr Tyr
            20                  25                  30

Gly Met Ala Trp Val Arg Gln Thr Pro Asp Arg Arg Leu Glu Leu Val
        35                  40                  45

Ala Thr Ile Asn Ser Asn Gly Gly Lys Thr Tyr His Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Thr Arg Glu Gly Phe Asp Tyr Trp Gly Gln Gly Thr Thr Leu Thr Val
            100                 105                 110

Ser Ser

<210>  41
<211>  237
<212>  PRT
<213>  Artificial Sequence

<220>
<223>  214D8  VL-VH

<400>  41

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Ile Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Lys Ser
65                  70                  75                  80

Glu Asp Leu Ala Asp Tyr Phe Cys Gln Gln Tyr Ser Thr Tyr Pro Leu
                85                  90                  95

Thr Phe Gly Ala Gly Thr Lys Leu Glu Leu Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Lys Leu Ser
    130                 135                 140

Cys Ala Ala Ser Gly Phe Thr Phe Ser Thr Tyr Gly Met Ala Trp Val
145                 150                 155                 160

Arg Gln Thr Pro Asp Arg Arg Leu Glu Leu Val Ala Thr Ile Asn Ser
                165                 170                 175

Asn Gly Gly Lys Thr Tyr His Pro Asp Ser Val Lys Gly Arg Phe Thr
            180                 185                 190

Ile Ser Arg Asp Asn Ala Lys Asn Thr Leu Tyr Leu Gln Met Ser Ser
        195                 200                 205

Leu Lys Ser Glu Asp Thr Ala Met Tyr Tyr Cys Thr Arg Glu Gly Phe
    210                 215                 220

Asp Tyr Trp Gly Gln Gly Thr Thr Leu Thr Val Ser Ser
225                 230                 235

<210>  42
<211>  30
<212>  PRT
<213>  Artificial Sequence

<220>
<223>  FR1 VH 215C2

<400>  42

Glu Val His Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser
            20                  25                  30

<210>  43
<211>  14
<212>  PRT
<213>  Artificial Sequence

<220>
<223>  FR2 VH 215C2

<400>  43

Trp Val Arg Gln Thr Pro Asp Lys Arg Leu Glu Leu Val Ala
1               5                   10

<210>  44
<211>  32

<212>  PRT
<213>  Artificial Sequence

<220>
<223>  FR3 VH 215C2

<400>  44

Arg Phe Thr Ile Ser Arg Asp Asn Ala Lys Asn Thr Leu Tyr Leu Gln
1               5                   10                  15

Met Asn Ser Leu Lys Ser Glu Asp Thr Ala Ile Tyr Tyr Cys Pro Arg
            20                  25                  30

<210>  45
<211>  11
<212>  PRT
<213>  Artificial Sequence

<220>
<223>  FR4 VH 215C2

<400>  45

Trp Gly Gln Gly Thr Thr Leu Thr Val Ser Ser
1               5                   10

<210>  46
<211>  23
<212>  PRT
<213>  Artificial Sequence

<220>
<223>  FR1 VL 215C2

<400>  46

Asp Ile Val Met Thr Gln Ser Gln Lys Phe Met Ser Thr Ser Glu Gly
1               5                   10                  15

Asp Arg Val Ser Ile Thr Cys
            20

<210>  47
<211>  15
<212>  PRT
<213>  Artificial Sequence

<220>
<223>  FR2 VL 215C2

<400>  47

Trp Tyr Gln Gln Lys Pro Gly Gln Ser Pro Lys Val Leu Ile Tyr
1               5                   10                  15

<210>  48

<211>  32
<212>  PRT
<213>  Artificial Sequence

<220>
<223>  FR3 VL 215C2

<400>  48

Gly Val Pro Asp Arg Phe Thr Gly Ser Gly Ser Gly Thr Asp Phe Thr
1               5                   10                  15

Leu Thr Ile Ser Asn Met Gln Ser Glu Asp Leu Ala Asp Tyr Phe Cys
            20                  25                  30


<210>  49
<211>  10
<212>  PRT
<213>  Artificial Sequence

<220>
<223>  FR4 VL 215C2

<400>  49

Phe Gly Ala Gly Thr Thr Leu Glu Leu Lys
1               5                   10


<210>  50
<211>  107
<212>  PRT
<213>  Artificial Sequence

<220>
<223>  VL 215C2

<400>  50

Asp Ile Val Met Thr Gln Ser Gln Lys Phe Met Ser Thr Ser Glu Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys
            100                 105

<210> 51
<211> 114
<212> PRT
<213> Artificial Sequence

<220>
<223> VH 215C2

<400> 51

Glu Val His Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Asp Tyr
            20                  25                  30

Gly Met Ser Trp Val Arg Gln Thr Pro Asp Lys Arg Leu Glu Leu Val
        35                  40                  45

Ala Thr Ile Asn Gly Asn Gly Val Lys Ile Tyr Tyr Val Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Ile Tyr Tyr Cys
                85                  90                  95

Pro Arg Glu Gly Phe Asp Tyr Trp Gly Gln Gly Thr Thr Leu Thr Val
            100                 105                 110

Ser Ser

<210> 52
<211> 237
<212> PRT
<213> Artificial Sequence

<220>
<223> 215C2 VL-VH

<400> 52

Asp Ile Val Met Thr Gln Ser Gln Lys Phe Met Ser Thr Ser Glu Gly
1               5                   10                  15

Asp Arg Val Ser Ile Thr Cys Lys Ala Ser Gln Asn Val Gly Thr Ala

            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys Thr Gly Ser Thr Ser
            100                 105                 110

Gly Ser Gly Lys Pro Gly Ser Gly Glu Gly Ser Glu Val His Leu Val
        115                 120                 125

Glu Ser Gly Gly Gly Leu Val Gln Pro Gly Gly Ser Leu Lys Leu Ser
    130                 135                 140

Cys Ala Ala Ser Gly Phe Thr Phe Ser Asp Tyr Gly Met Ser Trp Val
145                 150                 155                 160

Arg Gln Thr Pro Asp Lys Arg Leu Glu Leu Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ala Lys Asn Thr Leu Tyr Leu Gln Met Asn Ser
        195                 200                 205

Leu Lys Ser Glu Asp Thr Ala Ile Tyr Tyr Cys Pro Arg Glu Gly Phe
    210                 215                 220

Asp Tyr Trp Gly Gln Gly Thr Thr Leu Thr Val Ser Ser
225                 230                 235

<210> 53
<211> 114
<212> PRT
<213> Artificial Sequence

<220>
<223> VH H1-214D8

<400>  53

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Gly Met Ala Trp Val Arg Gln Thr Pro Asp Arg Arg Leu Glu Leu Val
        35                  40                  45

Ala Thr Ile Asn Ser Asn Gly Gly Lys Thr Tyr His Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Glu Gly Phe Asp Tyr Trp Gly Gln Gly Thr Leu Val Thr Val
            100                 105                 110

Ser Ser

<210>  54
<211>  107
<212>  PRT
<213>  Artificial Sequence

<220>
<223>  VL  H1-214D8

<400>  54

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys
            100                 105

<210>  55
<211>  114
<212>  PRT
<213>  Artificial Sequence

<220>
<223>  VH  H2-214D8

<400>  55

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Gly Met Ala Trp Val Arg Gln Ala Pro Gly Lys Gly Leu Glu Trp Val
        35                  40                  45

Ser Thr Ile Asn Ser Asn Gly Gly Lys Thr Tyr His Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Glu Gly Phe Asp Tyr Trp Gly Gln Gly Thr Leu Val Thr Val
            100                 105                 110

Ser Ser

<210>  56
<211>  107
<212>  PRT
<213>  Artificial Sequence

<220>
<223>  VL  H2-214D8

<400>  56

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys
            100                 105

<210>  57
<211>  111
<212>  PRT
<213>  Artificial Sequence

<220>
<223>  VH  H3-214D8

<400>  57

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met Ala Trp Val Arg Gln Ala Pro Gly Lys Gly Leu Glu Trp Val
        35                  40                  45

Ser Ala Ile Asn Ser Asn Gly Gly Lys Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Tyr Trp Gly Gln Gly Thr Leu Val Thr Val Ser Ser
            100                 105                 110

<210>  58
<211>  107
<212>  PRT
<213>  Artificial Sequence

<220>
<223>  VL  H3-214D8

<400>  58

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys
            100                 105


<210>  59
<211>  114
<212>  PRT
<213>  Artificial Sequence

<220>
<223>  VH  H1-215C2

<400>  59

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Met Ser Trp Val Arg Gln Ala Pro Gly Lys Arg Leu Glu Leu Val
        35                  40                  45

Ala Thr Ile Asn Gly Asn Gly Val Lys Ile Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg Glu Gly Phe Asp Tyr Trp Gly Gln Gly Thr Leu Val Thr Val
            100                 105                 110

Ser Ser

<210>  60
<211>  107
<212>  PRT
<213>  Artificial Sequence

<220>
<223>  VL  H1-215C2

<400>  60

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Val Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys
            100                 105

<210>  61
<211>  114
<212>  PRT
<213>  Artificial Sequence

<220>
<223>  VH  H2-215C2

<400>  61

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Met Ser Trp Val Arg Gln Ala Pro Gly Lys Gly Leu Glu Trp Val
        35                  40                  45

Ala Thr Ile Asn Gly Asn Gly Val Lys Ile Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg Glu Gly Phe Asp Tyr Trp Gly Gln Gly Thr Leu Val Thr Val
            100                 105                 110

Ser Ser

<210>  62
<211>  107
<212>  PRT
<213>  Artificial Sequence

<220>
<223>  VL  H2-215C2

<400>  62

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala
            20                  25                  30

Val Ala Trp Phe Gln Gln Lys Pro Gly Lys Ala Pro Lys Ser Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys
            100                 105

<210>  63
<211>  98
<212>  PRT
<213>  Artificial Sequence

<220>
<223>  VH  H3-215C2

<400>  63

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Met Ser Trp Val Arg Gln Ala Pro Gly Lys Gly Leu Glu Trp Val
        35                  40                  45

Ala Thr Ile Asn Gly Asn Gly Val Lys Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg

<210>  64
<211>  107
<212>  PRT
<213>  Artificial Sequence

<220>
<223>  VL  H3-215C2

<400>  64

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala

20                  25                  30

Val Ala Trp Phe Gln Gln Lys Pro Gly Lys Ala Pro Lys Ser Leu Ile
        35                  40                  45

Tyr Ser Ala Ser Asn Arg Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys
            100                 105

<210>  65
<211>  30
<212>  PRT
<213>  Artificial Sequence

<220>
<223>  FR1 VH  H1-214D8

<400>  65

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  66
<211>  14
<212>  PRT
<213>  Artificial Sequence

<220>
<223>  FR2 VH  H1-214D8

<400>  66

Trp Val Arg Gln Thr Pro Asp Arg Arg Leu Glu Leu Val Ala
1               5                   10

<210>  67
<211>  32
<212>  PRT
<213>  Artificial Sequence

<220>
<223>  FR3 VH  H1-214D8

<400>  67

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Thr Arg
            20                  25                  30

<210>  68
<211>  11
<212>  PRT
<213>  Artificial Sequence

<220>
<223>  FR4 VH  H1-214D8

<400>  68

Trp Gly Gln Gly Thr Leu Val Thr Val Ser Ser
1               5                   10

<210>  69
<211>  23
<212>  PRT
<213>  Artificial Sequence

<220>
<223>  FR1 VL  H1-214D8

<400>  69

Asp Ile Gln Met Thr Gln Ser Pro Ser Ser Leu Ser Ala Ser Val Gly
1               5                   10                  15

Asp Arg Val Thr Ile Thr Cys
            20

<210>  70
<211>  15
<212>  PRT
<213>  Artificial Sequence

<220>
<223>  FR2 VL  H1-214D8

<400>  70

Trp Phe Gln Gln Lys Pro Gly Lys Ala Pro Lys Ile Leu Ile Tyr
1               5                   10                  15

<210>  71
<211>  32
<212>  PRT
<213>  Artificial Sequence

<220>
<223>  FR3 VL  H1-214D8

<400>  71

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  72
<211>  10
<212>  PRT
<213>  Artificial Sequence

<220>
<223>  FR4 VL  H1-214D8

<400>  72

Phe Gly Gln Gly Thr Lys Leu Glu Ile Lys
1               5                   10

<210>  73
<211>  30
<212>  PRT
<213>  Artificial Sequence

<220>
<223>  FR1 VH  H2-214D8

<400>  73

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  74
<211>  14
<212>  PRT
<213>  Artificial Sequence

<220>
<223>  FR2 VH  H2-214D8

<400>  74

Trp Val Arg Gln Ala Pro Gly Lys Gly Leu Glu Trp Val Ser
1               5                   10

<210>  75
<211>  32
<212>  PRT
<213>  Artificial Sequence

<220>

<223>  FR3 VH  H2-214D8

<400>  75

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Thr Arg
            20                  25                  30

<210>  76
<211>  11
<212>  PRT
<213>  Artificial Sequence

<220>
<223>  FR4 VH  H2-214D8

<400>  76

Trp Gly Gln Gly Thr Leu Val Thr Val Ser Ser
1               5                   10

<210>  77
<211>  23
<212>  PRT
<213>  Artificial Sequence

<220>
<223>  FR1 VL  H2-214D8

<400>  77

Asp Ile Gln Met Thr Gln Ser Pro Ser Ser Leu Ser Ala Ser Val Gly
1               5                   10                  15

Asp Arg Val Thr Ile Thr Cys
            20

<210>  78
<211>  15
<212>  PRT
<213>  Artificial Sequence

<220>
<223>  FR2 VL  H2-214D8

<400>  78

Trp Phe Gln Gln Lys Pro Gly Lys Ala Pro Lys Ile Leu Ile Tyr
1               5                   10                  15

<210>  79
<211>  32
<212>  PRT
<213>  Artificial Sequence

<220>
<223>  FR3 VL  H2-214D8

<400>  79

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  80
<211>  10
<212>  PRT
<213>  Artificial Sequence

<220>
<223>  FR4 VL  H2-214D8

<400>  80

Phe Gly Gln Gly Thr Lys Leu Glu Ile Lys
1               5                   10

<210>  81
<211>  30
<212>  PRT
<213>  Artificial Sequence

<220>
<223>  FR1 VH  H3-214D8

<400>  81

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  82
<211>  14
<212>  PRT
<213>  Artificial Sequence

<220>
<223>  FR2 VH  H3-214D8

<400>  82

Trp Val Arg Gln Ala Pro Gly Lys Gly Leu Glu Trp Val Ser
1               5                   10

<210>  83
<211>  32
<212>  PRT
<213>  Artificial Sequence

<220>
<223>  FR3 VH  H3-214D8

<400>  83

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Ala Lys
            20                  25                  30

<210>  84
<211>  11
<212>  PRT
<213>  Artificial Sequence

<220>
<223>  FR4 VH  H3-214D8

<400>  84

Trp Gly Gln Gly Thr Leu Val Thr Val Ser Ser
1               5                   10

<210>  85
<211>  23
<212>  PRT
<213>  Artificial Sequence

<220>
<223>  FR1 VL  H3-214D8

<400>  85

Asp Ile Gln Met Thr Gln Ser Pro Ser Ser Leu Ser Ala Ser Val Gly
1               5                   10                  15

Asp Arg Val Thr Ile Thr Cys
            20

<210>  86
<211>  15
<212>  PRT
<213>  Artificial Sequence

<220>
<223>  FR2 VL  H3-214D8

<400>  86

Trp Phe Gln Gln Lys Pro Gly Lys Ala Pro Lys Ile Leu Ile Tyr
1               5                   10                  15

<210>  87
<211>  32
<212>  PRT

<213>  Artificial Sequence

<220>
<223>  FR3 VL  H3-214D8

<400>  87

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  88
<211>  10
<212>  PRT
<213>  Artificial Sequence

<220>
<223>  FR4 VL  H3-214D8

<400>  88

Phe Gly Gln Gly Thr Lys Leu Glu Ile Lys
1               5                   10

<210>  89
<211>  30
<212>  PRT
<213>  Artificial Sequence

<220>
<223>  FR1 VH  H1-215C2

<400>  89

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  90
<211>  14
<212>  PRT
<213>  Artificial Sequence

<220>
<223>  FR2 VH  H1-215C2

<400>  90

Trp Val Arg Gln Ala Pro Gly Lys Arg Leu Glu Leu Val Ala
1               5                   10

<210>  91
<211>  32

<212>  PRT
<213>  Artificial Sequence

<220>
<223>  FR3 VH  H1-215C2

<400>  91

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Pro Arg
            20                  25                  30

<210>  92
<211>  11
<212>  PRT
<213>  Artificial Sequence

<220>
<223>  FR4 VH  H1-215C2

<400>  92

Trp Gly Gln Gly Thr Leu Val Thr Val Ser Ser
1               5                   10

<210>  93
<211>  23
<212>  PRT
<213>  Artificial Sequence

<220>
<223>  FR1 VL  H1-215C2

<400>  93

Asp Ile Gln Met Thr Gln Ser Pro Ser Ser Leu Ser Ala Ser Val Gly
1               5                   10                  15

Asp Arg Val Thr Ile Thr Cys
            20

<210>  94
<211>  15
<212>  PRT
<213>  Artificial Sequence

<220>
<223>  FR2 VL  H1-215C2

<400>  94

Trp Phe Gln Gln Lys Pro Gly Lys Ala Pro Lys Val Leu Ile Tyr
1               5                   10                  15

<210>  95

<211>  32
<212>  PRT
<213>  Artificial Sequence

<220>
<223>  FR3 VL  H1-215C2

<400>  95

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  96
<211>  10
<212>  PRT
<213>  Artificial Sequence

<220>
<223>  FR4 VL  H1-215C2

<400>  96

Phe Gly Gln Gly Thr Lys Leu Glu Ile Lys
1               5                   10

<210>  97
<211>  30
<212>  PRT
<213>  Artificial Sequence

<220>
<223>  FR1 VH  H2-215C2

<400>  97

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  98
<211>  14
<212>  PRT
<213>  Artificial Sequence

<220>
<223>  FR2 VH  H2-215C2

<400>  98

Trp Val Arg Gln Ala Pro Gly Lys Gly Leu Glu Trp Val Ala
1               5                   10

<210>  99
<211>  32
<212>  PRT
<213>  Artificial Sequence

<220>
<223>  FR3 VH  H2-215C2

<400>  99

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Pro Arg
            20                  25                  30

<210>  100
<211>  11
<212>  PRT
<213>  Artificial Sequence

<220>
<223>  FR4 VH  H2-215C2

<400>  100

Trp Gly Gln Gly Thr Leu Val Thr Val Ser Ser
1               5                   10

<210>  101
<211>  23
<212>  PRT
<213>  Artificial Sequence

<220>
<223>  FR1 VL  H2-215C2

<400>  101

Asp Ile Gln Met Thr Gln Ser Pro Ser Ser Leu Ser Ala Ser Val Gly
1               5                   10                  15

Asp Arg Val Thr Ile Thr Cys
            20

<210>  102
<211>  15
<212>  PRT
<213>  Artificial Sequence

<220>
<223>  FR2 VL  H2-215C2

<400>  102

Trp Phe Gln Gln Lys Pro Gly Lys Ala Pro Lys Ser Leu Ile Tyr
1               5                   10                  15

<210>  103
<211>  32
<212>  PRT
<213>  Artificial Sequence

<220>
<223>  FR3 VL  H2-215C2

<400>  103

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  104
<211>  10
<212>  PRT
<213>  Artificial Sequence

<220>
<223>  FR4 VL  H2-215C2

<400>  104

Phe Gly Gln Gly Thr Lys Leu Glu Ile Lys
1               5                   10

<210>  105
<211>  30
<212>  PRT
<213>  Artificial Sequence

<220>
<223>  FR1 VH  H3-215C2

<400>  105

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  106
<211>  14
<212>  PRT
<213>  Artificial Sequence

<220>
<223>  FR2 VH  H3-215C2

<400>  106

Trp Val Arg Gln Ala Pro Gly Lys Gly Leu Glu Trp Val Ala
1               5                   10

<210>  107
<211>  32
<212>  PRT
<213>  Artificial Sequence

<220>
<223>  FR3 VH  H3-215C2

<400>  107

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Pro Arg
            20                  25                  30

<210>  108
<211>  11
<212>  PRT
<213>  Artificial Sequence

<220>
<223>  X

<400>  108

Trp Gly Gln Gly Thr Leu Val Thr Val Ser Ser
1               5                   10

<210>  109
<211>  23
<212>  PRT
<213>  Artificial Sequence

<220>
<223>  FR1 VL  H3-2015C2

<400>  109

Asp Ile Gln Met Thr Gln Ser Pro Ser Ser Leu Ser Ala Ser Val Gly
1               5                   10                  15

Asp Arg Val Thr Ile Thr Cys
            20

<210>  110
<211>  15
<212>  PRT
<213>  Artificial Sequence

<220>
<223>  FR2 VL  H3-215C2

<400>  110

Trp Phe Gln Gln Lys Pro Gly Lys Ala Pro Lys Ser Leu Ile Tyr

1                   5                   10                  15


<210>  111
<211>  32
<212>  PRT
<213>  Artificial Sequence

<220>
<223>  FR3 VL  H3-215C2

<400>  111

Gly Val Pro Ser Arg Phe Ser Gly Ser Gly Ser Gly Thr Asp Phe Thr
1               5                   10                  15


Leu Thr Ile Ser Ser Leu Gln Pro Glu Asp Phe Ala Thr Tyr Tyr Cys
            20                  25                  30


<210>  112
<211>  10
<212>  PRT
<213>  Artificial Sequence

<220>
<223>  FR4 VL  H3-215C2

<400>  112

Phe Gly Gln Gly Thr Lys Leu Glu Ile Lys
1               5                   10


<210>  113
<211>  29
<212>  PRT
<213>  Artificial Sequence

<220>
<223>  CD28 transmembrane domain

<400>  113

Phe Trp Val Leu Val Val Val Gly Gly Asx Cys Val Leu Ala Cys Tyr
1               5                   10                  15


Ser Leu Leu Val Thr Val Ala Phe Ile Ile Phe Trp Val
            20                  25


<210>  114
<211>  112
<212>  PRT
<213>  Artificial Sequence

<220>
<223>  CD3-zeta intracellular signalling domain

<400>  114

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  115
<211>  41
<212>  PRT
<213>  Artificial Sequence

<220>
<223>  intracellualr domain of the coestimulatory domain CD28

<400>  115

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  116
<211>  107
<212>  PRT
<213>  Artificial Sequence

<220>
<223>  Spacer

<400>  116

Gly Gln Pro Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ser Val Glu Trp Glu Ser Asn Gly Gln Pro Glu
        35                  40                  45

Asn Asn Tyr Lys Thr Thr Pro Pro Met Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210>  117
<211>  5
<212>  PRT
<213>  Artificial Sequence

<220>
<223>  linker

<400>  117

Gly Ser Gly Gly Ser
1               5

<210>  118
<211>  4
<212>  PRT
<213>  Artificial Sequence

<220>
<223>  linker

<400>  118

Gly Gly Gly Ser
1

<210>  119
<211>  4
<212>  PRT
<213>  Artificial Sequence

<220>
<223>  linker

<400>  119

Gly Gly Ser Gly
1

<210> 120
<211> 5
<212> PRT
<213> Artificial Sequence

<220>
<223> linker

<400> 120

Gly Gly Ser Gly Gly
1               5

<210> 121
<211> 5
<212> PRT
<213> Artificial Sequence

<220>
<223> linker

<400> 121

Gly Ser Gly Ser Gly
1               5

<210> 122
<211> 5
<212> PRT
<213> Artificial Sequence

<220>
<223> linker

<400> 122

Gly Ser Gly Gly Gly
1               5

<210> 123
<211> 5
<212> PRT
<213> Artificial Sequence

<220>
<223> linker

<400> 123

Gly Gly Gly Ser Gly
1               5

<210> 124
<211> 5
<212> PRT

<213>  Artificial Sequence

<220>
<223>  linker

<400>  124

Gly Ser Ser Ser Gly
1               5


<210>  125
<211>  5
<212>  PRT
<213>  Artificial Sequence

<220>
<223>  hinge region

<400>  125

Gly Ser Gly Gly Ser
1               5


<210>  126
<211>  4
<212>  PRT
<213>  Artificial Sequence

<220>
<223>  hinge region

<400>  126

Gly Gly Gly Ser
1


<210>  127
<211>  4
<212>  PRT
<213>  Artificial Sequence

<220>
<223>  hinge region

<400>  127

Gly Gly Ser Gly
1


<210>  128
<211>  5
<212>  PRT
<213>  Artificial Sequence

<220>
<223>  hinge region

<400>  128

Gly Gly Ser Gly Gly
1               5

<210>  129
<211>  5
<212>  PRT
<213>  Artificial Sequence

<220>
<223>  hinge region

<400>  129

Gly Ser Gly Ser Gly
1               5

<210>  130
<211>  5
<212>  PRT
<213>  Artificial Sequence

<220>
<223>  hinge region

<400>  130

Gly Ser Gly Gly Gly
1               5

<210>  131
<211>  5
<212>  PRT
<213>  Artificial Sequence

<220>
<223>  hinge region

<400>  131

Gly Gly Gly Ser Gly
1               5

<210>  132
<211>  5
<212>  PRT
<213>  Artificial Sequence

<220>
<223>  hinge region

<400>  132

Gly Ser Ser Ser Gly
1               5

<210>  133
<211>  4
<212>  PRT

<213>  Artificial Sequence

<220>
<223>  hinge region

<400>  133

Cys Pro Pro Cys
1


<210>  134
<211>  5
<212>  PRT
<213>  Artificial Sequence

<220>
<223>  hinge region

<400>  134

Asp Lys Thr His Thr
1               5


<210>  135

<400>  135
000

<210>  136
<211>  15
<212>  PRT
<213>  Artificial Sequence

<220>
<223>  hinge region

<400>  136

Cys Pro Glu Pro Lys Ser Cys Asp Thr Pro Pro Pro Cys Pro Arg
1               5                   10                  15


<210>  137
<211>  12
<212>  PRT
<213>  Artificial Sequence

<220>
<223>  hinge region

<400>  137

Glu Leu Lys Thr Pro Leu Gly Asp Thr Thr His Thr
1               5                   10


<210>  138
<211>  10
<212>  PRT
<213>  Artificial Sequence

<220>
<223>  hinge region

<400>  138

Lys Ser Cys Asp Lys Thr His Thr Cys Pro
1               5                   10


<210>  139
<211>  7
<212>  PRT
<213>  Artificial Sequence

<220>
<223>  hinge region

<400>  139

Lys Cys Cys Val Asp Cys Pro
1               5


<210>  140
<211>  7
<212>  PRT
<213>  Artificial Sequence

<220>
<223>  hinge region

<400>  140

Lys Tyr Gly Pro Pro Cys Pro
1               5


<210>  141
<211>  15
<212>  PRT
<213>  Artificial Sequence

<220>
<223>  hinge region

<400>  141

Glu Pro Lys Ser Cys Asp Lys Thr His Thr Cys Pro Pro Cys Pro
1               5                   10                  15


<210>  142
<211>  12
<212>  PRT
<213>  Artificial Sequence

<220>
<223>  hinge region

<400>  142

Glu Arg Lys Cys Cys Val Glu Cys Pro Pro Cys Pro
1               5                   10

<210>  143
<211>  17
<212>  PRT
<213>  Artificial Sequence

<220>
<223>  hinge region

<400>  143

Glu Leu Lys Thr Pro Leu Gly Asp Thr Thr His Thr Cys Pro Arg Cys
1               5                   10                  15

Pro

<210>  144
<211>  12
<212>  PRT
<213>  Artificial Sequence

<220>
<223>  hinge region

<400>  144

Ser Pro Asn Met Val Pro His Ala His His Ala Gln
1               5                   10

<210>  145
<211>  15
<212>  PRT
<213>  Artificial Sequence

<220>
<223>  hinge region

<400>  145

Glu Pro Lys Ser Cys Asp Lys Thr Tyr Thr Cys Pro Pro Cys Pro
1               5                   10                  15

<210>  146
<211>  45
<212>  PRT
<213>  Artificial Sequence

<220>
<223>  CD8 hinge region

<400>  146

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly

20                  25                  30

Gly Ala Val His Thr Arg Gly Leu Asp Phe Ala Cys Asp
        35                  40                  45

<210>  147
<211>  21
<212>  PRT
<213>  Artificial Sequence

<220>
<223>  leader sequence

<400>  147

Met Ala Leu Pro Val Thr Ala Leu Leu Leu Pro Leu Ala Leu Leu Leu
1               5                   10                  15

His Ala Ala Arg Pro
            20

<210>  148

<400>  148
000

<210>  149

<400>  149
000

<210>  150

<400>  150
000

<210>  151

<400>  151
000

<210>  152
<211>  30
<212>  PRT
<213>  Artificial Sequence

<220>
<223>  FR1 Vh m32H2

<400>  152

Glu Val Gln Leu Val Glu Ser Gly Gly Gly Ile Val Gln Pro Gly Gly
1               5                   10                  15

Ser Leu Lys Leu Ser Cys Ala Ala Ser Gly Phe Thr Phe Asn
            20                  25                  30

<210>  153
<211>  14
<212>  PRT
<213>  Artificial Sequence

<220>
<223>  FR2 VH m32H2

<400>  153

Trp Ile Arg Gln Thr Pro Asp Lys Arg Leu Glu Leu Val Ala
1               5                   10


<210>  154
<211>  33
<212>  PRT
<213>  Artificial Sequence

<220>
<223>  FR3 VH m32H2

<400>  154

Gly Arg Phe Ser Ile Ser Arg Asp Asn Ala Lys Lys Phe Val Tyr Leu
1               5                   10                  15


Gln Met Ser Ser Leu Lys Ser Asp Asp Thr Ala Ile Tyr Tyr Cys Pro
            20                  25                  30


Arg




<210>  155
<211>  11
<212>  PRT
<213>  Artificial Sequence

<220>
<223>  FR4 VH  m32H2

<400>  155

Trp Gly Gln Gly Thr Thr Leu Thr Val Ser Ser
1               5                   10


<210>  156
<211>  23
<212>  PRT
<213>  Artificial Sequence

<220>
<223>  FR1 VL  m32H2

<400>  156

Asp Ile Val Leu Thr Gln Ser Gln Lys Phe Met Ser Thr Ser Val Gly
1               5                   10                  15

Asp Arg Val Ser Ile Ile Cys
            20

<210>  157
<211>  15
<212>  PRT
<213>  Artificial Sequence

<220>
<223>  FR2 VL  m32H2

<400>  157

Trp Tyr Gln Leu Lys Ala Gly Gln Ser Pro Lys Leu Leu Ile Tyr
1               5                   10                  15

<210>  158
<211>  32
<212>  PRT
<213>  Artificial Sequence

<220>
<223>  FR3 VL  m32H2

<400>  158

Gly Val Pro Asp Arg Phe Thr Gly Ser Gly Ser Gly Thr Asp Phe Thr
1               5                   10                  15

Leu Thr Ile Ser Asn Val Gln Ser Glu Asp Leu Ala Asp Tyr Phe Cys
            20                  25                  30

<210>  159
<211>  10
<212>  PRT
<213>  Artificial Sequence

<220>
<223>  FR4 VL  m32H2

<400>  159

Phe Gly Ala Gly Thr Lys Leu Glu Leu Lys
1               5                   10

<210>  160
<211>  107
<212>  PRT
<213>  Artificial Sequence

<220>
<223>  VL  m32H2

<400>  160

Asp Ile Val Leu Thr Gln Ser Gln Lys Phe Met Ser Thr Ser Val Gly
1               5                   10                  15

Asp Arg Val Ser Ile Ile Cys Lys Ala Ser Gln Ser Val Gly Thr Ala
            20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys
            100                 105

<210>  161
<211>  114
<212>  PRT
<213>  Artificial Sequence

<220>
<223>  VH  m32H2

<400>  161

Glu Val Gln Leu Val Glu Ser Gly Gly Gly Ile Val Gln Pro Gly Gly
1               5                   10                  15

Ser Leu Lys Leu Ser Cys Ala Ala Ser Gly Phe Thr Phe Asn Asp Phe
            20                  25                  30

Gly Met Ser Trp Ile Arg Gln Thr Pro Asp Lys Arg Leu Glu Leu Val
        35                  40                  45

Ala Thr Ile Asn Thr Asn Gly Gly Thr Thr His Tyr Pro Asp Asn Val
    50                  55                  60

Lys Gly Arg Phe Ser Ile Ser Arg Asp Asn Ala Lys Lys Phe Val Tyr
65                  70                  75                  80

Leu Gln Met Ser Ser Leu Lys Ser Asp Asp Thr Ala Ile Tyr Tyr Cys
                85                  90                  95

Pro Arg Glu Gly Leu Asp Tyr Trp Gly Gln Gly Thr Thr Leu Thr Val
            100                 105                 110

Ser Ser

<210> 162
<211> 237
<212> PRT
<213> Artificial Sequence

<220>
<223> VL-VH m32H2

<400> 162

Asp Ile Val Leu Thr Gln Ser Gln Lys Phe Met Ser Thr Ser Val Gly
1               5                   10                  15

Asp Arg Val Ser Ile Ile Cys Lys Ala Ser Gln Ser Val Gly Thr Ala
            20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Ile Val Gln Pro Gly Gly Ser Leu Lys Leu Ser
    130                 135                 140

Cys Ala Ala Ser Gly Phe Thr Phe Asn Asp Phe Gly Met Ser Trp Ile
145                 150                 155                 160

Arg Gln Thr Pro Asp Lys Arg Leu Glu Leu Val Ala Thr Ile Asn Thr
                165                 170                 175

Asn Gly Gly Thr Thr His Tyr Pro Asp Asn Val Lys Gly Arg Phe Ser
            180                 185                 190

Ile Ser Arg Asp Asn Ala Lys Lys Phe Val Tyr Leu Gln Met Ser Ser
        195                 200                 205

Leu Lys Ser Asp Asp Thr Ala Ile Tyr Tyr Cys Pro Arg Glu Gly Leu
    210                 215                 220

Asp Tyr Trp Gly Gln Gly Thr Thr Leu Thr Val Ser Ser
225                 230                 235

<210>  163
<211>  30
<212>  PRT
<213>  Artificial Sequence

<220>
<223>  FR1 VH   H2-32H2

<400>  163

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn
            20                  25                  30

<210>  164
<211>  14
<212>  PRT
<213>  Artificial Sequence

<220>
<223>  FR2 VH  H2-32H2

<400>  164

Trp Val Arg Gln Ala Pro Gly Lys Gly Leu Glu Trp Val Ser
1               5                   10

<210>  165
<211>  33
<212>  PRT
<213>  Artificial Sequence

<220>
<223>  FR3 VH  H2-32H2

<400>  165

Gly Arg Phe Thr Ile Ser Arg Asp Asn Ser Lys Lys Phe Val Tyr Leu
1               5                   10                  15

Gln Met Asn Ser Leu Arg Ala Glu Asp Thr Ala Val Tyr Tyr Cys Pro
            20                  25                  30

Arg

<210>  166
<211>  11
<212>  PRT
<213>  Artificial Sequence

<220>
<223>  FR4 VH  H2-32H2

<400>  166

Trp Gly Gln Gly Thr Leu Val Thr Val Ser Ser
1               5                   10


<210>  167
<211>  23
<212>  PRT
<213>  Artificial Sequence

<220>
<223>  FR1 VL  H2-32H2

<400>  167

Glu Ile Val Leu Thr Gln Ser Pro Ala Thr Leu Ser Leu Ser Pro Gly
1               5                   10                  15


Glu Arg Ala Thr Leu Ser Cys
            20


<210>  168
<211>  15
<212>  PRT
<213>  Artificial Sequence

<220>
<223>  FR2 VL  H2-32H2

<400>  168

Trp Tyr Gln Leu Lys Pro Gly Gln Ala Pro Arg Leu Leu Ile Tyr
1               5                   10                  15


<210>  169
<211>  32
<212>  PRT
<213>  Artificial Sequence

<220>
<223>  FR3 VL  H2-32H2

<400>  169

Gly Ile Pro Ala Arg Phe Ser Gly Ser Gly Ser Gly Thr Asp Phe Thr
1               5                   10                  15

Leu Thr Ile Ser Ser Leu Glu Pro Glu Asp Phe Ala Val Tyr Tyr Cys
            20                  25                  30

<210>  170
<211>  10
<212>  PRT
<213>  Artificial Sequence

<220>
<223>  FR4 VL  H2-32H2

<400>  170

Phe Gly Gln Gly Thr Lys Leu Glu Ile Lys
1               5                   10

<210>  171
<211>  107
<212>  PRT
<213>  Artificial Sequence

<220>
<223>  VL  H2-32H2

<400>  171

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Leu Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys
            100                 105

<210>  172
<211>  114
<212>  PRT
<213>  Artificial Sequence

<220>
<223>  VH  H2-32H2

<400>  172

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Gly Met Ser Trp Val Arg Gln Ala Pro Gly Lys Gly Leu Glu Trp Val
        35                  40                  45

Ser Thr Ile Asn Thr Asn Gly Gly Thr Thr His Tyr Pro Asp Ser Val
    50                  55                  60

Lys Gly Arg Phe Thr Ile Ser Arg Asp Asn Ser Lys Lys Phe Val Tyr
65                  70                  75                  80

Leu Gln Met Asn Ser Leu Arg Ala Glu Asp Thr Ala Val Tyr Tyr Cys
                85                  90                  95

Pro Arg Glu Gly Leu Asp Tyr Trp Gly Gln Gly Thr Leu Val Thr Val
            100                 105                 110

Ser Ser

<210>  173
<211>  237
<212>  PRT
<213>  Artificial Sequence

<220>
<223>  VL-VH  H2-32H2

<400>  173

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Leu Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Asn Asp Phe Gly Met Ser Trp Val
145                 150                 155                 160

Arg Gln Ala Pro Gly Lys Gly Leu Glu Trp Val Ser Thr Ile Asn Thr
                165                 170                 175

Asn Gly Gly Thr Thr His Tyr Pro Asp Ser Val Lys Gly Arg Phe Thr
            180                 185                 190

Ile Ser Arg Asp Asn Ser Lys Lys Phe Val Tyr Leu Gln Met Asn Ser
        195                 200                 205

Leu Arg Ala Glu Asp Thr Ala Val Tyr Tyr Cys Pro Arg Glu Gly Leu
    210                 215                 220

Asp Tyr Trp Gly Gln Gly Thr Leu Val Thr Val Ser Ser
225                 230                 235

<210>  174
<211>  17
<212>  PRT
<213>  Artificial Sequence

<220>
<223>  CDR2 VH  H1-32H2

<400>  174

Thr Ile Asn Thr Asn Gly Gly Thr Thr His Tyr Pro Asp Ser Val Lys
1               5                   10                  15

Gly

<210>  175
<211>  11
<212>  PRT

<213>  Artificial Sequence

<220>
<223>  CDR1 VL  H1-32H2

<400>  175

Arg Ala Ser Gln Ser Val Gly Thr Ala Val Ala
1               5                   10


<210>  176
<211>  5
<212>  PRT
<213>  Artificial Sequence

<220>
<223>  CDR1 VH  H3-214D8

<400>  176

Ser Tyr Gly Met Ala
1               5


<210>  177
<211>  17
<212>  PRT
<213>  Artificial Sequence

<220>
<223>  CDR2 VH  H3-214D8

<400>  177

Ala Ile Asn Ser Asn Gly Gly Lys Thr Tyr Tyr Ala Asp Ser Val Lys
1               5                   10                  15


Gly




<210>  178

<400>  178
000

<210>  179
<211>  11
<212>  PRT
<213>  Artificial Sequence

<220>
<223>  CDR1 VL  H2-215C2

<400>  179

Arg Ala Ser Gln Asn Val Gly Thr Ala Val Ala
1               5                   10


<210>  180

<211>  107
<212>  PRT
<213>  Artificial Sequence

<220>
<223>  VL ScFv1 H1-32H2 (CDR2 174)

<400>  180

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys
            100                 105


<210>  181
<211>  115
<212>  PRT
<213>  Artificial Sequence

<220>
<223>  VH  H1-32H2 CDR1 175

<400>  181

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Gly Met Ser Trp Val Arg Gln Ala Pro Gly Lys Gly Leu Glu Trp Val
        35                  40                  45

Ser Thr Ile Asn Thr Asn Gly Gly Thr Thr His Tyr Pro Asp Ser Val
    50                  55                  60

Lys Gly Gly Arg Phe Thr Ile Ser Arg Asp Asn Ser Lys Asn Thr Leu
65                  70                  75                  80

Tyr Leu Gln Met Asn Ser Leu Arg Ala Glu Asp Thr Ala Val Tyr Tyr
                85                  90                  95

Cys Ala Lys Glu Gly Leu Asp Tyr Trp Gly Gln Gly Thr Leu Val Thr
            100                 105                 110

Val Ser Ser
        115

<210>  182
<211>  238
<212>  PRT
<213>  Artificial Sequence

<220>
<223>  VL-VH  H1-32H2 (VL CDR2 174 and VH CDR1 175)

<400>  182

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Asn Asp Phe Gly Met Ser Trp Val

145                 150                 155                 160

Arg Gln Ala Pro Gly Lys Gly Leu Glu Trp Val Ser Thr Ile Asn Thr
                165                 170                 175

Asn Gly Gly Thr Thr His Tyr Pro Asp Ser Val Lys Gly Gly Arg Phe
            180                 185                 190

Thr Ile Ser Arg Asp Asn Ser Lys Asn Thr Leu Tyr Leu Gln Met Asn
        195                 200                 205

Ser Leu Arg Ala Glu Asp Thr Ala Val Tyr Tyr Cys Ala Lys Glu Gly
    210                 215                 220

Leu Asp Tyr Trp Gly Gln Gly Thr Leu Val Thr Val Ser Ser
225                 230                 235

<210>  183
<211>  111
<212>  PRT
<213>  Artificial Sequence

<220>
<223>  VH  214D8 (CDR1, CDR2, CDR3 176, 177, 178)

<400>  183

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ala Trp Val Arg Gln Thr Pro Asp Arg Arg Leu Glu Leu Val
        35                  40                  45

Ala Ala Ile Asn Ser Asn Gly Gly Lys Thr Tyr Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Thr Arg Asp Tyr Trp Gly Gln Gly Thr Thr Leu Thr Val Ser Ser
            100                 105                 110

<210>  184
<211>  107

<212>   PRT
<213>   Artificial Sequence

<220>
<223>   VL 215C2 (CDR 179)

<400>   184

Asp Ile Val Met Thr Gln Ser Gln Lys Phe Met Ser Thr Ser Glu Gly
1               5                   10                  15

Asp Arg Val Ser Ile Thr Cys Arg Ala Ser Gln Asn Val Gly Thr Ala
            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys
            100                 105


<210>   185
<211>   236
<212>   PRT
<213>   Artificial Sequence

<220>
<223>   VL-VH 214D8 (VH 183)


<220>
<221>   misc_feature
<222>   (221)..(223)
<223>   Xaa can be any naturally occurring amino acid

<400>   185

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Ile Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Lys Ser
65                  70                  75                  80

Glu Asp Leu Ala Asp Tyr Phe Cys Gln Gln Tyr Ser Thr Tyr Pro Leu
                85                  90                  95

Thr Phe Gly Ala Gly Thr Lys Leu Glu Leu Lys Thr Gly Ser Thr Ser
            100                 105                 110

Gly Ser Gly Lys Pro Gly Ser Gly Glu Gly Glu Val Gln Leu Val Glu
        115                 120                 125

Ser Gly Gly Gly Leu Val Gln Pro Gly Gly Ser Leu Lys Leu Ser Cys
    130                 135                 140

Ala Ala Ser Gly Phe Thr Phe Ser Ser Tyr Gly Met Ala Trp Val Arg
145                 150                 155                 160

Gln Thr Pro Asp Arg Arg Leu Glu Leu Val Ala Ala Ile Asn Ser Asn
                165                 170                 175

Gly Gly Lys Thr Tyr Tyr Ala Asp Ser Val Lys Gly Arg Phe Thr Ile
            180                 185                 190

Ser Arg Asp Asn Ala Lys Asn Thr Leu Tyr Leu Gln Met Ser Ser Leu
        195                 200                 205

Lys Ser Glu Asp Thr Ala Met Tyr Tyr Cys Thr Arg Xaa Xaa Xaa Asp
    210                 215                 220

Tyr Trp Gly Gln Gly Thr Thr Leu Thr Val Ser Ser
225                 230                 235

<210>  186
<211>  237
<212>  PRT
<213>  Artificial Sequence

<220>
<223>  VL-VH 215C2 (VL 184)

<400>  186

Asp Ile Val Met Thr Gln Ser Gln Lys Phe Met Ser Thr Ser Glu Gly
1               5                   10                  15

Asp Arg Val Ser Ile Thr Cys Arg Ala Ser Gln Asn Val Gly Thr Ala
            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys Thr Gly Ser Thr Ser
            100                 105                 110

Gly Ser Gly Lys Pro Gly Ser Gly Glu Gly Ser Glu Val His Leu Val
        115                 120                 125

Glu Ser Gly Gly Gly Leu Val Gln Pro Gly Gly Ser Leu Lys Leu Ser
    130                 135                 140

Cys Ala Ala Ser Gly Phe Thr Phe Ser Asp Tyr Gly Met Ser Trp Val
145                 150                 155                 160

Arg Gln Thr Pro Asp Lys Arg Leu Glu Leu Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ala Lys Asn Thr Leu Tyr Leu Gln Met Asn Ser
        195                 200                 205

Leu Lys Ser Glu Asp Thr Ala Ile Tyr Tyr Cys Pro Arg Glu Gly Phe
    210                 215                 220

Asp Tyr Trp Gly Gln Gly Thr Thr Leu Thr Val Ser Ser
225                 230                 235

<210>  187
<211>  237
<212>  PRT
<213>  Artificial Sequence

<220>
<223>  VL-VH  H1-214D8

<400>  187

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Thr Tyr Gly Met Ala Trp Val
145                 150                 155                 160

Arg Gln Thr Pro Asp Arg Arg Leu Glu Leu Val Ala Thr Ile Asn Ser
                165                 170                 175

Asn Gly Gly Lys Thr Tyr His Pro Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Thr Arg Glu Gly Phe
    210                 215                 220

Asp Tyr Trp Gly Gln Gly Thr Leu Val Thr Val Ser Ser
225                 230                 235

<210>  188
<211>  237
<212>  PRT
<213>  Artificial Sequence

<220>
<223>  VL-VH  H2-214D8

<400>  188

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Thr Tyr Gly Met Ala Trp Val
145                 150                 155                 160

Arg Gln Ala Pro Gly Lys Gly Leu Glu Trp Val Ser Thr Ile Asn Ser
                165                 170                 175

Asn Gly Gly Lys Thr Tyr His Pro Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Thr Arg Glu Gly Phe

210                 215                 220

Asp Tyr Trp Gly Gln Gly Thr Leu Val Thr Val Ser Ser
225                 230                 235

<210>  189
<211>  234
<212>  PRT
<213>  Artificial Sequence

<220>
<223>  VL-VH  H3-214D8

<400>  189

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Ser Tyr Gly Met Ala Trp Val
145                 150                 155                 160

Arg Gln Ala Pro Gly Lys Gly Leu Glu Trp Val Ser Ala Ile Asn Ser
                165                 170                 175

Asn Gly Gly Lys Thr Tyr Tyr Ala Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Ala Lys Asp Tyr Trp
    210                 215                 220

Gly Gln Gly Thr Leu Val Thr Val Ser Ser
225                 230

<210>  190
<211>  237
<212>  PRT
<213>  Artificial Sequence

<220>
<223>  VL-VH  H1-215C2

<400>  190

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Val Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Asp Tyr Gly Met Ser Trp Val
145                 150                 155                 160

Arg Gln Ala Pro Gly Lys Arg Leu Glu Leu Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Pro Arg Glu Gly Phe
    210                 215                 220

Asp Tyr Trp Gly Gln Gly Thr Leu Val Thr Val Ser Ser
225                 230                 235

<210>  191
<211>  237
<212>  PRT
<213>  Artificial Sequence

<220>
<223>  VL-VH  H2-215C2

<400>  191

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala
            20                  25                  30

Val Ala Trp Phe Gln Gln Lys Pro Gly Lys Ala Pro Lys Ser Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Asp Tyr Gly Met Ser Trp Val
145                 150                 155                 160

Arg Gln Ala Pro Gly Lys Gly Leu Glu Trp Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Pro Arg Glu Gly Phe
    210                 215                 220

Asp Tyr Trp Gly Gln Gly Thr Leu Val Thr Val Ser Ser
225                 230                 235

<210>  192
<211>  221
<212>  PRT
<213>  Artificial Sequence

<220>
<223>  VL-VH  H3-215C2

<400>  192

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala
            20                  25                  30

Val Ala Trp Phe Gln Gln Lys Pro Gly Lys Ala Pro Lys Ser Leu Ile
        35                  40                  45

Tyr Ser Ala Ser Asn Arg Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys Thr Gly Ser Thr Ser

            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Asp Tyr Gly Met Ser Trp Val
145                 150                 155                 160

Arg Gln Ala Pro Gly Lys Gly Leu Glu Trp Val Ala Thr Ile Asn Gly
                165                 170                 175

Asn Gly Val Lys Thr Tyr Tyr Ala Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Pro Arg
    210                 215                 220

<210> 193
<211> 107
<212> PRT
<213> Artificial Sequence

<220>
<223> VL m32H2 (CDR1 VL 175)

<400> 193

Asp Ile Val Leu Thr Gln Ser Gln Lys Phe Met Ser Thr Ser Val Gly
1               5                   10                  15

Asp Arg Val Ser Ile Ile Cys Arg Ala Ser Gln Ser Val Gly Thr Ala
            20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys
            100                 105

<210>  194
<211>  115
<212>  PRT
<213>  Artificial Sequence

<220>
<223>  VH  m32H2 (CDR2 VH 174)

<400>  194

Glu Val Gln Leu Val Glu Ser Gly Gly Gly Ile Val Gln Pro Gly Gly
1               5                   10                  15

Ser Leu Lys Leu Ser Cys Ala Ala Ser Gly Phe Thr Phe Asn Asp Phe
            20                  25                  30

Gly Met Ser Trp Ile Arg Gln Thr Pro Asp Lys Arg Leu Glu Leu Val
        35                  40                  45

Ala Thr Ile Asn Thr Asn Gly Gly Thr Thr His Tyr Pro Asp Ser Val
    50                  55                  60

Lys Gly Gly Arg Phe Ser Ile Ser Arg Asp Asn Ala Lys Lys Phe Val
65                  70                  75                  80

Tyr Leu Gln Met Ser Ser Leu Lys Ser Asp Asp Thr Ala Ile Tyr Tyr
                85                  90                  95

Cys Pro Arg Glu Gly Leu Asp Tyr Trp Gly Gln Gly Thr Thr Leu Thr
            100                 105                 110

Val Ser Ser
        115

<210>  195
<211>  238
<212>  PRT
<213>  Artificial Sequence

<220>
<223>  VL-VH m32H2 (193 and 194)

<400>  195

Asp Ile Val Leu Thr Gln Ser Gln Lys Phe Met Ser Thr Ser Val Gly
1               5                   10                  15

Asp Arg Val Ser Ile Ile Cys Arg Ala Ser Gln Ser Val Gly Thr Ala

20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Ile Val Gln Pro Gly Gly Ser Leu Lys Leu Ser
    130                 135                 140

Cys Ala Ala Ser Gly Phe Thr Phe Asn Asp Phe Gly Met Ser Trp Ile
145                 150                 155                 160

Arg Gln Thr Pro Asp Lys Arg Leu Glu Leu Val Ala Thr Ile Asn Thr
                165                 170                 175

Asn Gly Gly Thr Thr His Tyr Pro Asp Ser Val Lys Gly Gly Arg Phe
            180                 185                 190

Ser Ile Ser Arg Asp Asn Ala Lys Lys Phe Val Tyr Leu Gln Met Ser
        195                 200                 205

Ser Leu Lys Ser Asp Asp Thr Ala Ile Tyr Tyr Cys Pro Arg Glu Gly
    210                 215                 220

Leu Asp Tyr Trp Gly Gln Gly Thr Thr Leu Thr Val Ser Ser
225                 230                 235

SEQUENCE LISTING

<110>  FUNDACIÓ PRIVADA INSTITUT D'INVESTIGACIÓ ONCOLÒGICA DE VALL
        HEBRON
       INSTITUCIÓ CATALANA DE RECERCA I ESTUDIS AVANÇATS

<120>  CHIMERIC ANTIGEN RECEPTORS SPECIFIC FOR P95HER2 AND USES THEREOF

<130>  P18520EP00

<160>  203

<170>  PatentIn version 3.5

<210>  1
<211>  5
<212>  PRT
<213>  Artificial Sequence

<220>
<223>  CDR1 VH m32H2

<400>  1

Asp Phe Gly Met Ser
1               5


<210>  2
<211>  17
<212>  PRT
<213>  Artificial Sequence

<220>
<223>  CDR2 VH m32H2

<400>  2

Thr Ile Asn Thr Asn Gly Gly Thr Thr His Tyr Pro Asp Asn Val Lys
1               5                   10                  15


Gly




<210>  3
<211>  5
<212>  PRT
<213>  Artificial Sequence

<220>
<223>  CDR3 VH m32H2

<400>  3

Glu Gly Leu Asp Tyr
1               5


<210>  4
<211>  11
<212>  PRT

<213>  Artificial Sequence

<220>
<223>  CDR1 VL m32H2

<400>  4

Lys Ala Ser Gln Ser Val Gly Thr Ala Val Ala
1               5                   10


<210>  5
<211>  7
<212>  PRT
<213>  Artificial Sequence

<220>
<223>  CDR2 VL m32H2

<400>  5

Ser Ala Ser Asn Arg Phe Thr
1               5


<210>  6
<211>  9
<212>  PRT
<213>  Artificial Sequence

<220>
<223>  CDR3 VL m32H2

<400>  6

Gln Gln Tyr Ser Thr Tyr Pro Leu Ala
1               5


<210>  7
<211>  5
<212>  PRT
<213>  Artificial Sequence

<220>
<223>  CDR1 VH 214D8

<400>  7

Thr Tyr Gly Met Ala
1               5


<210>  8
<211>  17
<212>  PRT
<213>  Artificial Sequence

<220>
<223>  CDR2 VH 214D8

<400>  8

Thr Ile Asn Ser Asn Gly Gly Lys Thr Tyr His Pro Asp Ser Val Lys
1               5                   10                  15

Gly

<210>  9
<211>  5
<212>  PRT
<213>  Artificial Sequence

<220>
<223>  CDR3 VH 214D8

<400>  9

Glu Gly Phe Asp Tyr
1               5

<210>  10
<211>  11
<212>  PRT
<213>  Artificial Sequence

<220>
<223>  CDR1 VL 214D8

<400>  10

Lys Ala Ser Gln Asn Val Gly Thr Ala Val Ala
1               5                   10

<210>  11
<211>  7
<212>  PRT
<213>  Artificial Sequence

<220>
<223>  CDR2 VL 214VL

<400>  11

Ser Ala Ser Asn Arg Tyr Thr
1               5

<210>  12
<211>  9
<212>  PRT
<213>  Artificial Sequence

<220>
<223>  CDR3 VL 214D8

<400>  12

Gln Gln Tyr Ser Thr Tyr Pro Leu Thr
1               5

<210>  13
<211>  5
<212>  PRT
<213>  Artificial Sequence

<220>
<223>  CDR1 VH 215C2

<400>  13

Asp Tyr Gly Met Ser
1               5


<210>  14
<211>  17
<212>  PRT
<213>  Artificial Sequence

<220>
<223>  CDR2 VH 215C2

<400>  14

Thr Ile Asn Gly Asn Gly Val Lys Ile Tyr Tyr Val Asp Ser Val Lys
1               5                   10                  15


Gly




<210>  15
<211>  5
<212>  PRT
<213>  Artificial Sequence

<220>
<223>  CDR3 VH 215C2

<400>  15

Glu Gly Phe Asp Tyr
1               5


<210>  16
<211>  11
<212>  PRT
<213>  Artificial Sequence

<220>
<223>  CDR1 VL 215C2

<400>  16

Lys Ala Ser Gln Asn Val Gly Thr Ala Val Ala
1               5                   10


<210>  17
<211>  7

<212>  PRT
<213>  Artificial Sequence

<220>
<223>  CDR2 VL 215C2

<400>  17

Ser Ala Ser Asn Arg Tyr Thr
1               5


<210>  18
<211>  9
<212>  PRT
<213>  Artificial Sequence

<220>
<223>  CDR3 VL 215C2

<400>  18

Gln Gln Tyr Ser Ser Tyr Pro Leu Thr
1               5


<210>  19
<211>  30
<212>  PRT
<213>  Artificial Sequence

<220>
<223>  FR1 VH  H1-32H2

<400>  19

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Asn
            20                  25                  30


<210>  20
<211>  14
<212>  PRT
<213>  Artificial Sequence

<220>
<223>  FR2 VH  H1-32H2

<400>  20

Trp Val Arg Gln Ala Pro Gly Lys Gly Leu Glu Trp Val Ser
1               5                   10


<210>  21
<211>  32
<212>  PRT
<213>  Artificial Sequence

<220>
<223>  FR3 VH  H1-32H2

<400>  21

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Ala Lys
            20                  25                  30

<210>  22
<211>  11
<212>  PRT
<213>  Artificial Sequence

<220>
<223>  FR4 VH  H1-32H2

<400>  22

Trp Gly Gln Gly Thr Leu Val Thr Val Ser Ser
1               5                   10

<210>  23
<211>  23
<212>  PRT
<213>  Artificial Sequence

<220>
<223>  FR1 VL  H1-32H2

<400>  23

Glu Ile Val Leu Thr Gln Ser Pro Ala Thr Leu Ser Leu Ser Pro Gly
1               5                   10                  15

Glu Arg Ala Thr Leu Ser Cys
            20

<210>  24
<211>  15
<212>  PRT
<213>  Artificial Sequence

<220>
<223>  FR2 VH  H1-32H2

<400>  24

Trp Tyr Gln Gln Lys Pro Gly Gln Ala Pro Arg Leu Leu Ile Tyr
1               5                   10                  15

<210>  25
<211>  32
<212>  PRT
<213>  Artificial Sequence

<220>
<223>  FR3 VL  H1-32H2

<400>  25

Gly Ile Pro Ala Arg Phe Ser Gly Ser Gly Ser Gly Thr Asp Phe Thr
1               5                   10                  15

Leu Thr Ile Ser Ser Leu Glu Pro Glu Asp Phe Ala Val Tyr Tyr Cys
            20                  25                  30

<210>  26
<211>  10
<212>  PRT
<213>  Artificial Sequence

<220>
<223>  FR4 VL  H1-32H2

<400>  26

Phe Gly Gln Gly Thr Lys Leu Glu Ile Lys
1               5                   10

<210>  27
<211>  107
<212>  PRT
<213>  Artificial Sequence

<220>
<223>  VL  (FRs H1-32H2 and CDRs m32H2)

<400>  27

Glu Ile Val Leu Thr Gln Ser Pro Ala Thr Leu Ser Leu Ser Pro Gly
1               5                   10                  15

Glu Arg Ala Thr Leu Ser Cys Lys Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys

100                 105

<210>  28
<211>  114
<212>  PRT
<213>  Artificial Sequence

<220>
<223>  VH  (FRs H1 32H2 and CDRs m32H2)

<400>  28

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Gly Met Ser Trp Val Arg Gln Ala Pro Gly Lys Gly Leu Glu Trp Val
        35                  40                  45

Ser Thr Ile Asn Thr Asn Gly Gly Thr Thr His Tyr Pro Asp Asn Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Glu Gly Leu Asp Tyr Trp Gly Gln Gly Thr Leu Val Thr Val
            100                 105                 110

Ser Ser

<210>  29
<211>  16
<212>  PRT
<213>  Artificial Sequence

<220>
<223>  linker coneting VH and VL regions

<400>  29

Thr Gly Ser Thr Ser Gly Ser Gly Lys Pro Gly Ser Gly Glu Gly Ser
1               5                   10                  15

<210>  30
<211>  237
<212>  PRT
<213>  Artificial Sequence

<220>
<223>  VL-VH (CDRs m32H2 and FRs H1 32H2)

<400>  30

Glu Ile Val Leu Thr Gln Ser Pro Ala Thr Leu Ser Leu Ser Pro Gly
1               5                   10                  15

Glu Arg Ala Thr Leu Ser Cys Lys Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Asn Asp Phe Gly Met Ser Trp Val
145                 150                 155                 160

Arg Gln Ala Pro Gly Lys Gly Leu Glu Trp Val Ser Thr Ile Asn Thr
                165                 170                 175

Asn Gly Gly Thr Thr His Tyr Pro Asp Asn Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Ala Lys Glu Gly Leu
    210                 215                 220

Asp Tyr Trp Gly Gln Gly Thr Leu Val Thr Val Ser Ser

225                 230                 235

<210>  31
<211>  30
<212>  PRT
<213>  Artificial Sequence

<220>
<223>  FR1 VH 214D8

<400>  31

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser
            20                  25                  30

<210>  32
<211>  14
<212>  PRT
<213>  Artificial Sequence

<220>
<223>  FR2 VH 214D8

<400>  32

Trp Val Arg Gln Thr Pro Asp Arg Arg Leu Glu Leu Val Ala
1               5                   10

<210>  33
<211>  32
<212>  PRT
<213>  Artificial Sequence

<220>
<223>  FR3 VH 214D8

<400>  33

Arg Phe Thr Ile Ser Arg Asp Asn Ala Lys Asn Thr Leu Tyr Leu Gln
1               5                   10                  15

Met Ser Ser Leu Lys Ser Glu Asp Thr Ala Met Tyr Tyr Cys Thr Arg
            20                  25                  30

<210>  34
<211>  11
<212>  PRT
<213>  Artificial Sequence

<220>
<223>  FR4 VH 214D8

<400>  34

Trp Gly Gln Gly Thr Thr Leu Thr Val Ser Ser
1               5                   10

<210>  35
<211>  23
<212>  PRT
<213>  Artificial Sequence

<220>
<223>  FR1 VL 214D8

<400>  35

Asp Ile Val Met Thr Gln Ser Gln Lys Phe Met Ser Thr Ser Val Gly
1               5                   10                  15

Asp Arg Val Ser Ile Thr Cys
            20

<210>  36
<211>  15
<212>  PRT
<213>  Artificial Sequence

<220>
<223>  FR2 VL 214D8

<400>  36

Trp Tyr Gln Gln Lys Pro Gly Gln Ser Pro Lys Ile Leu Ile Tyr
1               5                   10                  15

<210>  37
<211>  32
<212>  PRT
<213>  Artificial Sequence

<220>
<223>  FR3 VL 214C8

<400>  37

Gly Val Pro Asp Arg Phe Thr Gly Ser Gly Ser Gly Thr Asp Phe Thr
1               5                   10                  15

Leu Thr Ile Ser Asn Met Lys Ser Glu Asp Leu Ala Asp Tyr Phe Cys
            20                  25                  30

<210>  38
<211>  10
<212>  PRT
<213>  Artificial Sequence

<220>
<223>  FR4 VL 214D8

<400>  38

Phe Gly Ala Gly Thr Lys Leu Glu Leu Lys
1               5                   10

<210>  39
<211>  107
<212>  PRT
<213>  Artificial Sequence

<220>
<223>  VL 214D8

<400>  39

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Ile Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Lys Ser
65                  70                  75                  80

Glu Asp Leu Ala Asp Tyr Phe Cys Gln Gln Tyr Ser Thr Tyr Pro Leu
                85                  90                  95

Thr Phe Gly Ala Gly Thr Lys Leu Glu Leu Lys
            100                 105

<210>  40
<211>  114
<212>  PRT
<213>  Artificial Sequence

<220>
<223>  VH 214D8

<400>  40

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Thr Tyr
            20                  25                  30

Gly Met Ala Trp Val Arg Gln Thr Pro Asp Arg Arg Leu Glu Leu Val
        35                  40                  45

Ala Thr Ile Asn Ser Asn Gly Gly Lys Thr Tyr His Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Thr Arg Glu Gly Phe Asp Tyr Trp Gly Gln Gly Thr Thr Leu Thr Val
            100                 105                 110

Ser Ser

<210>  41
<211>  237
<212>  PRT
<213>  Artificial Sequence

<220>
<223>  214D8  VL-VH

<400>  41

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Ile Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Lys Ser
65                  70                  75                  80

Glu Asp Leu Ala Asp Tyr Phe Cys Gln Gln Tyr Ser Thr Tyr Pro Leu
                85                  90                  95

Thr Phe Gly Ala Gly Thr Lys Leu Glu Leu Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Lys Leu Ser
    130                 135                 140

Cys Ala Ala Ser Gly Phe Thr Phe Ser Thr Tyr Gly Met Ala Trp Val
145                 150                 155                 160

Arg Gln Thr Pro Asp Arg Arg Leu Glu Leu Val Ala Thr Ile Asn Ser
                165                 170                 175

Asn Gly Gly Lys Thr Tyr His Pro Asp Ser Val Lys Gly Arg Phe Thr
            180                 185                 190

Ile Ser Arg Asp Asn Ala Lys Asn Thr Leu Tyr Leu Gln Met Ser Ser
        195                 200                 205

Leu Lys Ser Glu Asp Thr Ala Met Tyr Tyr Cys Thr Arg Glu Gly Phe
    210                 215                 220

Asp Tyr Trp Gly Gln Gly Thr Thr Leu Thr Val Ser Ser
225                 230                 235

<210>  42
<211>  30
<212>  PRT
<213>  Artificial Sequence

<220>
<223>  FR1 VH 215C2

<400>  42

Glu Val His Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser
            20                  25                  30

<210>  43
<211>  14
<212>  PRT
<213>  Artificial Sequence

<220>
<223>  FR2 VH 215C2

<400>  43

Trp Val Arg Gln Thr Pro Asp Lys Arg Leu Glu Leu Val Ala
1               5                   10

<210>  44
<211>  32

<212>  PRT
<213>  Artificial Sequence

<220>
<223>  FR3 VH 215C2

<400>  44

Arg Phe Thr Ile Ser Arg Asp Asn Ala Lys Asn Thr Leu Tyr Leu Gln
1               5                   10                  15

Met Asn Ser Leu Lys Ser Glu Asp Thr Ala Ile Tyr Tyr Cys Pro Arg
            20                  25                  30

<210>  45
<211>  11
<212>  PRT
<213>  Artificial Sequence

<220>
<223>  FR4 VH 215C2

<400>  45

Trp Gly Gln Gly Thr Thr Leu Thr Val Ser Ser
1               5                   10

<210>  46
<211>  23
<212>  PRT
<213>  Artificial Sequence

<220>
<223>  FR1 VL 215C2

<400>  46

Asp Ile Val Met Thr Gln Ser Gln Lys Phe Met Ser Thr Ser Glu Gly
1               5                   10                  15

Asp Arg Val Ser Ile Thr Cys
            20

<210>  47
<211>  15
<212>  PRT
<213>  Artificial Sequence

<220>
<223>  FR2 VL 215C2

<400>  47

Trp Tyr Gln Gln Lys Pro Gly Gln Ser Pro Lys Val Leu Ile Tyr
1               5                   10                  15

<210>  48

<211>  32
<212>  PRT
<213>  Artificial Sequence

<220>
<223>  FR3 VL 215C2

<400>  48

Gly Val Pro Asp Arg Phe Thr Gly Ser Gly Ser Gly Thr Asp Phe Thr
1               5                   10                  15

Leu Thr Ile Ser Asn Met Gln Ser Glu Asp Leu Ala Asp Tyr Phe Cys
            20                  25                  30


<210>  49
<211>  10
<212>  PRT
<213>  Artificial Sequence

<220>
<223>  FR4 VL 215C2

<400>  49

Phe Gly Ala Gly Thr Thr Leu Glu Leu Lys
1               5                   10


<210>  50
<211>  107
<212>  PRT
<213>  Artificial Sequence

<220>
<223>  VL 215C2

<400>  50

Asp Ile Val Met Thr Gln Ser Gln Lys Phe Met Ser Thr Ser Glu Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys
            100                 105

<210> 51
<211> 114
<212> PRT
<213> Artificial Sequence

<220>
<223> VH 215C2

<400> 51

Glu Val His Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Asp Tyr
            20                  25                  30

Gly Met Ser Trp Val Arg Gln Thr Pro Asp Lys Arg Leu Glu Leu Val
        35                  40                  45

Ala Thr Ile Asn Gly Asn Gly Val Lys Ile Tyr Tyr Val Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Ile Tyr Tyr Cys
                85                  90                  95

Pro Arg Glu Gly Phe Asp Tyr Trp Gly Gln Gly Thr Thr Leu Thr Val
            100                 105                 110

Ser Ser

<210> 52
<211> 237
<212> PRT
<213> Artificial Sequence

<220>
<223> 215C2 VL-VH

<400> 52

Asp Ile Val Met Thr Gln Ser Gln Lys Phe Met Ser Thr Ser Glu Gly
1               5                   10                  15

Asp Arg Val Ser Ile Thr Cys Lys Ala Ser Gln Asn Val Gly Thr Ala

            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys Thr Gly Ser Thr Ser
            100                 105                 110

Gly Ser Gly Lys Pro Gly Ser Gly Glu Gly Ser Glu Val His Leu Val
        115                 120                 125

Glu Ser Gly Gly Gly Leu Val Gln Pro Gly Gly Ser Leu Lys Leu Ser
    130                 135                 140

Cys Ala Ala Ser Gly Phe Thr Phe Ser Asp Tyr Gly Met Ser Trp Val
145                 150                 155                 160

Arg Gln Thr Pro Asp Lys Arg Leu Glu Leu Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ala Lys Asn Thr Leu Tyr Leu Gln Met Asn Ser
        195                 200                 205

Leu Lys Ser Glu Asp Thr Ala Ile Tyr Tyr Cys Pro Arg Glu Gly Phe
    210                 215                 220

Asp Tyr Trp Gly Gln Gly Thr Thr Leu Thr Val Ser Ser
225                 230                 235

<210> 53
<211> 114
<212> PRT
<213> Artificial Sequence

<220>
<223> VH H1-214D8

<400>  53

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Gly Met Ala Trp Val Arg Gln Thr Pro Asp Arg Arg Leu Glu Leu Val
        35                  40                  45

Ala Thr Ile Asn Ser Asn Gly Gly Lys Thr Tyr His Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Glu Gly Phe Asp Tyr Trp Gly Gln Gly Thr Leu Val Thr Val
            100                 105                 110

Ser Ser

<210>  54
<211>  107
<212>  PRT
<213>  Artificial Sequence

<220>
<223>  VL  H1-214D8

<400>  54

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys
            100                 105

<210>  55
<211>  114
<212>  PRT
<213>  Artificial Sequence

<220>
<223>  VH  H2-214D8

<400>  55

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Gly Met Ala Trp Val Arg Gln Ala Pro Gly Lys Gly Leu Glu Trp Val
        35                  40                  45

Ser Thr Ile Asn Ser Asn Gly Gly Lys Thr Tyr His Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Glu Gly Phe Asp Tyr Trp Gly Gln Gly Thr Leu Val Thr Val
            100                 105                 110

Ser Ser

<210>  56
<211>  107
<212>  PRT
<213>  Artificial Sequence

<220>
<223>  VL  H2-214D8

<400>  56

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys
            100                 105

<210>  57
<211>  111
<212>  PRT
<213>  Artificial Sequence

<220>
<223>  VH  H3-214D8

<400>  57

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met Ala Trp Val Arg Gln Ala Pro Gly Lys Gly Leu Glu Trp Val
        35                  40                  45

Ser Ala Ile Asn Ser Asn Gly Gly Lys Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Tyr Trp Gly Gln Gly Thr Leu Val Thr Val Ser Ser
            100                 105                 110

<210>  58
<211>  107
<212>  PRT
<213>  Artificial Sequence

<220>
<223>  VL  H3-214D8

<400>  58

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys
            100                 105


<210>  59
<211>  114
<212>  PRT
<213>  Artificial Sequence

<220>
<223>  VH  H1-215C2

<400>  59

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Met Ser Trp Val Arg Gln Ala Pro Gly Lys Arg Leu Glu Leu Val
        35                  40                  45

Ala Thr Ile Asn Gly Asn Gly Val Lys Ile Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg Glu Gly Phe Asp Tyr Trp Gly Gln Gly Thr Leu Val Thr Val
            100                 105                 110

Ser Ser

<210>  60
<211>  107
<212>  PRT
<213>  Artificial Sequence

<220>
<223>  VL  H1-215C2

<400>  60

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Val Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys
            100                 105

<210>  61
<211>  114
<212>  PRT
<213>  Artificial Sequence

<220>
<223>  VH  H2-215C2

<400>  61

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Met Ser Trp Val Arg Gln Ala Pro Gly Lys Gly Leu Glu Trp Val
        35                  40                  45

Ala Thr Ile Asn Gly Asn Gly Val Lys Ile Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg Glu Gly Phe Asp Tyr Trp Gly Gln Gly Thr Leu Val Thr Val
            100                 105                 110

Ser Ser

<210>  62
<211>  107
<212>  PRT
<213>  Artificial Sequence

<220>
<223>  VL  H2-215C2

<400>  62

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala
            20                  25                  30

Val Ala Trp Phe Gln Gln Lys Pro Gly Lys Ala Pro Lys Ser Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys
            100                 105

<210>  63
<211>  98
<212>  PRT
<213>  Artificial Sequence

<220>
<223>  VH  H3-215C2

<400>  63

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Met Ser Trp Val Arg Gln Ala Pro Gly Lys Gly Leu Glu Trp Val
        35                  40                  45

Ala Thr Ile Asn Gly Asn Gly Val Lys Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg

<210>  64
<211>  107
<212>  PRT
<213>  Artificial Sequence

<220>
<223>  VL  H3-215C2

<400>  64

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala

20                  25                  30

Val Ala Trp Phe Gln Gln Lys Pro Gly Lys Ala Pro Lys Ser Leu Ile
        35                  40                  45

Tyr Ser Ala Ser Asn Arg Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys
            100                 105

<210>  65
<211>  30
<212>  PRT
<213>  Artificial Sequence

<220>
<223>  FR1 VH  H1-214D8

<400>  65

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  66
<211>  14
<212>  PRT
<213>  Artificial Sequence

<220>
<223>  FR2 VH  H1-214D8

<400>  66

Trp Val Arg Gln Thr Pro Asp Arg Arg Leu Glu Leu Val Ala
1               5                   10

<210>  67
<211>  32
<212>  PRT
<213>  Artificial Sequence

<220>
<223>  FR3 VH  H1-214D8

<400>  67

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Thr Arg
            20                  25                  30

<210>  68
<211>  11
<212>  PRT
<213>  Artificial Sequence

<220>
<223>  FR4 VH  H1-214D8

<400>  68

Trp Gly Gln Gly Thr Leu Val Thr Val Ser Ser
1               5                   10

<210>  69
<211>  23
<212>  PRT
<213>  Artificial Sequence

<220>
<223>  FR1 VL  H1-214D8

<400>  69

Asp Ile Gln Met Thr Gln Ser Pro Ser Ser Leu Ser Ala Ser Val Gly
1               5                   10                  15

Asp Arg Val Thr Ile Thr Cys
            20

<210>  70
<211>  15
<212>  PRT
<213>  Artificial Sequence

<220>
<223>  FR2 VL  H1-214D8

<400>  70

Trp Phe Gln Gln Lys Pro Gly Lys Ala Pro Lys Ile Leu Ile Tyr
1               5                   10                  15

<210>  71
<211>  32
<212>  PRT
<213>  Artificial Sequence

<220>
<223>  FR3 VL  H1-214D8

<400>  71

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  72
<211>  10
<212>  PRT
<213>  Artificial Sequence

<220>
<223>  FR4 VL  H1-214D8

<400>  72

Phe Gly Gln Gly Thr Lys Leu Glu Ile Lys
1               5                   10

<210>  73
<211>  30
<212>  PRT
<213>  Artificial Sequence

<220>
<223>  FR1 VH  H2-214D8

<400>  73

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  74
<211>  14
<212>  PRT
<213>  Artificial Sequence

<220>
<223>  FR2 VH  H2-214D8

<400>  74

Trp Val Arg Gln Ala Pro Gly Lys Gly Leu Glu Trp Val Ser
1               5                   10

<210>  75
<211>  32
<212>  PRT
<213>  Artificial Sequence

<220>

<223>  FR3 VH  H2-214D8

<400>  75

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Thr Arg
            20                  25                  30

<210>  76
<211>  11
<212>  PRT
<213>  Artificial Sequence

<220>
<223>  FR4 VH  H2-214D8

<400>  76

Trp Gly Gln Gly Thr Leu Val Thr Val Ser Ser
1               5                   10

<210>  77
<211>  23
<212>  PRT
<213>  Artificial Sequence

<220>
<223>  FR1 VL  H2-214D8

<400>  77

Asp Ile Gln Met Thr Gln Ser Pro Ser Ser Leu Ser Ala Ser Val Gly
1               5                   10                  15

Asp Arg Val Thr Ile Thr Cys
            20

<210>  78
<211>  15
<212>  PRT
<213>  Artificial Sequence

<220>
<223>  FR2 VL  H2-214D8

<400>  78

Trp Phe Gln Gln Lys Pro Gly Lys Ala Pro Lys Ile Leu Ile Tyr
1               5                   10                  15

<210>  79
<211>  32
<212>  PRT
<213>  Artificial Sequence

<220>
<223>  FR3 VL  H2-214D8

<400>  79

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  80
<211>  10
<212>  PRT
<213>  Artificial Sequence

<220>
<223>  FR4 VL  H2-214D8

<400>  80

Phe Gly Gln Gly Thr Lys Leu Glu Ile Lys
1               5                   10

<210>  81
<211>  30
<212>  PRT
<213>  Artificial Sequence

<220>
<223>  FR1 VH  H3-214D8

<400>  81

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  82
<211>  14
<212>  PRT
<213>  Artificial Sequence

<220>
<223>  FR2 VH  H3-214D8

<400>  82

Trp Val Arg Gln Ala Pro Gly Lys Gly Leu Glu Trp Val Ser
1               5                   10

<210>  83
<211>  32
<212>  PRT
<213>  Artificial Sequence

<220>
<223>  FR3 VH  H3-214D8

<400>  83

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Ala Lys
            20                  25                  30

<210>  84
<211>  11
<212>  PRT
<213>  Artificial Sequence

<220>
<223>  FR4 VH  H3-214D8

<400>  84

Trp Gly Gln Gly Thr Leu Val Thr Val Ser Ser
1               5                   10

<210>  85
<211>  23
<212>  PRT
<213>  Artificial Sequence

<220>
<223>  FR1 VL  H3-214D8

<400>  85

Asp Ile Gln Met Thr Gln Ser Pro Ser Ser Leu Ser Ala Ser Val Gly
1               5                   10                  15

Asp Arg Val Thr Ile Thr Cys
            20

<210>  86
<211>  15
<212>  PRT
<213>  Artificial Sequence

<220>
<223>  FR2 VL  H3-214D8

<400>  86

Trp Phe Gln Gln Lys Pro Gly Lys Ala Pro Lys Ile Leu Ile Tyr
1               5                   10                  15

<210>  87
<211>  32
<212>  PRT

<213>  Artificial Sequence

<220>
<223>  FR3 VL  H3-214D8

<400>  87

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  88
<211>  10
<212>  PRT
<213>  Artificial Sequence

<220>
<223>  FR4 VL  H3-214D8

<400>  88

Phe Gly Gln Gly Thr Lys Leu Glu Ile Lys
1               5                   10

<210>  89
<211>  30
<212>  PRT
<213>  Artificial Sequence

<220>
<223>  FR1 VH  H1-215C2

<400>  89

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  90
<211>  14
<212>  PRT
<213>  Artificial Sequence

<220>
<223>  FR2 VH  H1-215C2

<400>  90

Trp Val Arg Gln Ala Pro Gly Lys Arg Leu Glu Leu Val Ala
1               5                   10

<210>  91
<211>  32

<212>  PRT
<213>  Artificial Sequence

<220>
<223>  FR3 VH  H1-215C2

<400>  91

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Pro Arg
            20                  25                  30

<210>  92
<211>  11
<212>  PRT
<213>  Artificial Sequence

<220>
<223>  FR4 VH  H1-215C2

<400>  92

Trp Gly Gln Gly Thr Leu Val Thr Val Ser Ser
1               5                   10

<210>  93
<211>  23
<212>  PRT
<213>  Artificial Sequence

<220>
<223>  FR1 VL  H1-215C2

<400>  93

Asp Ile Gln Met Thr Gln Ser Pro Ser Ser Leu Ser Ala Ser Val Gly
1               5                   10                  15

Asp Arg Val Thr Ile Thr Cys
            20

<210>  94
<211>  15
<212>  PRT
<213>  Artificial Sequence

<220>
<223>  FR2 VL  H1-215C2

<400>  94

Trp Phe Gln Gln Lys Pro Gly Lys Ala Pro Lys Val Leu Ile Tyr
1               5                   10                  15

<210>  95

<211>  32
<212>  PRT
<213>  Artificial Sequence

<220>
<223>  FR3 VL  H1-215C2

<400>  95

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  96
<211>  10
<212>  PRT
<213>  Artificial Sequence

<220>
<223>  FR4 VL  H1-215C2

<400>  96

Phe Gly Gln Gly Thr Lys Leu Glu Ile Lys
1               5                   10

<210>  97
<211>  30
<212>  PRT
<213>  Artificial Sequence

<220>
<223>  FR1 VH  H2-215C2

<400>  97

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  98
<211>  14
<212>  PRT
<213>  Artificial Sequence

<220>
<223>  FR2 VH  H2-215C2

<400>  98

Trp Val Arg Gln Ala Pro Gly Lys Gly Leu Glu Trp Val Ala
1               5                   10

<210>  99
<211>  32
<212>  PRT
<213>  Artificial Sequence

<220>
<223>  FR3 VH  H2-215C2

<400>  99

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Pro Arg
            20                  25                  30

<210>  100
<211>  11
<212>  PRT
<213>  Artificial Sequence

<220>
<223>  FR4 VH  H2-215C2

<400>  100

Trp Gly Gln Gly Thr Leu Val Thr Val Ser Ser
1               5                   10

<210>  101
<211>  23
<212>  PRT
<213>  Artificial Sequence

<220>
<223>  FR1 VL  H2-215C2

<400>  101

Asp Ile Gln Met Thr Gln Ser Pro Ser Ser Leu Ser Ala Ser Val Gly
1               5                   10                  15

Asp Arg Val Thr Ile Thr Cys
            20

<210>  102
<211>  15
<212>  PRT
<213>  Artificial Sequence

<220>
<223>  FR2 VL  H2-215C2

<400>  102

Trp Phe Gln Gln Lys Pro Gly Lys Ala Pro Lys Ser Leu Ile Tyr
1               5                   10                  15

<210>  103
<211>  32
<212>  PRT
<213>  Artificial Sequence

<220>
<223>  FR3 VL  H2-215C2

<400>  103

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  104
<211>  10
<212>  PRT
<213>  Artificial Sequence

<220>
<223>  FR4 VL  H2-215C2

<400>  104

Phe Gly Gln Gly Thr Lys Leu Glu Ile Lys
1               5                   10

<210>  105
<211>  30
<212>  PRT
<213>  Artificial Sequence

<220>
<223>  FR1 VH  H3-215C2

<400>  105

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser
            20                  25                  30

<210>  106
<211>  14
<212>  PRT
<213>  Artificial Sequence

<220>
<223>  FR2 VH  H3-215C2

<400>  106

Trp Val Arg Gln Ala Pro Gly Lys Gly Leu Glu Trp Val Ala
1               5                   10

<210>  107
<211>  32
<212>  PRT
<213>  Artificial Sequence

<220>
<223>  FR3 VH  H3-215C2

<400>  107

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Pro Arg
            20                  25                  30

<210>  108
<211>  11
<212>  PRT
<213>  Artificial Sequence

<220>
<223>  X

<400>  108

Trp Gly Gln Gly Thr Leu Val Thr Val Ser Ser
1               5                   10

<210>  109
<211>  23
<212>  PRT
<213>  Artificial Sequence

<220>
<223>  FR1 VL  H3-2015C2

<400>  109

Asp Ile Gln Met Thr Gln Ser Pro Ser Ser Leu Ser Ala Ser Val Gly
1               5                   10                  15

Asp Arg Val Thr Ile Thr Cys
            20

<210>  110
<211>  15
<212>  PRT
<213>  Artificial Sequence

<220>
<223>  FR2 VL  H3-215C2

<400>  110

Trp Phe Gln Gln Lys Pro Gly Lys Ala Pro Lys Ser Leu Ile Tyr

1                   5                   10                  15


<210>  111
<211>  32
<212>  PRT
<213>  Artificial Sequence

<220>
<223>  FR3 VL  H3-215C2

<400>  111

Gly Val Pro Ser Arg Phe Ser Gly Ser Gly Ser Gly Thr Asp Phe Thr
1               5                   10                  15


Leu Thr Ile Ser Ser Leu Gln Pro Glu Asp Phe Ala Thr Tyr Tyr Cys
            20                  25                  30


<210>  112
<211>  10
<212>  PRT
<213>  Artificial Sequence

<220>
<223>  FR4 VL  H3-215C2

<400>  112

Phe Gly Gln Gly Thr Lys Leu Glu Ile Lys
1               5                   10


<210>  113
<211>  29
<212>  PRT
<213>  Artificial Sequence

<220>
<223>  CD28 transmembrane domain

<400>  113

Phe Trp Val Leu Val Val Val Gly Gly Asx Cys Val Leu Ala Cys Tyr
1               5                   10                  15


Ser Leu Leu Val Thr Val Ala Phe Ile Ile Phe Trp Val
            20                  25


<210>  114
<211>  112
<212>  PRT
<213>  Artificial Sequence

<220>
<223>  CD3-zeta intracellular signalling domain

<400>  114

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  115
<211>  41
<212>  PRT
<213>  Artificial Sequence

<220>
<223>  intracellualr domain of the coestimulatory domain CD28

<400>  115

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  116
<211>  107
<212>  PRT
<213>  Artificial Sequence

<220>
<223>  Spacer

<400>  116

Gly Gln Pro Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ser Val Glu Trp Glu Ser Asn Gly Gln Pro Glu
        35                  40                  45

Asn Asn Tyr Lys Thr Thr Pro Pro Met Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210>  117
<211>  5
<212>  PRT
<213>  Artificial Sequence

<220>
<223>  linker

<400>  117

Gly Ser Gly Gly Ser
1               5

<210>  118
<211>  4
<212>  PRT
<213>  Artificial Sequence

<220>
<223>  linker

<400>  118

Gly Gly Gly Ser
1

<210>  119
<211>  4
<212>  PRT
<213>  Artificial Sequence

<220>
<223>  linker

<400>  119

Gly Gly Ser Gly
1

<210> 120
<211> 5
<212> PRT
<213> Artificial Sequence

<220>
<223> linker

<400> 120

Gly Gly Ser Gly Gly
1               5

<210> 121
<211> 5
<212> PRT
<213> Artificial Sequence

<220>
<223> linker

<400> 121

Gly Ser Gly Ser Gly
1               5

<210> 122
<211> 5
<212> PRT
<213> Artificial Sequence

<220>
<223> linker

<400> 122

Gly Ser Gly Gly Gly
1               5

<210> 123
<211> 5
<212> PRT
<213> Artificial Sequence

<220>
<223> linker

<400> 123

Gly Gly Gly Ser Gly
1               5

<210> 124
<211> 5
<212> PRT

<213>  Artificial Sequence

<220>
<223>  linker

<400>  124

Gly Ser Ser Ser Gly
1               5


<210>  125
<211>  5
<212>  PRT
<213>  Artificial Sequence

<220>
<223>  hinge region

<400>  125

Gly Ser Gly Gly Ser
1               5


<210>  126
<211>  4
<212>  PRT
<213>  Artificial Sequence

<220>
<223>  hinge region

<400>  126

Gly Gly Gly Ser
1


<210>  127
<211>  4
<212>  PRT
<213>  Artificial Sequence

<220>
<223>  hinge region

<400>  127

Gly Gly Ser Gly
1


<210>  128
<211>  5
<212>  PRT
<213>  Artificial Sequence

<220>
<223>  hinge region

<400>  128

Gly Gly Ser Gly Gly
1               5

<210>  129
<211>  5
<212>  PRT
<213>  Artificial Sequence

<220>
<223>  hinge region

<400>  129

Gly Ser Gly Ser Gly
1               5

<210>  130
<211>  5
<212>  PRT
<213>  Artificial Sequence

<220>
<223>  hinge region

<400>  130

Gly Ser Gly Gly Gly
1               5

<210>  131
<211>  5
<212>  PRT
<213>  Artificial Sequence

<220>
<223>  hinge region

<400>  131

Gly Gly Gly Ser Gly
1               5

<210>  132
<211>  5
<212>  PRT
<213>  Artificial Sequence

<220>
<223>  hinge region

<400>  132

Gly Ser Ser Ser Gly
1               5

<210>  133
<211>  4
<212>  PRT

<213>  Artificial Sequence

<220>
<223>  hinge region

<400>  133

Cys Pro Pro Cys
1


<210>  134
<211>  5
<212>  PRT
<213>  Artificial Sequence

<220>
<223>  hinge region

<400>  134

Asp Lys Thr His Thr
1               5


<210>  135

<400>  135
000

<210>  136
<211>  15
<212>  PRT
<213>  Artificial Sequence

<220>
<223>  hinge region

<400>  136

Cys Pro Glu Pro Lys Ser Cys Asp Thr Pro Pro Pro Cys Pro Arg
1               5                   10                  15


<210>  137
<211>  12
<212>  PRT
<213>  Artificial Sequence

<220>
<223>  hinge region

<400>  137

Glu Leu Lys Thr Pro Leu Gly Asp Thr Thr His Thr
1               5                   10


<210>  138
<211>  10
<212>  PRT
<213>  Artificial Sequence

<220>
<223>  hinge region

<400>  138

Lys Ser Cys Asp Lys Thr His Thr Cys Pro
1               5                   10


<210>  139
<211>  7
<212>  PRT
<213>  Artificial Sequence

<220>
<223>  hinge region

<400>  139

Lys Cys Cys Val Asp Cys Pro
1               5


<210>  140
<211>  7
<212>  PRT
<213>  Artificial Sequence

<220>
<223>  hinge region

<400>  140

Lys Tyr Gly Pro Pro Cys Pro
1               5


<210>  141
<211>  15
<212>  PRT
<213>  Artificial Sequence

<220>
<223>  hinge region

<400>  141

Glu Pro Lys Ser Cys Asp Lys Thr His Thr Cys Pro Pro Cys Pro
1               5                   10                  15


<210>  142
<211>  12
<212>  PRT
<213>  Artificial Sequence

<220>
<223>  hinge region

<400>  142

Glu Arg Lys Cys Cys Val Glu Cys Pro Pro Cys Pro
1               5                   10

<210>  143
<211>  17
<212>  PRT
<213>  Artificial Sequence

<220>
<223>  hinge region

<400>  143

Glu Leu Lys Thr Pro Leu Gly Asp Thr Thr His Thr Cys Pro Arg Cys
1               5                   10                  15

Pro

<210>  144
<211>  12
<212>  PRT
<213>  Artificial Sequence

<220>
<223>  hinge region

<400>  144

Ser Pro Asn Met Val Pro His Ala His His Ala Gln
1               5                   10

<210>  145
<211>  15
<212>  PRT
<213>  Artificial Sequence

<220>
<223>  hinge region

<400>  145

Glu Pro Lys Ser Cys Asp Lys Thr Tyr Thr Cys Pro Pro Cys Pro
1               5                   10                  15

<210>  146
<211>  45
<212>  PRT
<213>  Artificial Sequence

<220>
<223>  CD8 hinge region

<400>  146

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly

20                  25                  30

Gly Ala Val His Thr Arg Gly Leu Asp Phe Ala Cys Asp
        35                  40                  45

<210>  147
<211>  21
<212>  PRT
<213>  Artificial Sequence

<220>
<223>  leader sequence

<400>  147

Met Ala Leu Pro Val Thr Ala Leu Leu Leu Pro Leu Ala Leu Leu Leu
1               5                   10                  15

His Ala Ala Arg Pro
            20

<210>  148

<400>  148
000

<210>  149

<400>  149
000

<210>  150

<400>  150
000

<210>  151

<400>  151
000

<210>  152
<211>  30
<212>  PRT
<213>  Artificial Sequence

<220>
<223>  FR1 Vh m32H2

<400>  152

Glu Val Gln Leu Val Glu Ser Gly Gly Gly Ile Val Gln Pro Gly Gly
1               5                   10                  15

Ser Leu Lys Leu Ser Cys Ala Ala Ser Gly Phe Thr Phe Asn
            20                  25                  30

<210>  153
<211>  14
<212>  PRT
<213>  Artificial Sequence

<220>
<223>  FR2 VH m32H2

<400>  153

Trp Ile Arg Gln Thr Pro Asp Lys Arg Leu Glu Leu Val Ala
1               5                   10


<210>  154
<211>  33
<212>  PRT
<213>  Artificial Sequence

<220>
<223>  FR3 VH m32H2

<400>  154

Gly Arg Phe Ser Ile Ser Arg Asp Asn Ala Lys Lys Phe Val Tyr Leu
1               5                   10                  15


Gln Met Ser Ser Leu Lys Ser Asp Asp Thr Ala Ile Tyr Tyr Cys Pro
            20                  25                  30


Arg




<210>  155
<211>  11
<212>  PRT
<213>  Artificial Sequence

<220>
<223>  FR4 VH  m32H2

<400>  155

Trp Gly Gln Gly Thr Thr Leu Thr Val Ser Ser
1               5                   10


<210>  156
<211>  23
<212>  PRT
<213>  Artificial Sequence

<220>
<223>  FR1 VL  m32H2

<400>  156

Asp Ile Val Leu Thr Gln Ser Gln Lys Phe Met Ser Thr Ser Val Gly
1               5                   10                  15

Asp Arg Val Ser Ile Ile Cys
            20

<210>  157
<211>  15
<212>  PRT
<213>  Artificial Sequence

<220>
<223>  FR2 VL  m32H2

<400>  157

Trp Tyr Gln Leu Lys Ala Gly Gln Ser Pro Lys Leu Leu Ile Tyr
1               5                   10                  15

<210>  158
<211>  32
<212>  PRT
<213>  Artificial Sequence

<220>
<223>  FR3 VL  m32H2

<400>  158

Gly Val Pro Asp Arg Phe Thr Gly Ser Gly Ser Gly Thr Asp Phe Thr
1               5                   10                  15

Leu Thr Ile Ser Asn Val Gln Ser Glu Asp Leu Ala Asp Tyr Phe Cys
            20                  25                  30

<210>  159
<211>  10
<212>  PRT
<213>  Artificial Sequence

<220>
<223>  FR4 VL  m32H2

<400>  159

Phe Gly Ala Gly Thr Lys Leu Glu Leu Lys
1               5                   10

<210>  160
<211>  107
<212>  PRT
<213>  Artificial Sequence

<220>
<223>  VL  m32H2

<400>  160

Asp Ile Val Leu Thr Gln Ser Gln Lys Phe Met Ser Thr Ser Val Gly
1               5                   10                  15

Asp Arg Val Ser Ile Ile Cys Lys Ala Ser Gln Ser Val Gly Thr Ala
            20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys
            100                 105

<210>  161
<211>  114
<212>  PRT
<213>  Artificial Sequence

<220>
<223>  VH  m32H2

<400>  161

Glu Val Gln Leu Val Glu Ser Gly Gly Gly Ile Val Gln Pro Gly Gly
1               5                   10                  15

Ser Leu Lys Leu Ser Cys Ala Ala Ser Gly Phe Thr Phe Asn Asp Phe
            20                  25                  30

Gly Met Ser Trp Ile Arg Gln Thr Pro Asp Lys Arg Leu Glu Leu Val
        35                  40                  45

Ala Thr Ile Asn Thr Asn Gly Gly Thr Thr His Tyr Pro Asp Asn Val
    50                  55                  60

Lys Gly Arg Phe Ser Ile Ser Arg Asp Asn Ala Lys Lys Phe Val Tyr
65                  70                  75                  80

Leu Gln Met Ser Ser Leu Lys Ser Asp Asp Thr Ala Ile Tyr Tyr Cys
                85                  90                  95

Pro Arg Glu Gly Leu Asp Tyr Trp Gly Gln Gly Thr Thr Leu Thr Val
            100                 105                 110

Ser Ser

<210> 162
<211> 237
<212> PRT
<213> Artificial Sequence

<220>
<223> VL-VH m32H2

<400> 162

Asp Ile Val Leu Thr Gln Ser Gln Lys Phe Met Ser Thr Ser Val Gly
1               5                   10                  15

Asp Arg Val Ser Ile Ile Cys Lys Ala Ser Gln Ser Val Gly Thr Ala
            20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Ile Val Gln Pro Gly Gly Ser Leu Lys Leu Ser
    130                 135                 140

Cys Ala Ala Ser Gly Phe Thr Phe Asn Asp Phe Gly Met Ser Trp Ile
145                 150                 155                 160

Arg Gln Thr Pro Asp Lys Arg Leu Glu Leu Val Ala Thr Ile Asn Thr
                165                 170                 175

Asn Gly Gly Thr Thr His Tyr Pro Asp Asn Val Lys Gly Arg Phe Ser
            180                 185                 190

Ile Ser Arg Asp Asn Ala Lys Lys Phe Val Tyr Leu Gln Met Ser Ser
        195                 200                 205

Leu Lys Ser Asp Asp Thr Ala Ile Tyr Tyr Cys Pro Arg Glu Gly Leu
    210                 215                 220

Asp Tyr Trp Gly Gln Gly Thr Thr Leu Thr Val Ser Ser
225                 230                 235

<210>  163
<211>  30
<212>  PRT
<213>  Artificial Sequence

<220>
<223>  FR1 VH   H2-32H2

<400>  163

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn
            20                  25                  30

<210>  164
<211>  14
<212>  PRT
<213>  Artificial Sequence

<220>
<223>  FR2 VH  H2-32H2

<400>  164

Trp Val Arg Gln Ala Pro Gly Lys Gly Leu Glu Trp Val Ser
1               5                   10

<210>  165
<211>  33
<212>  PRT
<213>  Artificial Sequence

<220>
<223>  FR3 VH  H2-32H2

<400>  165

Gly Arg Phe Thr Ile Ser Arg Asp Asn Ser Lys Lys Phe Val Tyr Leu
1               5                   10                  15

Gln Met Asn Ser Leu Arg Ala Glu Asp Thr Ala Val Tyr Tyr Cys Pro
            20                  25                  30

Arg

<210>  166
<211>  11
<212>  PRT
<213>  Artificial Sequence

<220>
<223>  FR4 VH  H2-32H2

<400>  166

Trp Gly Gln Gly Thr Leu Val Thr Val Ser Ser
1               5                   10


<210>  167
<211>  23
<212>  PRT
<213>  Artificial Sequence

<220>
<223>  FR1 VL  H2-32H2

<400>  167

Glu Ile Val Leu Thr Gln Ser Pro Ala Thr Leu Ser Leu Ser Pro Gly
1               5                   10                  15


Glu Arg Ala Thr Leu Ser Cys
            20


<210>  168
<211>  15
<212>  PRT
<213>  Artificial Sequence

<220>
<223>  FR2 VL  H2-32H2

<400>  168

Trp Tyr Gln Leu Lys Pro Gly Gln Ala Pro Arg Leu Leu Ile Tyr
1               5                   10                  15


<210>  169
<211>  32
<212>  PRT
<213>  Artificial Sequence

<220>
<223>  FR3 VL  H2-32H2

<400>  169

Gly Ile Pro Ala Arg Phe Ser Gly Ser Gly Ser Gly Thr Asp Phe Thr
1               5                   10                  15

Leu Thr Ile Ser Ser Leu Glu Pro Glu Asp Phe Ala Val Tyr Tyr Cys
            20                  25                  30

<210>  170
<211>  10
<212>  PRT
<213>  Artificial Sequence

<220>
<223>  FR4 VL  H2-32H2

<400>  170

Phe Gly Gln Gly Thr Lys Leu Glu Ile Lys
1               5                   10

<210>  171
<211>  107
<212>  PRT
<213>  Artificial Sequence

<220>
<223>  VL  H2-32H2

<400>  171

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Leu Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys
            100                 105

<210>  172
<211>  114
<212>  PRT
<213>  Artificial Sequence

<220>
<223>  VH  H2-32H2

<400>  172

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Gly Met Ser Trp Val Arg Gln Ala Pro Gly Lys Gly Leu Glu Trp Val
        35                  40                  45

Ser Thr Ile Asn Thr Asn Gly Gly Thr Thr His Tyr Pro Asp Ser Val
    50                  55                  60

Lys Gly Arg Phe Thr Ile Ser Arg Asp Asn Ser Lys Lys Phe Val Tyr
65                  70                  75                  80

Leu Gln Met Asn Ser Leu Arg Ala Glu Asp Thr Ala Val Tyr Tyr Cys
                85                  90                  95

Pro Arg Glu Gly Leu Asp Tyr Trp Gly Gln Gly Thr Leu Val Thr Val
            100                 105                 110

Ser Ser

<210>  173
<211>  237
<212>  PRT
<213>  Artificial Sequence

<220>
<223>  VL-VH  H2-32H2

<400>  173

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Leu Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Asn Asp Phe Gly Met Ser Trp Val
145                 150                 155                 160

Arg Gln Ala Pro Gly Lys Gly Leu Glu Trp Val Ser Thr Ile Asn Thr
                165                 170                 175

Asn Gly Gly Thr Thr His Tyr Pro Asp Ser Val Lys Gly Arg Phe Thr
            180                 185                 190

Ile Ser Arg Asp Asn Ser Lys Lys Phe Val Tyr Leu Gln Met Asn Ser
        195                 200                 205

Leu Arg Ala Glu Asp Thr Ala Val Tyr Tyr Cys Pro Arg Glu Gly Leu
    210                 215                 220

Asp Tyr Trp Gly Gln Gly Thr Leu Val Thr Val Ser Ser
225                 230                 235

<210>  174
<211>  17
<212>  PRT
<213>  Artificial Sequence

<220>
<223>  CDR2 VH  H1-32H2

<400>  174

Thr Ile Asn Thr Asn Gly Gly Thr Thr His Tyr Pro Asp Ser Val Lys
1               5                   10                  15

Gly

<210>  175
<211>  11
<212>  PRT

<213>  Artificial Sequence

<220>
<223>  CDR1 VL  H1-32H2

<400>  175

Arg Ala Ser Gln Ser Val Gly Thr Ala Val Ala
1               5                   10


<210>  176
<211>  5
<212>  PRT
<213>  Artificial Sequence

<220>
<223>  CDR1 VH  H3-214D8

<400>  176

Ser Tyr Gly Met Ala
1               5


<210>  177
<211>  17
<212>  PRT
<213>  Artificial Sequence

<220>
<223>  CDR2 VH  H3-214D8

<400>  177

Ala Ile Asn Ser Asn Gly Gly Lys Thr Tyr Tyr Ala Asp Ser Val Lys
1               5                   10                  15


Gly




<210>  178

<400>  178
000

<210>  179
<211>  11
<212>  PRT
<213>  Artificial Sequence

<220>
<223>  CDR1 VL  H2-215C2

<400>  179

Arg Ala Ser Gln Asn Val Gly Thr Ala Val Ala
1               5                   10


<210>  180

<211>  107
<212>  PRT
<213>  Artificial Sequence

<220>
<223>  VL ScFv1 H1-32H2 (CDR2 174)

<400>  180

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys
            100                 105


<210>  181
<211>  115
<212>  PRT
<213>  Artificial Sequence

<220>
<223>  VH  H1-32H2 CDR1 175

<400>  181

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Gly Met Ser Trp Val Arg Gln Ala Pro Gly Lys Gly Leu Glu Trp Val
        35                  40                  45

Ser Thr Ile Asn Thr Asn Gly Gly Thr Thr His Tyr Pro Asp Ser Val
    50                  55                  60

Lys Gly Gly Arg Phe Thr Ile Ser Arg Asp Asn Ser Lys Asn Thr Leu
65                  70                  75                  80

Tyr Leu Gln Met Asn Ser Leu Arg Ala Glu Asp Thr Ala Val Tyr Tyr
                85                  90                  95

Cys Ala Lys Glu Gly Leu Asp Tyr Trp Gly Gln Gly Thr Leu Val Thr
            100                 105                 110

Val Ser Ser
        115

<210>  182
<211>  238
<212>  PRT
<213>  Artificial Sequence

<220>
<223>  VL-VH  H1-32H2 (VL CDR2 174 and VH CDR1 175)

<400>  182

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Thr Ala
            20                  25                  30

Val Ala Trp Tyr Gln Gln Lys Pro Gly Gln Ala Pro Arg Leu Leu Ile
        35                  40                  45

Tyr Ser Ala Ser Asn Arg Phe Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Tyr Ser Thr Tyr Pro Leu
                85                  90                  95

Ala Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Asn Asp Phe Gly Met Ser Trp Val

145                 150                 155                 160

Arg Gln Ala Pro Gly Lys Gly Leu Glu Trp Val Ser Thr Ile Asn Thr
                165                 170                 175

Asn Gly Gly Thr Thr His Tyr Pro Asp Ser Val Lys Gly Gly Arg Phe
            180                 185                 190

Thr Ile Ser Arg Asp Asn Ser Lys Asn Thr Leu Tyr Leu Gln Met Asn
        195                 200                 205

Ser Leu Arg Ala Glu Asp Thr Ala Val Tyr Tyr Cys Ala Lys Glu Gly
    210                 215                 220

Leu Asp Tyr Trp Gly Gln Gly Thr Leu Val Thr Val Ser Ser
225                 230                 235

<210>  183
<211>  111
<212>  PRT
<213>  Artificial Sequence

<220>
<223>  VH  214D8 (CDR1, CDR2, CDR3 176, 177, 178)

<400>  183

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ala Trp Val Arg Gln Thr Pro Asp Arg Arg Leu Glu Leu Val
        35                  40                  45

Ala Ala Ile Asn Ser Asn Gly Gly Lys Thr Tyr Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Thr Arg Asp Tyr Trp Gly Gln Gly Thr Thr Leu Thr Val Ser Ser
            100                 105                 110

<210>  184
<211>  107

<212>   PRT
<213>   Artificial Sequence

<220>
<223>   VL 215C2 (CDR 179)

<400>   184

Asp Ile Val Met Thr Gln Ser Gln Lys Phe Met Ser Thr Ser Glu Gly
1               5                   10                  15

Asp Arg Val Ser Ile Thr Cys Arg Ala Ser Gln Asn Val Gly Thr Ala
            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys
            100                 105


<210>   185
<211>   236
<212>   PRT
<213>   Artificial Sequence

<220>
<223>   VL-VH 214D8 (VH 183)


<220>
<221>   misc_feature
<222>   (221)..(223)
<223>   Xaa can be any naturally occurring amino acid

<400>   185

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Ile Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Lys Ser
65                  70                  75                  80

Glu Asp Leu Ala Asp Tyr Phe Cys Gln Gln Tyr Ser Thr Tyr Pro Leu
                85                  90                  95

Thr Phe Gly Ala Gly Thr Lys Leu Glu Leu Lys Thr Gly Ser Thr Ser
            100                 105                 110

Gly Ser Gly Lys Pro Gly Ser Gly Glu Gly Glu Val Gln Leu Val Glu
        115                 120                 125

Ser Gly Gly Gly Leu Val Gln Pro Gly Gly Ser Leu Lys Leu Ser Cys
    130                 135                 140

Ala Ala Ser Gly Phe Thr Phe Ser Ser Tyr Gly Met Ala Trp Val Arg
145                 150                 155                 160

Gln Thr Pro Asp Arg Arg Leu Glu Leu Val Ala Ala Ile Asn Ser Asn
                165                 170                 175

Gly Gly Lys Thr Tyr Tyr Ala Asp Ser Val Lys Gly Arg Phe Thr Ile
            180                 185                 190

Ser Arg Asp Asn Ala Lys Asn Thr Leu Tyr Leu Gln Met Ser Ser Leu
        195                 200                 205

Lys Ser Glu Asp Thr Ala Met Tyr Tyr Cys Thr Arg Xaa Xaa Xaa Asp
    210                 215                 220

Tyr Trp Gly Gln Gly Thr Thr Leu Thr Val Ser Ser
225                 230                 235

<210>  186
<211>  237
<212>  PRT
<213>  Artificial Sequence

<220>
<223>  VL-VH 215C2 (VL 184)

<400>  186

Asp Ile Val Met Thr Gln Ser Gln Lys Phe Met Ser Thr Ser Glu Gly
1               5                   10                  15

Asp Arg Val Ser Ile Thr Cys Arg Ala Ser Gln Asn Val Gly Thr Ala
            20                  25                  30

Val Ala Trp Tyr Gln Gln Lys Pro Gly Gln Ser Pro Lys Val Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Thr Leu Glu Leu Lys Thr Gly Ser Thr Ser
            100                 105                 110

Gly Ser Gly Lys Pro Gly Ser Gly Glu Gly Ser Glu Val His Leu Val
        115                 120                 125

Glu Ser Gly Gly Gly Leu Val Gln Pro Gly Gly Ser Leu Lys Leu Ser
    130                 135                 140

Cys Ala Ala Ser Gly Phe Thr Phe Ser Asp Tyr Gly Met Ser Trp Val
145                 150                 155                 160

Arg Gln Thr Pro Asp Lys Arg Leu Glu Leu Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ala Lys Asn Thr Leu Tyr Leu Gln Met Asn Ser
        195                 200                 205

Leu Lys Ser Glu Asp Thr Ala Ile Tyr Tyr Cys Pro Arg Glu Gly Phe
    210                 215                 220

Asp Tyr Trp Gly Gln Gly Thr Thr Leu Thr Val Ser Ser
225                 230                 235

<210>  187
<211>  237
<212>  PRT
<213>  Artificial Sequence

<220>
<223>  VL-VH  H1-214D8

<400>  187

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Thr Tyr Gly Met Ala Trp Val
145                 150                 155                 160

Arg Gln Thr Pro Asp Arg Arg Leu Glu Leu Val Ala Thr Ile Asn Ser
                165                 170                 175

Asn Gly Gly Lys Thr Tyr His Pro Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Thr Arg Glu Gly Phe
    210                 215                 220

Asp Tyr Trp Gly Gln Gly Thr Leu Val Thr Val Ser Ser
225                 230                 235

<210>  188
<211>  237
<212>  PRT
<213>  Artificial Sequence

<220>
<223>  VL-VH  H2-214D8

<400>  188

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Thr Tyr Gly Met Ala Trp Val
145                 150                 155                 160

Arg Gln Ala Pro Gly Lys Gly Leu Glu Trp Val Ser Thr Ile Asn Ser
                165                 170                 175

Asn Gly Gly Lys Thr Tyr His Pro Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Thr Arg Glu Gly Phe

210                 215                 220

Asp Tyr Trp Gly Gln Gly Thr Leu Val Thr Val Ser Ser
225                 230                 235

<210>  189
<211>  234
<212>  PRT
<213>  Artificial Sequence

<220>
<223>  VL-VH  H3-214D8

<400>  189

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Ile Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Ser Tyr Gly Met Ala Trp Val
145                 150                 155                 160

Arg Gln Ala Pro Gly Lys Gly Leu Glu Trp Val Ser Ala Ile Asn Ser
                165                 170                 175

Asn Gly Gly Lys Thr Tyr Tyr Ala Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Ala Lys Asp Tyr Trp
    210                 215                 220

Gly Gln Gly Thr Leu Val Thr Val Ser Ser
225                 230

<210>  190
<211>  237
<212>  PRT
<213>  Artificial Sequence

<220>
<223>  VL-VH  H1-215C2

<400>  190

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Ala
            20                  25                  30

Val Ala Trp Phe Gln Gln Lys Pro Gly Lys Ala Pro Lys Val Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Asp Tyr Gly Met Ser Trp Val
145                 150                 155                 160

Arg Gln Ala Pro Gly Lys Arg Leu Glu Leu Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Pro Arg Glu Gly Phe
    210                 215                 220

Asp Tyr Trp Gly Gln Gly Thr Leu Val Thr Val Ser Ser
225                 230                 235

<210>  191
<211>  237
<212>  PRT
<213>  Artificial Sequence

<220>
<223>  VL-VH  H2-215C2

<400>  191

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala
            20                  25                  30

Val Ala Trp Phe Gln Gln Lys Pro Gly Lys Ala Pro Lys Ser Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Asp Tyr Gly Met Ser Trp Val
145                 150                 155                 160

Arg Gln Ala Pro Gly Lys Gly Leu Glu Trp Val Ala Thr Ile Asn Gly
                165                 170                 175

Asn Gly Val Lys Ile Tyr Tyr Val Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Pro Arg Glu Gly Phe
    210                 215                 220

Asp Tyr Trp Gly Gln Gly Thr Leu Val Thr Val Ser Ser
225                 230                 235

<210>  192
<211>  221
<212>  PRT
<213>  Artificial Sequence

<220>
<223>  VL-VH  H3-215C2

<400>  192

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Gly Thr Ala
            20                  25                  30

Val Ala Trp Phe Gln Gln Lys Pro Gly Lys Ala Pro Lys Ser Leu Ile
        35                  40                  45

Tyr Ser Ala Ser Asn Arg Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Ser Tyr Pro Leu
                85                  90                  95

Thr Phe Gly Gln Gly Thr Lys Leu Glu Ile Lys Thr Gly Ser Thr Ser

            100                 105                 110

Gly Ser Gly Lys Pro Gly Ser Gly Glu Gly Ser Glu Val Gln Leu Val
        115                 120                 125

Glu Ser Gly Gly Gly Leu Val Gln Pro Gly Gly Ser Leu Arg Leu Ser
    130                 135                 140

Cys Ala Ala Ser Gly Phe Thr Phe Ser Asp Tyr Gly Met Ser Trp Val
145                 150                 155                 160

Arg Gln Ala Pro Gly Lys Gly Leu Glu Trp Val Ala Thr Ile Asn Gly
                165                 170                 175

Asn Gly Val Lys Thr Tyr Tyr Ala Asp Ser Val Lys Gly Arg Phe Thr
            180                 185                 190

Ile Ser Arg Asp Asn Ser Lys Asn Thr Leu Tyr Leu Gln Met Asn Ser
        195                 200                 205

Leu Arg Ala Glu Asp Thr Ala Val Tyr Tyr Cys Pro Arg
    210                 215                 220

<210> 193
<211> 107
<212> PRT
<213> Artificial Sequence

<220>
<223> VL m32H2 (CDR1 VL 175)

<400> 193

Asp Ile Val Leu Thr Gln Ser Gln Lys Phe Met Ser Thr Ser Val Gly
1               5                   10                  15

Asp Arg Val Ser Ile Ile Cys Arg Ala Ser Gln Ser Val Gly Thr Ala
            20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys
            100                 105

<210>  194
<211>  115
<212>  PRT
<213>  Artificial Sequence

<220>
<223>  VH  m32H2 (CDR2 VH 174)

<400>  194

Glu Val Gln Leu Val Glu Ser Gly Gly Gly Ile Val Gln Pro Gly Gly
1               5                   10                  15

Ser Leu Lys Leu Ser Cys Ala Ala Ser Gly Phe Thr Phe Asn Asp Phe
            20                  25                  30

Gly Met Ser Trp Ile Arg Gln Thr Pro Asp Lys Arg Leu Glu Leu Val
        35                  40                  45

Ala Thr Ile Asn Thr Asn Gly Gly Thr Thr His Tyr Pro Asp Ser Val
    50                  55                  60

Lys Gly Gly Arg Phe Ser Ile Ser Arg Asp Asn Ala Lys Lys Phe Val
65                  70                  75                  80

Tyr Leu Gln Met Ser Ser Leu Lys Ser Asp Asp Thr Ala Ile Tyr Tyr
                85                  90                  95

Cys Pro Arg Glu Gly Leu Asp Tyr Trp Gly Gln Gly Thr Thr Leu Thr
            100                 105                 110

Val Ser Ser
        115

<210>  195
<211>  238
<212>  PRT
<213>  Artificial Sequence

<220>
<223>  VL-VH m32H2 (193 and 194)

<400>  195

Asp Ile Val Leu Thr Gln Ser Gln Lys Phe Met Ser Thr Ser Val Gly
1               5                   10                  15

Asp Arg Val Ser Ile Ile Cys Arg Ala Ser Gln Ser Val Gly Thr Ala

20                  25                  30

Val Ala Trp Tyr Gln Leu Lys Ala Gly Gln Ser Pro Lys Leu Leu Ile
        35                  40                  45

Tyr Ser Ala Ser Asn Arg Phe Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Thr Tyr Pro Leu
                85                  90                  95

Ala Phe Gly Ala Gly Thr Lys Leu Glu Leu Lys Thr Gly Ser Thr Ser
            100                 105                 110

Gly Ser Gly Lys Pro Gly Ser Gly Glu Gly Ser Glu Val Gln Leu Val
        115                 120                 125

Glu Ser Gly Gly Gly Ile Val Gln Pro Gly Gly Ser Leu Lys Leu Ser
    130                 135                 140

Cys Ala Ala Ser Gly Phe Thr Phe Asn Asp Phe Gly Met Ser Trp Ile
145                 150                 155                 160

Arg Gln Thr Pro Asp Lys Arg Leu Glu Leu Val Ala Thr Ile Asn Thr
                165                 170                 175

Asn Gly Gly Thr Thr His Tyr Pro Asp Ser Val Lys Gly Gly Arg Phe
            180                 185                 190

Ser Ile Ser Arg Asp Asn Ala Lys Lys Phe Val Tyr Leu Gln Met Ser
        195                 200                 205

Ser Leu Lys Ser Asp Asp Thr Ala Ile Tyr Tyr Cys Pro Arg Glu Gly
    210                 215                 220

Leu Asp Tyr Trp Gly Gln Gly Thr Thr Leu Thr Val Ser Ser
225                 230                 235

<210>  196
<211>  481
<212>  PRT
<213>  Artificial Sequence

<220>
<223>  H3 21408 CAR

<400>  196

Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu
            20                  25                  30

Ser Ala Ser Val Gly Asp Arg Val Thr Ile Thr Cys Lys Ala Ser Gln
        35                  40                  45

Asn Val Gly Thr Ala Val Ala Trp Phe Gln Gln Lys Pro Gly Lys Ala
    50                  55                  60

Pro Lys Ile Leu Ile Tyr Ser Ala Ser Asn Arg Tyr Thr Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Tyr
            100                 105                 110

Ser Thr Tyr Pro Leu Thr Phe Gly Gln Gly Thr Lys Leu Glu Ile Lys
        115                 120                 125

Thr Gly Ser Thr Ser Gly Ser Gly Lys Pro Gly Ser Gly Glu Gly Ser
    130                 135                 140

Glu Val Gln Leu Val Glu Ser Gly Gly Gly Leu Val Gln Pro Gly Gly
145                 150                 155                 160

Ser Leu Arg Leu Ser Cys Ala Ala Ser Gly Phe Thr Phe Ser Ser Tyr
                165                 170                 175

Gly Met Ala Trp Val Arg Gln Ala Pro Gly Lys Gly Leu Glu Trp Val
            180                 185                 190

Ser Ala Ile Asn Ser Asn Gly Gly Lys Thr Tyr Tyr Ala Asp Ser Val
        195                 200                 205

Lys Gly Arg Phe Thr Ile Ser Arg Asp Asn Ser Lys Asn Thr Leu Tyr
    210                 215                 220

Leu Gln Met Asn Ser Leu Arg Ala Glu Asp Thr Ala Val Tyr Tyr Cys
225                 230                 235                 240

Ala Lys Asp Tyr Trp Gly Gln Gly Thr Leu Val Thr Val Ser Ser Thr

                245                 250                 255

Thr Thr Pro Ala Pro Arg Pro Pro Thr Pro Ala Pro Thr Ile Ala Ser
            260                 265                 270

Gln Pro Leu Ser Leu Arg Pro Glu Ala Cys Arg Pro Ala Ala Gly Gly
        275                 280                 285

Ala Val His Thr Arg Gly Leu Asp Phe Ala Cys Asp Phe Trp Val Leu
    290                 295                 300

Val Val Val Gly Gly Val Leu Ala Cys Tyr Ser Leu Leu Val Thr Val
305                 310                 315                 320

Ala Phe Ile Ile Phe Trp Val Arg Ser Lys Arg Ser Arg Leu Leu His
                325                 330                 335

Ser Asp Tyr Met Asn Met Thr Pro Arg Arg Pro Gly Pro Thr Arg Lys
            340                 345                 350

His Tyr Gln Pro Tyr Ala Pro Pro Arg Asp Phe Ala Ala Tyr Arg Ser
        355                 360                 365

Arg Val Lys Phe Ser Arg Ser Ala Asp Ala Pro Ala Tyr Gln Gln Gly
    370                 375                 380

Gln Asn Gln Leu Tyr Asn Glu Leu Asn Leu Gly Arg Arg Glu Glu Tyr
385                 390                 395                 400

Asp Val Leu Asp Lys Arg Arg Gly Arg Asp Pro Glu Met Gly Gly Lys
                405                 410                 415

Pro Arg Arg Lys Asn Pro Gln Glu Gly Leu Tyr Asn Glu Leu Gln Lys
            420                 425                 430

Asp Lys Met Ala Glu Ala Tyr Ser Glu Ile Gly Met Lys Gly Glu Arg
        435                 440                 445

Arg Arg Gly Lys Gly His Asp Gly Leu Tyr Gln Gly Leu Ser Thr Ala
    450                 455                 460

Thr Lys Asp Thr Tyr Asp Ala Leu His Met Asx Gln Ala Leu Pro Pro
465                 470                 475                 480

Arg

<210>  197
<211>  483
<212>  PRT
<213>  Artificial Sequence

<220>
<223>  215C2 CAR

<400>  197

Met Ala Leu Pro Val Thr Ala Leu Leu Leu Pro Leu Ala Leu Leu Leu
1               5                   10                  15

His Ala Ala Arg Pro Asp Ile Val Met Thr Gln Ser Gln Lys Phe Met
            20                  25                  30

Ser Thr Ser Glu Gly Asp Arg Val Ser Ile Thr Cys Lys Ala Ser Gln
        35                  40                  45

Asn Val Gly Thr Ala Val Ala Trp Tyr Gln Gln Lys Pro Gly Gln Ser
    50                  55                  60

Pro Lys Val Leu Ile Tyr Ser Ala Ser Asn Arg Tyr Thr Gly Val Pro
65                  70                  75                  80

Asp Arg Phe Thr Gly Ser Gly Ser Gly Thr Asp Phe Thr Leu Thr Ile
                85                  90                  95

Ser Asn Met Gln Ser Glu Asp Leu Ala Asp Tyr Phe Cys Gln Gln Tyr
            100                 105                 110

Ser Ser Tyr Pro Leu Thr Phe Gly Ala Gly Thr Thr Leu Glu Leu Lys
        115                 120                 125

Thr Gly Ser Thr Ser Gly Ser Gly Lys Pro Gly Ser Gly Glu Gly Ser
    130                 135                 140

Glu Val His Leu Val Glu Ser Gly Gly Gly Leu Val Gln Pro Gly Gly
145                 150                 155                 160

Ser Leu Lys Leu Ser Cys Ala Ala Ser Gly Phe Thr Phe Ser Asp Tyr
                165                 170                 175

Gly Met Ser Trp Val Arg Gln Thr Pro Asp Lys Arg Leu Glu Leu Val
            180                 185                 190

Ala Thr Ile Asn Gly Asn Gly Val Lys Ile Tyr Tyr Val Asp Ser Val
        195                 200                 205

Lys Gly Arg Phe Thr Ile Ser Arg Asp Asn Ala Lys Asn Thr Leu Tyr

210                 215                 220

Leu Gln Met Asn Ser Leu Lys Ser Glu Asp Thr Ala Ile Tyr Tyr Cys
225                 230                 235                 240

Pro Arg Glu Gly Phe Asp Tyr Trp Gly Gln Gly Thr Thr Leu Thr Val
                245                 250                 255

Ser Ser Thr Thr Thr Pro Ala Pro Arg Pro Pro Thr Pro Ala Pro Thr
            260                 265                 270

Ile Ala Ser Gln Pro Leu Ser Leu Arg Pro Glu Ala Cys Arg Pro Ala
        275                 280                 285

Ala Gly Gly Ala Val His Thr Arg Gly Leu Asp Phe Ala Cys Asp Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210>  198
<211>  482
<212>  PRT
<213>  Artificial Sequence

<220>
<223>  H1215C2 CAR

<400>  198

Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu
            20                  25                  30

Ser Ala Ser Val Gly Asp Arg Val Thr Ile Thr Cys Lys Ala Ser Gln
        35                  40                  45

Asn Val Gly Thr Ala Val Ala Trp Phe Gln Gln Lys Pro Gly Lys Ala
    50                  55                  60

Pro Lys Val Leu Ile Tyr Ser Ala Ser Asn Arg Tyr Thr Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Tyr
            100                 105                 110

Ser Ser Tyr Pro Leu Thr Phe Gly Gln Gly Thr Lys Leu Glu Ile Lys
        115                 120                 125

Thr Gly Ser Thr Ser Gly Ser Gly Lys Pro Gly Ser Gly Glu Gly Ser
    130                 135                 140

Glu Val Gln Leu Val Glu Ser Gly Gly Gly Leu Val Gln Pro Gly Gly
145                 150                 155                 160

Ser Leu Arg Leu Ser Cys Ala Ala Ser Gly Phe Thr Phe Ser Asp Tyr
                165                 170                 175

Gly Met Ser Trp Val Arg Gln Ala Pro Gly Lys Arg Leu Glu Leu Val

180                 185                 190

Ala Thr Ile Asn Gly Asn Gly Val Lys Ile Tyr Tyr Val Asp Ser Val
        195                 200                 205

Lys Gly Arg Phe Thr Ile Ser Arg Asp Asn Ser Lys Asn Thr Leu Tyr
    210                 215                 220

Leu Gln Met Asn Ser Leu Arg Ala Glu Asp Thr Ala Val Tyr Tyr Cys
225                 230                 235                 240

Pro Arg Glu Gly Phe Asp Tyr Trp Gly Gln Gly Thr Leu Val Thr Val
                245                 250                 255

Ser Ser Thr Thr Thr Pro Ala Pro Arg Pro Pro Thr Pro Ala Pro Thr
            260                 265                 270

Ile Ala Ser Gln Pro Leu Ser Leu Arg Pro Glu Ala Cys Arg Pro Ala
        275                 280                 285

Ala Gly Gly Ala Val His Thr Arg Cys Leu Asp Phe Ala Cys Asp Phe
    290                 295                 300

Trp Val Leu Val Val Gly Gly Val Leu Ala Cys Tyr Ser Leu Leu Val
305                 310                 315                 320

Thr Val Ala Phe Ile Ile Phe Trp Val Arg Ser Lys Arg Ser Arg Leu
                325                 330                 335

Leu His Ser Asp Tyr Met Asn Met Thr Pro Arg Arg Pro Gly Pro Thr
            340                 345                 350

Arg Lys His Tyr Gln Pro Tyr Ala Pro Pro Arg Asp Phe Ala Ala Tyr
        355                 360                 365

Arg Ser Arg Val Lys Phe Ser Arg Ser Ala Asp Ala Pro Ala Tyr Gln
    370                 375                 380

Gln Gly Gln Asn Gln Leu Tyr Asn Glu Leu Asn Leu Gly Arg Arg Glu
385                 390                 395                 400

Glu Tyr Asp Val Leu Asp Lys Arg Arg Gly Arg Asp Pro Glu Met Gly
                405                 410                 415

Gly Lys Pro Arg Arg Lys Asn Pro Gln Glu Gly Leu Tyr Asn Glu Leu
            420                 425                 430

Gln Lys Asp Lys Met Ala Glu Ala Tyr Ser Glu Ile Gly Met Lys Gly
        435                 440                 445

Glu Arg Arg Arg Gly Lys Gly His Asp Gly Leu Tyr Gln Gly Leu Ser
    450                 455                 460

Thr Ala Thr Lys Asp Thr Tyr Asp Ala Leu His Met Gln Ala Leu Pro
465                 470                 475                 480

Pro Arg

<210>  199
<211>  483
<212>  PRT
<213>  Artificial Sequence

<220>
<223>  H2 215C2 CAR

<400>  199

Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu
            20                  25                  30

Ser Ala Ser Val Gly Asp Arg Val Thr Ile Thr Cys Arg Ala Ser Gln
        35                  40                  45

Asn Val Gly Thr Ala Val Ala Trp Phe Gln Gln Lys Pro Gly Lys Ala
    50                  55                  60

Pro Lys Ser Leu Ile Tyr Ser Ala Ser Asn Arg Tyr Thr Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Tyr
            100                 105                 110

Ser Ser Tyr Pro Leu Thr Phe Gly Gln Gly Thr Lys Leu Glu Ile Lys
        115                 120                 125

Thr Gly Ser Thr Ser Gly Ser Gly Lys Pro Gly Ser Gly Glu Gly Ser
    130                 135                 140

Glu Val Gln Leu Val Glu Ser Gly Gly Gly Leu Val Gln Pro Gly Gly

145                 150                 155                 160

Ser Leu Arg Leu Ser Cys Ala Ala Ser Gly Phe Thr Phe Ser Asp Tyr
                165                 170                 175

Gly Met Ser Trp Val Arg Gln Ala Pro Gly Lys Gly Leu Glu Trp Val
            180                 185                 190

Ala Thr Ile Asn Gly Asn Gly Val Lys Ile Tyr Tyr Val Asp Ser Val
        195                 200                 205

Lys Gly Arg Phe Thr Ile Ser Arg Asp Asn Ser Lys Asn Thr Leu Tyr
    210                 215                 220

Leu Gln Met Asn Ser Leu Arg Ala Glu Asp Thr Ala Val Tyr Tyr Cys
225                 230                 235                 240

Pro Arg Glu Gly Phe Asp Tyr Trp Gly Gln Gly Thr Leu Val Thr Val
                245                 250                 255

Ser Ser Thr Thr Thr Pro Ala Pro Arg Pro Pro Thr Pro Ala Pro Thr
            260                 265                 270

Ile Ala Ser Gln Pro Leu Ser Leu Arg Pro Glu Ala Cys Arg Pro Ala
        275                 280                 285

Ala Gly Gly Ala Val His Thr Arg Gly Leu Asp Phe Ala Cys Asp Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210>  200
<211>  480
<212>  PRT
<213>  Artificial Sequence

<220>
<223>  H3 214D8 CAR

<400>  200

Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu
            20                  25                  30

Ser Ala Ser Val Gly Asp Arg Val Thr Ile Thr Cys Lys Ala Ser Gln
        35                  40                  45

Asn Val Gly Thr Ala Val Ala Trp Phe Gln Gln Lys Pro Gly Lys Ala
    50                  55                  60

Pro Lys Ile Leu Ile Tyr Ser Ala Ser Asn Arg Tyr Thr Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Tyr
            100                 105                 110

Ser Thr Tyr Pro Leu Thr Phe Gly Gln Gly Thr Lys Leu Glu Ile Lys

115                 120                 125

Thr Gly Ser Thr Ser Gly Ser Gly Lys Pro Gly Ser Gly Glu Gly Ser
    130                 135                 140

Glu Val Gln Leu Val Glu Ser Gly Gly Gly Leu Val Gln Pro Gly Gly
145                 150                 155                 160

Ser Leu Arg Leu Ser Cys Ala Ala Ser Gly Phe Thr Phe Ser Ser Tyr
                165                 170                 175

Gly Met Ala Trp Val Arg Gln Ala Pro Gly Lys Gly Leu Glu Trp Val
            180                 185                 190

Ser Ala Ile Asn Ser Asn Gly Gly Lys Thr Tyr Tyr Ala Asp Ser Val
        195                 200                 205

Lys Gly Arg Phe Thr Ile Ser Arg Asp Asn Ser Lys Asn Thr Leu Tyr
    210                 215                 220

Leu Gln Met Asn Ser Leu Arg Ala Glu Asp Thr Ala Val Tyr Tyr Cys
225                 230                 235                 240

Ala Lys Asp Tyr Trp Gly Gln Gly Thr Leu Val Thr Val Ser Ser Thr
                245                 250                 255

Thr Thr Pro Ala Pro Arg Pro Pro Thr Pro Ala Pro Thr Ile Ala Ser
            260                 265                 270

Gln Pro Leu Ser Leu Arg Pro Glu Ala Cys Arg Pro Ala Ala Gly Gly
        275                 280                 285

Ala Val His Thr Arg Gly Leu Asp Phe Ala Cys Asp Phe Trp Val Leu
    290                 295                 300

Val Val Val Gly Gly Val Leu Ala Cys Tyr Ser Leu Leu Val Thr Val
305                 310                 315                 320

Ala Phe Ile Ile Phe Trp Val Arg Ser Lys Arg Ser Arg Leu Leu His
                325                 330                 335

Ser Asp Tyr Met Asn Met Thr Pro Arg Arg Pro Gly Pro Thr Arg Lys
            340                 345                 350

His Tyr Gln Pro Tyr Ala Pro Pro Arg Asp Phe Ala Ala Tyr Arg Ser
        355                 360                 365

Arg Val Lys Phe Ser Arg Ser Ala Asp Ala Pro Ala Tyr Gln Gln Gly
    370                 375                 380

Gln Asn Gln Leu Tyr Asn Glu Leu Asn Leu Gly Arg Arg Glu Glu Tyr
385                 390                 395                 400

Asp Val Leu Asp Lys Arg Arg Gly Arg Asp Pro Glu Met Gly Gly Lys
                405                 410                 415

Pro Arg Arg Lys Asn Pro Gln Glu Gly Leu Tyr Asn Glu Leu Gln Lys
            420                 425                 430

Asp Lys Met Ala Glu Ala Tyr Ser Glu Ile Gly Met Lys Gly Glu Arg
        435                 440                 445

Arg Arg Gly Lys Gly His Asp Gly Leu Tyr Gln Gly Leu Ser Thr Ala
    450                 455                 460

Thr Lys Asp Thr Tyr Asp Ala Leu His Met Gln Ala Leu Pro Pro Arg
465                 470                 475                 480

<210>  201
<211>  483
<212>  PRT
<213>  Artificial Sequence

<220>
<223>  215C2 CAR

<400>  201

Met Ala Leu Pro Val Thr Ala Leu Leu Leu Pro Leu Ala Leu Leu Leu
1               5                   10                  15

His Ala Ala Arg Pro Asp Ile Val Met Thr Gln Ser Gln Lys Phe Met
            20                  25                  30

Ser Thr Ser Glu Gly Asp Arg Val Ser Ile Thr Cys Lys Ala Ser Gln
        35                  40                  45

Asn Val Gly Thr Ala Val Ala Trp Tyr Gln Gln Lys Pro Gly Gln Ser
    50                  55                  60

Pro Lys Val Leu Ile Tyr Ser Ala Ser Asn Arg Tyr Thr Gly Val Pro
65                  70                  75                  80

Asp Arg Phe Thr Gly Ser Gly Ser Gly Thr Asp Phe Thr Leu Thr Ile
                85                  90                  95

Ser Asn Met Gln Ser Glu Asp Leu Ala Asp Tyr Phe Cys Gln Gln Tyr

            100                 105                 110

Ser Ser Tyr Pro Leu Thr Phe Gly Ala Gly Thr Thr Leu Glu Leu Lys
        115                 120                 125

Thr Gly Ser Thr Ser Gly Ser Gly Lys Pro Gly Ser Gly Glu Gly Ser
    130                 135                 140

Glu Val His Leu Val Glu Ser Gly Gly Gly Leu Val Gln Pro Gly Gly
145                 150                 155                 160

Ser Leu Lys Leu Ser Cys Ala Ala Ser Gly Phe Thr Phe Ser Asp Tyr
                165                 170                 175

Gly Met Ser Trp Val Arg Gln Thr Pro Asp Lys Arg Leu Glu Leu Val
            180                 185                 190

Ala Thr Ile Asn Gly Asn Gly Val Lys Ile Tyr Tyr Val Asp Ser Val
        195                 200                 205

Lys Gly Arg Phe Thr Ile Ser Arg Asp Asn Ala Lys Asn Thr Leu Tyr
    210                 215                 220

Leu Gln Met Asn Ser Leu Lys Ser Glu Asp Thr Ala Ile Tyr Tyr Cys
225                 230                 235                 240

Pro Arg Glu Gly Phe Asp Tyr Trp Gly Gln Gly Thr Thr Leu Thr Val
                245                 250                 255

Ser Ser Thr Thr Thr Pro Ala Pro Arg Pro Pro Thr Pro Ala Pro Thr
            260                 265                 270

Ile Ala Ser Gln Pro Leu Ser Leu Arg Pro Glu Ala Cys Arg Pro Ala
        275                 280                 285

Ala Gly Gly Ala Val His Thr Arg Gly Leu Asp Phe Ala Cys Asp Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210>  202
<211>  483
<212>  PRT
<213>  Artificial Sequence

<220>
<223>  H1 215C2 CAR

<400>  202

Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu
            20                  25                  30

Ser Ala Ser Val Gly Asp Arg Val Thr Ile Thr Cys Lys Ala Ser Gln
        35                  40                  45

Asn Val Gly Thr Ala Val Ala Trp Phe Gln Gln Lys Pro Gly Lys Ala
    50                  55                  60

Pro Lys Val Leu Ile Tyr Ser Ala Ser Asn Arg Tyr Thr Gly Val Pro

65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Tyr
            100                 105                 110

Ser Ser Tyr Pro Leu Thr Phe Gly Gln Gly Thr Lys Leu Glu Ile Lys
        115                 120                 125

Thr Gly Ser Thr Ser Gly Ser Gly Lys Pro Gly Ser Gly Glu Gly Ser
    130                 135                 140

Glu Val Gln Leu Val Glu Ser Gly Gly Gly Leu Val Gln Pro Gly Gly
145                 150                 155                 160

Ser Leu Arg Leu Ser Cys Ala Ala Ser Gly Phe Thr Phe Ser Asp Tyr
                165                 170                 175

Gly Met Ser Trp Val Arg Gln Ala Pro Gly Lys Arg Leu Glu Leu Val
            180                 185                 190

Ala Thr Ile Asn Gly Asn Gly Val Lys Ile Tyr Tyr Val Asp Ser Val
        195                 200                 205

Lys Gly Arg Phe Thr Ile Ser Arg Asp Asn Ser Lys Asn Thr Leu Tyr
    210                 215                 220

Leu Gln Met Asn Ser Leu Arg Ala Glu Asp Thr Ala Val Tyr Tyr Cys
225                 230                 235                 240

Pro Arg Glu Gly Phe Asp Tyr Trp Gly Gln Gly Thr Leu Val Thr Val
                245                 250                 255

Ser Ser Thr Thr Thr Pro Ala Pro Arg Pro Pro Thr Pro Ala Pro Thr
            260                 265                 270

Ile Ala Ser Gln Pro Leu Ser Leu Arg Pro Glu Ala Cys Arg Pro Ala
        275                 280                 285

Ala Gly Gly Ala Val His Thr Arg Gly Leu Asp Phe Ala Cys Asp Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Gln Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210>  203
<211>  483
<212>  PRT
<213>  Artificial Sequence

<220>
<223>  H2 215C2 CAR

<400>  203

Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu
            20                  25                  30

Ser Ala Ser Val Gly Asp Arg Val Thr Ile Thr Cys Arg Ala Ser Gln

35                  40                  45

Asn Val Gly Thr Ala Val Ala Trp Phe Gln Gln Lys Pro Gly Lys Ala
    50                  55                  60

Pro Lys Ser Leu Ile Tyr Ser Ala Ser Asn Arg Tyr Thr Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Tyr
            100                 105                 110

Ser Ser Tyr Pro Leu Thr Phe Gly Gln Gly Thr Lys Leu Glu Ile Lys
        115                 120                 125

Thr Gly Ser Thr Ser Gly Ser Gly Lys Pro Gly Ser Gly Glu Gly Ser
    130                 135                 140

Glu Val Gln Leu Val Glu Ser Gly Gly Gly Leu Val Gln Pro Gly Gly
145                 150                 155                 160

Ser Leu Arg Leu Ser Cys Ala Ala Ser Gly Phe Thr Phe Ser Asp Tyr
                165                 170                 175

Gly Met Ser Trp Val Arg Gln Ala Pro Gly Lys Gly Leu Glu Trp Val
            180                 185                 190

Ala Thr Ile Asn Gly Asn Gly Val Lys Ile Tyr Tyr Val Asp Ser Val
        195                 200                 205

Lys Gly Arg Phe Thr Ile Ser Arg Asp Asn Ser Lys Asn Thr Leu Tyr
    210                 215                 220

Leu Gln Met Asn Ser Leu Arg Ala Glu Asp Thr Ala Val Tyr Tyr Cys
225                 230                 235                 240

Pro Arg Glu Gly Phe Asp Tyr Trp Gly Gln Gly Thr Leu Val Thr Val
                245                 250                 255

Ser Ser Thr Thr Thr Pro Ala Pro Arg Pro Pro Thr Pro Ala Pro Thr
            260                 265                 270

Ile Ala Ser Gln Pro Leu Ser Leu Arg Pro Glu Ala Cys Arg Pro Ala
        275                 280                 285

Ala Gly Gly Ala Val His Thr Arg Gly Leu Asp Phe Ala Cys Asp Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

## Description

### FIELD OF THE INVENTION

The present invention is comprised within the field of biotechnology and biomedicine. It specifically relates to antibodies specific against the p95 fragment of HER2 as well as to antibody-drug conjugates and chimeric antigen receptors comprising said antibodies and the uses thereof in in the treatment of cancer.

### BACKGROUND OF THE INVENTION

Cancer is one of the leading causes of morbidity and mortality worldwide. It is now responsible for almost one in six deaths globally and the number of new cases is expected to rise by about 70% over the next 2 decades.

Many drugs are now available to be used in the treatment of cancer. However, in many cases the cancer fails to respond to the anti-cancer therapy or its growth and/or metastasis is only slowed. Even when a tumor initially responds to an anti-cancer therapy by decreasing in size or going into remission, the tumor often develops resistance to the drug. For these reasons, there is a need for new anti-cancer agents and drugs which can be used to treat cancers for which there is still no treatment available and for multi-drug resistance cancers.

HER2 is a receptor tyrosine kinase overexpressed in -25% of breast and gastric cancers. Despite the success of anti-HER2 therapies, such as the monoclonal antibody trastuzumab or the inhibitor lapatinib, a high proportion (40%) of advanced breast cancer cases eventually progress. Furthermore, cardiotoxicity, due to expression of HER2 in cardiomyocytes, has been frequently observed in treated patients. Therefore, there is a clinical need to develop more effective and safer treatments against HER2-driven tumours. CARs targeting HER2 have also been developed. However, adoptive cell therapy directed against HER2 has been limited by the expression of HER2 in healthy tissues, which leads to serious side effects.

p95HER2 is a fragment of HER2 expressed exclusively in some HER2- positive tumours. It has been shown that T cells can be safely directed against p95HER2 via T cell bispecific antibodies. However, no adoptive cell therapy based on chimeric antigen receptor (CARs) against specific for p95HER2 has been developed. In fact, earlier attempts to generate p95HER2 CAR failed to be expressed at the T cell surface and were uncapable of killing cells expressing p95HER2 (Research Disclosure, database number 667070). Accordingly, there is a need in the art for anti-tumor therapies which specifically target p95HER2-expressing cells.

### SUMMARY OF THE INVENTION

The authors of the present invention have obtained a chimeric antigen receptor (CAR) which is capable of targeting p95HER2-expressing cells and inducing potent anti-tumour activity against p95HER2-positive tumours, but, with no apparent activity on cells that express normal levels of HER2. The CAR has been obtained using a ScFv from an anti-p95HER2 ScFv that had previously failed to provide a functional CAR and required the humanization of the ScFv and the modification of the order of the VH and VL regions within the ScFv into a specific arrangement. This is shown in Example 1 of the present document, where it is demonstrated that the CARs of the invention induced specific cytotoxic effect in cells expressing p95HER2 and, in contrast, did not have any effect on cells not expressing p95HER2.

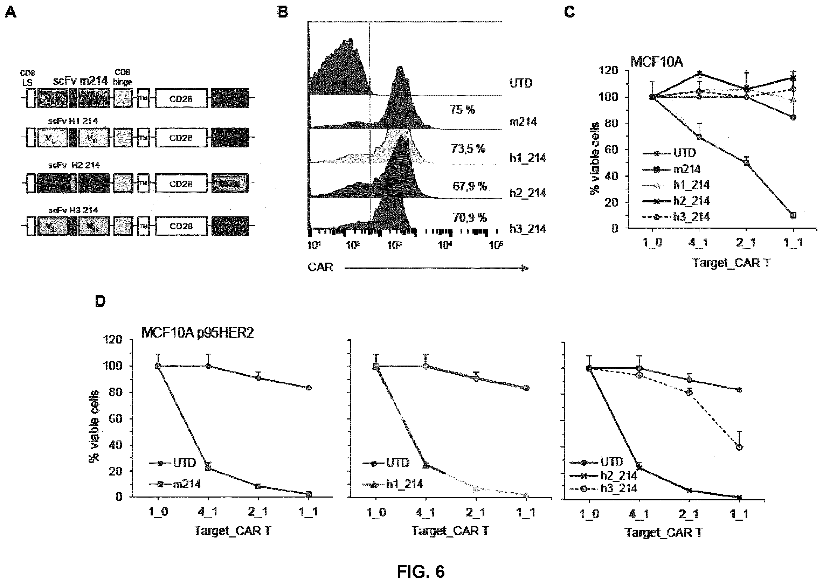

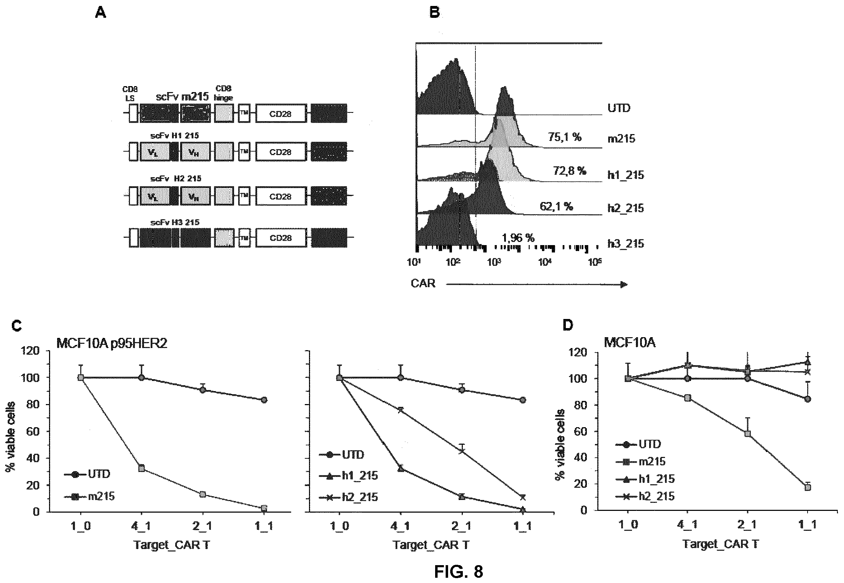

In addition, the authors of the present invention have generated CARs from a different anti-p95HER2 ScFv and shown that the CARs are capable of inducing a strong cytotoxic effect on p95HER2-expressing cells. This is shown in Examples 2 and 3 of the present document. Moreover, the use of humanized ScFv versions generates CAR Ts more specific for p95HER2 due to the decrease in the killing of cells expressing normal levels of HER2, compared with the non-humanized versions as it is shown in Figures 6 and 8. Thus, in a first aspect the invention relates to a chimeric antigen receptor (CAR) comprising:
(i) an antigen-binding domain specific for p95HER2,
(ii) a transmembrane domain and
(iii) at least one intracellular signaling domain and/or costimulatory domain
wherein the antigen-binding domain is selected from the group consisting of
(i) an ScFv (ScFv1), characterized in that:
   - the framework regions of the VL and VH regions are humanized;
   - the VL region is located N-terminally with respect to the VH region,
   - the CDR1, CDR2 and CDR3 of the VH region comprise, respectively, the sequences of SEQ ID NO: 1, 2 and 3 or functionally equivalent variant thereof, or the sequences of SEQ ID NO: 1, 174 and 3 or functionally equivalent variant thereof, and
   - the CDR1, CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof,
(ii) an antigen-binding domain (antigen-binding domain 1), characterized in that:
   - it has at least one VH and at least one VL region,
   - the CDR1, CDR2 and CDR3 of the at least VH region comprise, respectively, the sequences of SEQ ID NO: 7, 8 and 9 or SEQ ID NO: 176, 177 and DY or functionally equivalent variants thereof, and
   - the CDR1, CDR2 and CDR3 of the at least VL region comprise respectively, the sequences of SEQ ID NO: 10, 11, and 12 or functionally equivalent variants thereof, and
(iii) an antigen-binding domain (antigen-binding domain 2), characterized in that:
   - it has at least one VH and at least one VL regions,
   - the CDR1, CDR2 and CDR3 of the at least VH region comprise, respectively, the sequences of SEQ ID NO: 13, 14 and 15 or functionally equivalent variants thereof, and
   - the CDR1, CDR2 and CDR3 of the at least VL region comprise respectively, the sequences of SEQ ID NO: 16, 17, and 18 or functionally equivalent variants thereof or the sequences of SEQ ID NO: 179, 17 and 18 or functionally equivalent variants thereof.

In a second aspect, the invention relates to a nucleic acid encoding the CAR of the invention.

In a third aspect, the invention relates to an expression vector comprising the nucleic acid of the second aspect of the invention.

In a fourth aspect, the invention relates to a host cell comprising the nucleic acid of the second aspect of the invention or the vector of the third aspect of the invention.

In a fifth aspect, the invention relates to an ScFv characterized in that:
- the CDR1, CDR2 and CDR3 of the VH region comprise, respectively, the sequences of SEQ ID NO: 1, 2 and 3 or functionally equivalent variants thereof or the sequences of SEQ ID NO: 1, 174 and 3 or functionally equivalent variants thereof, and
- the CDR1 CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof.

In a sixth aspect, the invention relates to an antigen-binding domain characterized in that:
- it has at least one VH region and at least one VL region,
- the CDR1, CDR2 and CDR3 of the at least VH region comprise, respectively, the sequences of SEQ ID NO: 7, 8 and 9 or functionally equivalent variants thereof or the sequences of SEQ ID NO: 176, 177 and 178 or functionally equivalent variants thereof and
- the CDR1, CDR2 and CDR3 of the at least VL region comprise respectively, the sequences of SEQ ID NO: 10, 11, and 12 or functionally equivalent variants thereof.

In a seventh aspect, the invention relates to an antibody or a fragment thereof characterized in that:
it has at least one VH region and at least one VL region,
- the CDR1, CDR2 and CDR3 of the at least VH region comprise, respectively, the sequences of SEQ ID NO: 13, 14 and 15 or functionally equivalent variants thereof and
- the CDR1, CDR2 and CDR3 of the at least VL region comprise respectively, the sequences of SEQ ID NO: 16, 17, and 18 or functionally equivalent variants thereof or the sequences of SEQ ID NO: 179, 17 and 18 or functionally equivalent variants thereof.

In an eight aspect, the invention relates to a nucleic acid encoding the ScFv, antigen-binding domain or antibody according to the fifth, sixth and seventh aspects of the invention.

In a ninth aspect, the invention relates to an expression vector comprising the nucleic acid of the eighth aspect of the invention.

In a tenth aspect, the invention relates to a host cell comprising the nucleic acid of the eighth aspect of the invention or the expression vector of the ninth aspect of the invention.

In an eleventh aspect, the invention relates to an antibody-drug conjugate (ADC) comprising:
- a therapeutic agent, and
- an antibody or antigen-binding domain specific for p95HER2 selected from the group consisting of:
   (i) an antibody or antibody fragment thereof characterized in that:
      - it has at least one VH region and one VL region,
      - the CDR1, CDR2 and CDR3 of the at least VH region comprise, respectively, the sequences of SEQ ID NO: 1, 2 and 3 or functionally equivalent variants thereof or the sequences of SEQ ID NO: 1, 174 and 3 or functionally equivalent variants thereof,
      - the CDR1 CDR2 and CDR3 of the at least VL region comprise respectively, the sequences of SEQ ID NO: 4, 5, and 6 or functionally equivalent variants thereof or the sequences of SEQ ID NO: 175, 5 and 6 or functionally equivalent variants thereof
   (ii) an antibody or antibody fragment thereof characterized in that:
      - it has at least one VH region and one VL region,
      - the CDR1, CDR2 and CDR3 of the at least VH region comprise, respectively, the sequences of SEQ ID NO: 7, 8 and 9 or SEQ ID NO: 176, 177 and DY or functionally equivalent variants thereof and
      - the CDR1, CDR2 and CDR3 of the at least VL region comprise respectively, the sequences of SEQ ID NO: 10, 11, and 12 or functionally equivalent variants thereof, and
   (iii) an antibody or antibody fragment thereof characterized in that:
      - it has at least one VH region and at least one VL region,
      - the CDR1, CDR2 and CDR3 of the at least VH region comprise, respectively, the sequences of SEQ ID NO: 13, 14 and 15 or functionally equivalent variants thereof and
      - the CDR1 CDR2 and CDR3 of the at least VL region comprise respectively, the sequences of SEQ ID NO: 16, 17, and 18 or functionally equivalent variants thereof or the sequences of SEQ ID NO: 179, 17 and 18 or functionally equivalent variants thereof.

In a twelfth aspect, the invention relates to a method of cancer diagnosis in a patient which comprises:
(i) contacting a sample of the patient containing tumor cells with an ScFv, antigen-binding domain or antibody according to the fifth, sixth or seventh aspects of the invention and
(ii) detecting the binding of the ScFv, antigen-binding domain or antibody to cells in the sample
wherein the presence of binding is indicative that the patient suffers from cancer.

In a thirteenth aspect, the invention relates to pharmaceutical composition comprising the host cell of the fourth aspect of the invention and/or an ScFv, antigen-binding domain or antibody according to the fifth, sixth or seventh aspects of the invention and/or the ADC of the eleventh aspect of the invention and at least one pharmaceutically acceptable excipient and/or vehicle.

In a fourteenth aspect, the invention relates to the host cell of the fourth aspect of the invention and/or the ScFv, antigen-binding domain or antibody of the fifth, sixth and seventh aspects of the invention and/or the ADC of the eleventh aspect of the invention for use in medicine.

In a final aspect, the invention relates to the host cell of the fourth aspect of the invention, and/or the ScFv, antigen binding domain or antibody of the fifth, sixth and seventh aspects of the invention and/or the ADC of the eleventh aspect of the invention for use in a method of preventing or treating cancer.

### DESCRIPTION OF THE FIGURES

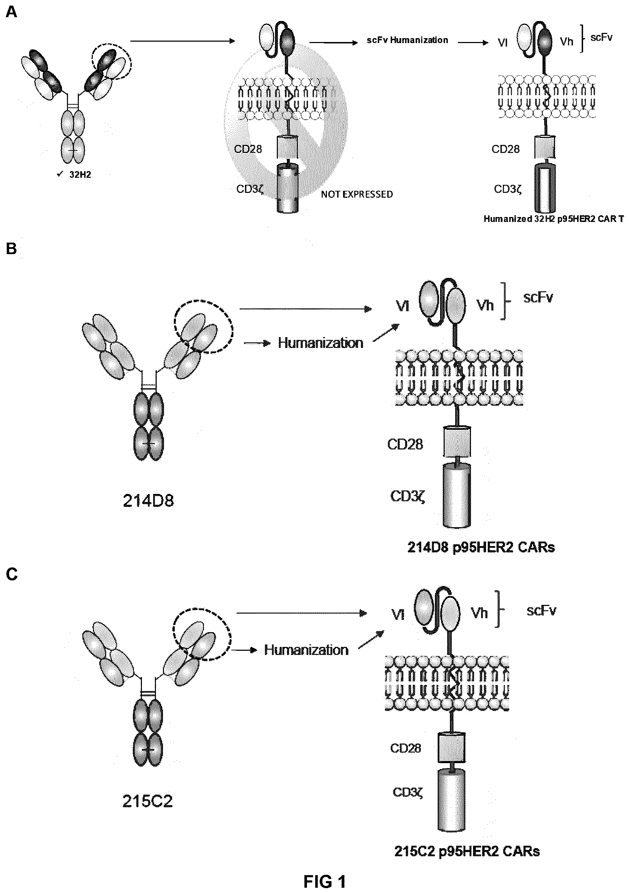

**Figure 1****.** *Schematic representation of the three p95HER2 CARs disclosed in the present document.* (A) Humanized 32H2 p95HER2 CAR. (B) 214D8 p95HER2 CAR. (C) 215C2 p95HER2 CAR.

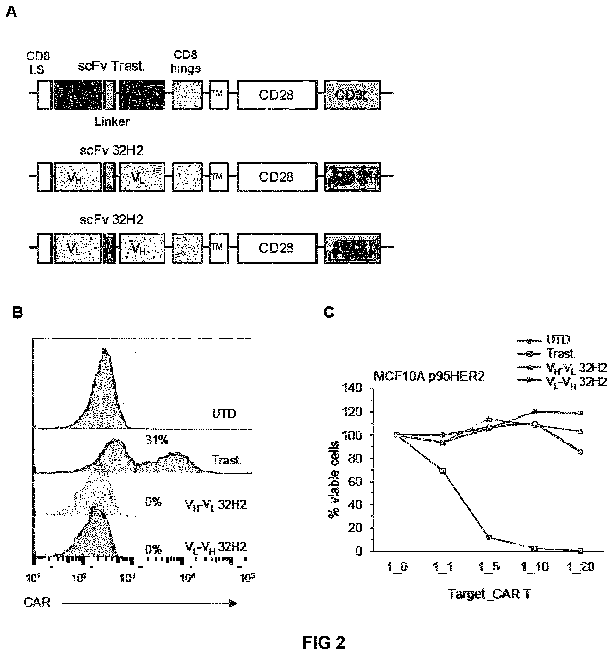

**Figure 2****.** *Design, expression and cytotoxicity of 32H2 p95HER2 CAR Ts.* (A) Schematic representation of the chimeric receptors containing an ScFv that binds to full-length HER2 or p95HER2. (B) Surface expression of the indicated CARs in A on T cells at day 5 post-transduction; percentage of positive-CAR T from total T cells are indicated. (C) MCF10A p95HER2 cells were co-cultured with CAR T cells at the indicated ratios. At 48h, viable target cells were assessed by flow cytometry UTD: untransduced T cells; Trast: Trastuzumab-based CAR.

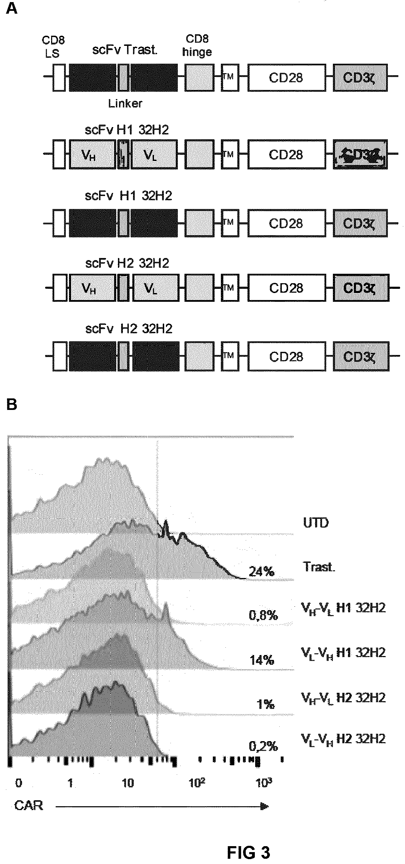

**Figure 3****.** *Design and expression of humanized 32H2 p95HER2 CAR Ts.* (A) Schematic representation of the chimeric receptors containing an ScFv that binds to full-length HER2 or p95HER2. (B) Surface expression of the indicated CARs in A on T cells at day 5 post-transduction; percentage of positive-CAR T from total T cells are indicated. UTD: untransduced T cells; Trast: Trastuzumab-based CAR.

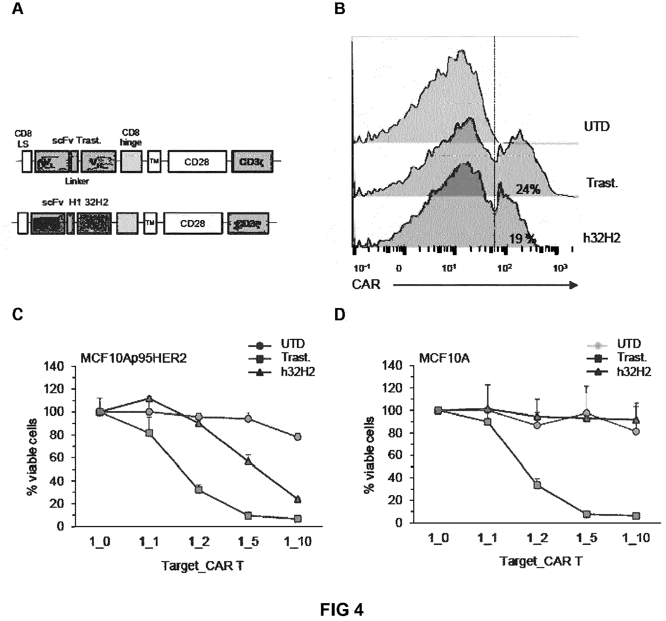

**Figure 4****.** *Design, expression and cytotoxicity of h32H2 p95HER2 CAR Ts.* (A) Schematic representation of the chimeric receptors containing an ScFv that binds to full-length HER2 or p95HER2. (B) Surface expression of the indicated CARs in A on T cells at day 5 post-transduction; percentage of positive-CAR T from total T cells are indicated. (C) MCF10A p95HER2 cells were co-cultured with CAR T cells at the indicated ratios. At 48h, viable target cells were assessed by flow cytometry. (D) MCF10A cells were co-cultured with CAR T cells at the indicated ratios. At 48h, viable target cells were assessed by flow cytometry. UTD: untransduced T cells; Trast: Trastuzumab-based CAR.

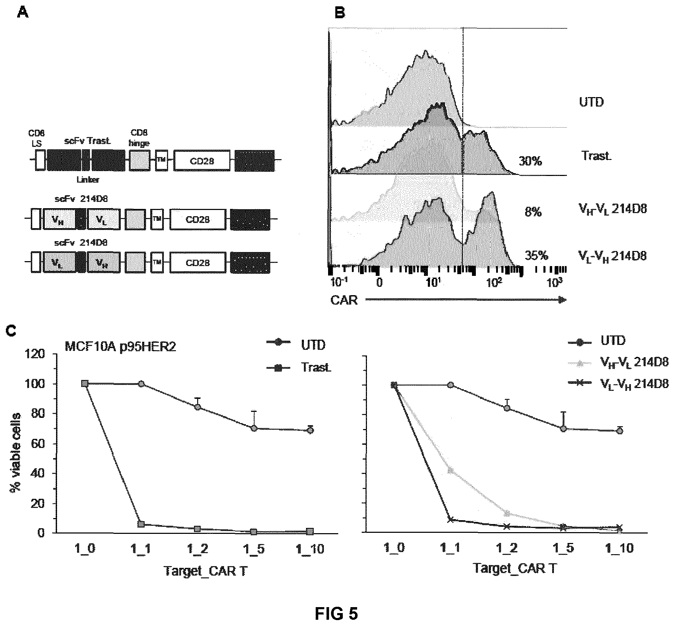

**Figure 5****.** *Design, expression and cytotoxicity of 214D8 p95HER2 CAR* T. (A) Schematic representation of the chimeric receptors containing an scFv that binds to full-length HER2 or p95HER2. (B) Surface expression of the indicated CARs in A on T cells at day 5 post-transduction; percentage of positive-CAR T from total T cells are indicated. (C) MCF10A p95HER2 cells were co-cultured with CAR T cells at the indicated ratios. At 48h, viable target cells were assessed by flow cytometry. UTD: untransduced T cells; Trast: Trastuzumab-based CAR.
**Figure 6****.** *Design, expression and cytotoxicity of humanized 214D8 p95HER2 CAR Ts.* (A) Schematic representation of the chimeric receptors containing an scFv that binds to p95HER2 (B) Surface expression of the indicated CARs in A on T cells at day 5 post-transduction; percentage of positive-CAR T from total T cells are indicated. (C) MCF10A p95HER2 cells or MCF10A wild type were co-cultured with CAR T cells at the indicated ratios. At 48h, viable target cells were assessed by flow cytometry. UTD: untransduced T cells.

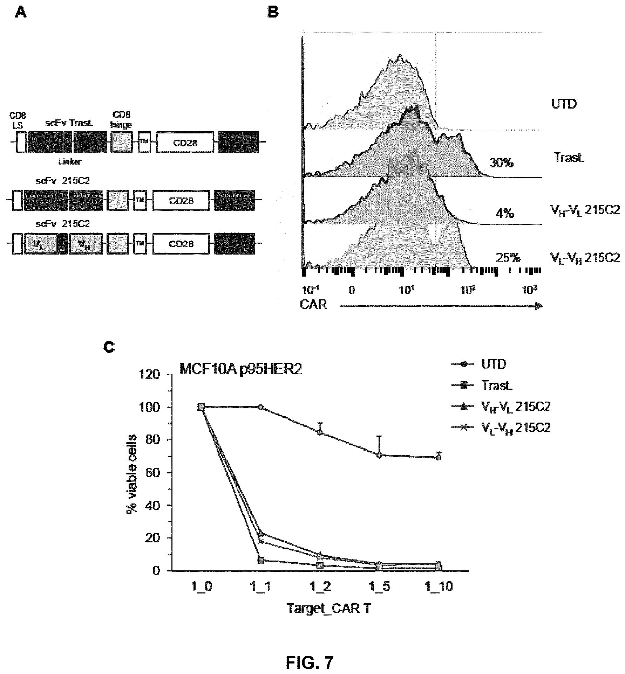

**Figure 7****.** *Design, expression and cytotoxicity* of 215C2 *p95HER2 CAR Ts.* (A) Schematic representation of the chimeric receptors containing an scFv that binds to full-length HER2 or p95HER2. (B) Surface expression of the indicated CARs in A on T cells at day 5 post-transduction; percentage of positive-CAR T from total T cells are indicated. (C) MCF10A p95HER2 cells were co-cultured with CAR T cells at the indicated ratios. At 48h, viable target cells were assessed by flow cytometry. UTD: untransduced T cells; Trast: Trastuzumab-based CAR.
**Figure 8****.** *Design, expression and cytotoxicity of humanized* 215C2 *p95HER2 CAR Ts.* (A) Schematic representation of the chimeric receptors containing an scFv that binds to p95HER2 (B) Surface expression of the indicated CARs in A on T cells at day 5 post-transduction; percentage of positive-CAR T from total T cells are indicated. (C) MCF10A p95HER2 cells or MCF10A wild type were co-cultured with CAR T cells at the indicated ratios. At 48h, viable target cells were assessed by flow cytometry. UTD: untransduced T cells.

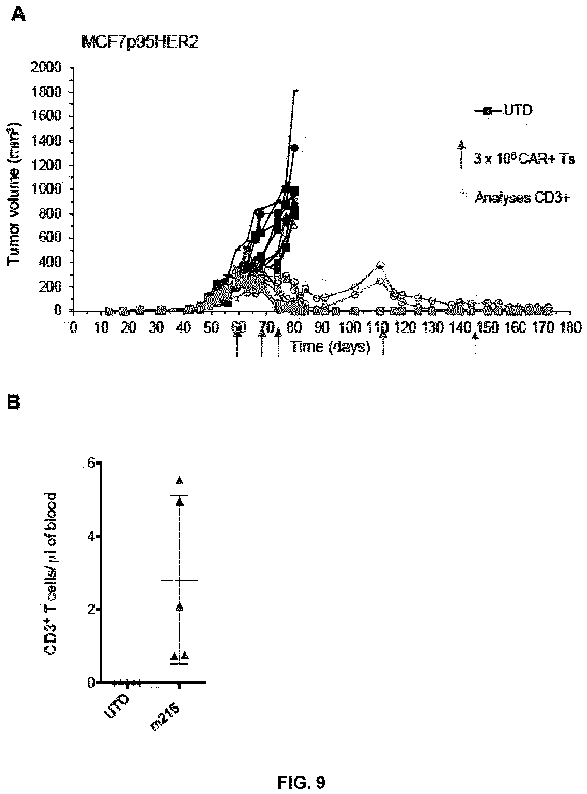

**Figure 9****:** *Effect of m215-derived p95HER2 CAR T on the growth of p95HER2-positive tumours in vivo.* (A) Mice were orthotopic implantated with MCF7p95HER2 cells. When tumors reached aproximetly 300 mm³ they were treated with 3 x 10⁶ CAR+ T cells. (B) Percentage of circulating human CD3+ T cells per µl of blood, relative to total leukocytes, at day 144.

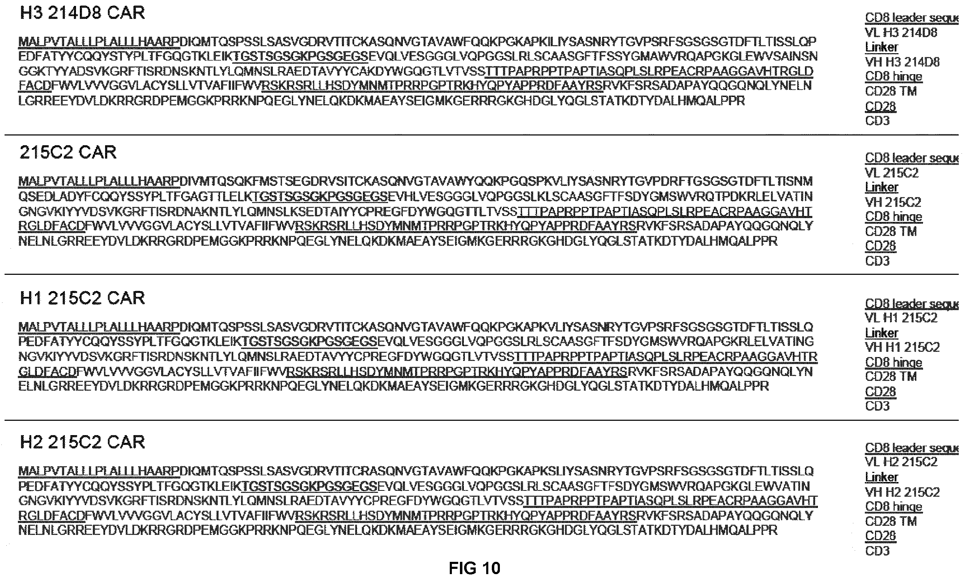

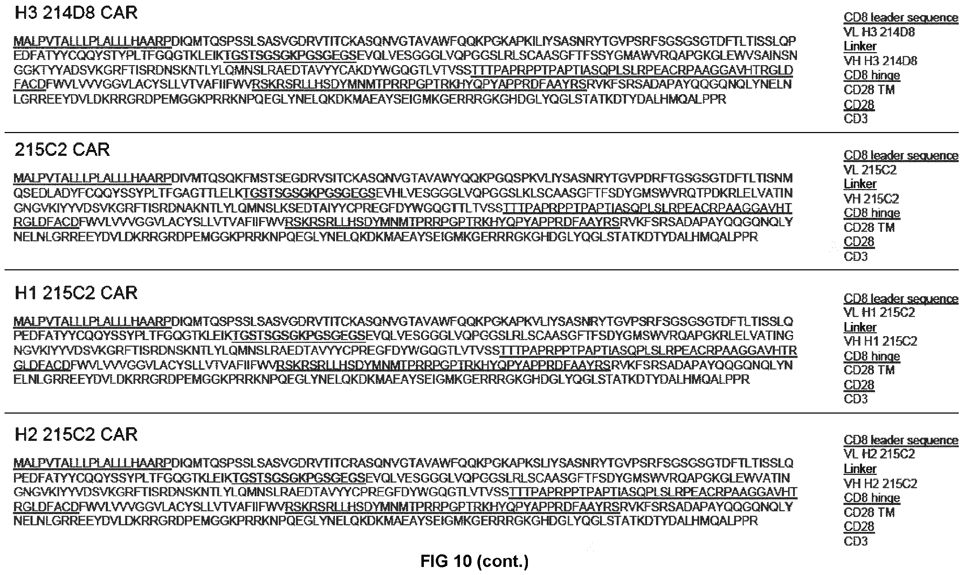

**Figure 10****.** Complete amino acid sequence of the p95HER2-CARs disclosed in the present document.

### DETAILED DESCRIPTION OF THE INVENTION

The present invention relates to the provision of new compounds for the treatment of cancer.

Unless otherwise defined, all technical terms used herein have the same meaning as commonly understood by one of ordinary skill in the art to which this invention belongs.

All the embodiments and definitions disclosed in the context of one aspect of the invention are also applicable to the other aspects of the invention.

### Chimeric antigen receptors (CARs)

In a first aspect, the invention relates to a chimeric antigen receptor (CAR) comprising:
(i) an antigen-binding domain specific for p95HER2,
(ii) a transmembrane domain and
(iii) at least one intracellular signaling domain and/or costimulatory domain
wherein the antigen-binding domain is selected from the group consisting of:
(i) an ScFv1 ,characterized in that:
   - The framework regions of the VL and VH regions are humanized,
   - the VL region is located N-terminally with respect to the VH region, and in that the CDR1, CDR2 and CDR3 of the VH region comprise, respectively, the sequences of SEQ ID NO: 1, 2 and 3 or functionally equivalent variants thereof or the sequences of SEQ ID NO: 1, 174 and 3 or functionally equivalent variants thereof; and
   - the CDR1 CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof.
(ii) an antigen-binding domain (antigen-binding domain 1), characterized in that:
   - it has at least one VH region and at least one VL region,
   - the CDR1, CDR2 and CDR3 of the at least VH region comprise, respectively, the sequences of SEQ ID NO: 7, 8 and 9 or SEQ ID NO: 176, 177 and DY or functionally equivalent variants thereof and
   - the CDR1, CDR2 and CDR3 of the at least VL region comprise respectively, the sequences of SEQ ID NO: 10, 11, and 12 or functionally equivalent variants thereof, and
(iii) an antigen-binding domain (antigen-binding domain 2), characterized in that:
   - it has at least one VH region and at least one VL region,
   - the CDR1, CDR2 and CDR3 of the at least one VH region comprise, respectively, the sequences of SEQ ID NO: 13, 14 and 15 or functionally equivalent variants thereof, and
   - the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.

As used herein, a "chimeric antigen receptor (CAR)" also known as chimeric T cell receptors, a T-body, artificial T cell receptors and chimeric immune receptors (CIR), are engineered receptors, which graft an arbitrary specificity onto an immune effector cell. In a classical CAR, the specificity of a monoclonal antibody is grafted on to a T cell. CARs are therefore fusion proteins which comprise at least, an extracellular domain or antigen binding domain capable of binding to an antigen, a transmembrane domain derived from a polypeptide different from a polypeptide from which the extracellular domain is derived, and at least one intracellular costimulatory domain.

According to the present invention, the expressions "extracellular domain", antigen-binding domain", "antigen-binding fragment" or "antibody fragment" are used interchangeably and refer to any oligopeptide or polypeptide that can bind to a certain antigen. It may comprise an antibody fragment, which refers to at least one portion of an intact antibody, or recombinant variants thereof, for example an antigen variable region of an intact antibody that is sufficient to allow recognition and specific binding of an antibody fragment to a target. The antigen-binding domain of the invention comprises at least a VH region and a VL region. Examples of antibody fragments include, but are not limited to Fab, Fab'-, F(ab')₂ and Fv fragments, ScFv antibody fragments and linear antibodies. Within the context of the present invention, the antigen-binding domain or antibody fragment comprise at least one VH and one VL regions, but it may comprise two VL regions and two VH regions. Thus, for example, in an embodiment, the antigen-binding domain is an ScFv, and therefore, it will comprise only one VL and one VH regions. In another embodiment, the antigen-binding domain is a Fab fragment, in which case it will comprise one VL and VH (Fab or Fab') or two VH and two VL regions (Fab₂, or F(ab')₂).

In a particular embodiment, the antigen-binding domain is humanized.

As used herein, "humanized" forms of non-human (e.g., murine) antibodies or antigen-binding domains are chimeric antibodies or antigen-binding domains that contain minimal sequence, or no sequence, derived from non-human immunoglobulin. For the most part, humanized antibodies or antigen-binding domains are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies or antigen-binding domains can comprise residues that are not found in the recipient antibody or in the donor antibody. These modifications are generally made to further refine antibody or antigen-binding domain performance. In general, the humanized antibody or antigen-binding domain will comprise substantially all of at least one, and typically two, variable domains, in which all or substantially all of the hypervariable loops correspond to those of a nonhuman immunoglobulin and all or substantially all of the FR residues are those of a human immunoglobulin sequence. The humanized antibody can also comprise at least a portion of an immunoglobulin constant region (Fc), typically that of a human immunoglobulin.

The antigen-binding domain of the CAR of the invention specifically recognizes the carboxy-terminal fragment of HER2, p95HER2.

The terms "HER2" and "HER2 receptor" are used interchangeably herein, and refer to the ErbB2 protein (also referred to as HER2/neu in the literature). As used herein, the terms are intended to include variants (e.g., splice variants), isoforms, and homologs of HER2 (both orthologs and paralogs). In some aspects, binding of an anti-HER2 binding molecule disclosed herein to HER2 inhibits the growth of cells expressing HER2 (i.e. typically tumor cells, and in particular cancer cells expressing low levels of HER2) by inhibiting formation of heteromeric complexes between HER2 and other ErbB family members, e.g. inhibiting heterodimerization with EGFR or HER3.

HER2 is a receptor tyrosine kinase and is composed of an extracellular domain (ECD), which consists of (i) two leucine-rich domains (domain I/L1 and domain III/L2) responsible for ligand binding, and (ii) two cysteine-rich domains (domain II/CR1 and domain IV/CR2) responsible for receptor dimerization; a transmembrane domain; and an intracellular tyrosine kinase domain. Alternative splice variants of HER2 exist and may also be part of the present invention.

The term "p95HER2" as used herein refers to a carboxy terminal fragment (CTF) of the HER2 receptor protein, which is also known as "611-CTF" or "100-115 kDa p95HER2". The p95HER2 fragment is generated in the cell through initiation of translation of the HER2 mRNA at codon position 611 of the full-length HER2 molecule (Anido et al, EMBO J 25; 3234-44 (2006)). It has a molecular weight of 100 to 115 kDa and is expressed at the cell membrane, where it can form homodimers maintained by intermolecular disulfide bonds.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) or complementary determining regions (CDRs). A single VH or VL domain may be sufficient to confer antigen-binding specificity.

The term "hypervariable region", "HVR", "complementarity determining regions" or "CDRs" as used herein, refers to each of the regions of an antibody variable domain which are hypervariable in sequence and/or form structurally defined loops ("hypervariable loops"). Generally, native four-chain antibodies comprise six CDRs; three in the VH (H1, H2, H3), and three in the VL (L1, L2, L3). Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy variable chain and the light variable chain are characterized by three CDRs, respectively VH-CDR1, VH-CDR2, VH-CDR3 and VL-CDR1, VL-CDR2, VL-CDR3.

The CDR sequences can be determined according to conventional criteria, for example by means of the criteria of IgBLAST: http://www.ncbi.nlm.nih.gov/igblast/ (Ye et al., 2013, Nucleic Acids Res 41 (Web Server issue:W34-40), by following the numbering provided by Kabat et al, Sequences of Proteins of Immunological Interest, 5th ed.,Public Health Service, National Institutes of Health, Bethesda, Md. (1991), or by following the numbering provided by Chothia et al. (1989, Nature 342:877-83).This particular region has been described by Kabat et al., Sequences of Proteins of Immunological Interest, 5th Ed. Public Health Service, National Institutes of Health, Bethesda, Md. (1991) and by Chothia et al., J. Mol. Biol. 196:901-917 (1987), where the definitions include overlapping or subsets of amino acid residues when compared against each other. The exact residue numbers which encompass a particular CDR will vary depending on the sequence and size of the CDR. Those skilled in the art can routinely determine which residues comprise a particular CDR given the variable region amino acid sequence of the antibody. The CDR sequences given herein are generally according to the Kabat definition.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H1 (L1)-FR2-H2(L2)-FR3-H3 (L3)-FR4.

In a particular embodiment, the antigen-binding domain of the CAR of the invention is an ScFv.

As used herein, a "single chain variable fragment (ScFv)" means a single chain polypeptide derived from an antibody which retains the ability to bind to an antigen. An example of the ScFv includes an antibody polypeptide which is formed by a recombinant DNA technique and in which variable (Fv) regions of immunoglobulin heavy chain (VH chain) and light chain (VL chain) fragments are linked via a spacer sequence. Various methods for preparing an ScFv are known, and include methods described in US Patent No. 4694778, , Nature, vol. 334, p. 54454 (1989), and Science, vol. 242, pp. 1038-1041 (1988).

The second element of the CARs according to the present invention is a transmembrane domain that is attached to the extracellular domain of the CAR.

As used herein, "transmembrane domain" (TMD) refers to the area of CAR that crosses the cell membrane. The transmembrane domain of the CAR of the invention is the transmembrane domain of a transmembrane protein (e.g., a type I transmembrane protein), an artificial hydrophobic sequence, or a combination thereof. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR is used. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the CART cell surface. In a different aspect, the amino acid sequence of the transmembrane domain may be modified or substituted so as to minimize interactions with the binding domains of the native binding partner present in the same CART.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. Non limiting examples or transmembrane domains of particular use in this invention may include at least the transmembrane region(s) of e.g., the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154, CD3 zeta, KIRDS2, OX40, CD2, CD27, LFA-1 (CD1 la, CD18), ICOS (CD 278), 4-1BB (CD137), GITR, CD40, CTLA4, BAFFR, HVEM (LIGHTR), SLAMF7, NKp80 (KLRFI), CD160, CD19, IL2R beta, IL2R gamma, IL7Ra, ITGA1, VLA1, CD49a, ITGA4, IA4 CD49D, ITGA6, VLA-6, CD49f, ITGAD, CDGA, CDGA, CD103, ITGAL, CDLa, LFA-1, ITGAM, CDIIb, ITGAX, CDIc, ITGB1, CD29, ITGB2, CD18, LFA-1, LGA ITGB7, TNFR2, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), SLAMF6 (NTB-A), LyI08), SLAM (SLAMF1, CD150, IPO-3), BLAME (SLAMF8), SELPLG (CD162), LTBR, PAG/Cbp, NKp44, Kp30, NKp46, including NKG2D, and/or a transmembrane domain selected from the transmembrane domain of NKG2C.

In a particular embodiment, the transmembrane domain is selected from the group consisting of the CD4 transmembrane domain, the CD8 transmembrane domain, the CD28 transmembrane domain, the 4-1BB transmembrane domain, the CTLA4 transmembrane domain, the CD27 transmembrane domain and the CD3 zeta transmembrane domain.

In a particular embodiment, the transmembrane domain is the CD28 transmembrane domain. In a particular embodiment, the CD28 transmembrane domain comprises the sequence FWVLVVVGGVLACYSLLVTVAFIIFWV (SEQ ID NO: 113)

The CARs according to the present invention comprise at least one intracellular signaling domain and/or costimulatory domain.

"Intracellular signaling domain," as the term is used here, refers to the intracellular portion of a molecule and more specifically to any oligopeptide or polypeptide known to function as a domain that transmits a signal to cause activation or inhibition of a biological process in a cell. The intracellular signaling domain generates a signal that stimulates the immune effector function of CAR-containing cells, for example, CAR-T cells. The effector function of a T cell, for example, may be cytolytic function or helper activity including the secretion of cytokines. Thus, the intracellular signalling domain may be a portion of a protein which transduces the effector function signal and directs the cell (e.g. T cell) to perform a specialised function.

Generally, the whole intracellular signalling domain can be used; however, it is appreciated that it is not necessary to use the entire domain, provided that whatever part of the signalling domain that is used is still capable of transducing the effector function signal. It will also be appreciated that variants of such intracellular signalling domains with substantially the same or greater functional capability may also be used. By this we include the meaning that the variants should have substantially the same or greater transduction of the effector functional signal. Typically, substantially the same or greater signal transduction includes at least 80%, 85%, 90%, 95%, 100%, 105%, 110%, 115%, or 120%, or more of the signal transduction of the unmodified intracellular signalling domain, wherein signal transduction of the unmodified intracellular signalling domain corresponds to 100%. Methods for assessing transduction of effector function signal are well known to those skilled in the art and include, for example, assessing the amounts and/or activity of molecules (e.g. proteins such as cytokines) that are indicative of the transduced signal. Thus, when the signal is the cytolytic function of a T-cell, the methods may involve measurement of one or more cytokines secreted by the T-cell, which cytokines are known to have a cytolytic activity (e.g. I FN gamma). Another means of assessing the cytolytic function is by CFSE staining and counting positive cells by Flow cytometry or by a chromium release assay as is well known in the art.

Examples of intracellular signalling domains for use in the CAR of the invention include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are generally insufficient for full activation of a T cell and that a secondary and/or costimulatory signal may also be required. Thus, T cell activation can be said to be mediated by two distinct classes of intracellular signaling sequences: those that initiate antigen-dependent primary activation through the TCR (primary intracellular signaling domains) and those that act in an antigen- independent manner to provide a secondary or costimulatory signal (secondary intracellular signalling domain, such as a costimulatory domain).

Costimulatory domains promote activation of effector functions and may also promote persistence of the effector function and/or survival of the cell.

A primary intracellular signaling domain regulates primary activation of the TCR complex either in a stimulatory way, or in an inhibitory way. Primary intracellular signaling domains that act in a stimulatory manner may contain signalling motifs which are known as immunoreceptor tyrosine-based activation motifs or ITAMs (e.g. 2, 3, 4, 5 or more ITAMs). Thus, the intracellular signalling domain may comprise one or more ITAMs. Examples of ITAM containing primary intracellular signaling domains that are of particular use in the invention include those of CD3 zeta, Fc receptor gamma, Fc receptor beta, CD3 gamma, CD3 delta, CD3 epsilon, CD5, CD22, CD79a, CD79b, and CD66d.

In one embodiment, the intracellular signaling domain of the CAR of the invention is the CD3-zeta, and more particularly, the CAR of the invention comprises the sequence RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDALHMQALPP R (SEQ ID NO: 114) .

The term "zeta" or alternatively "zeta chain", "CD3-zeta" or "TCR-zeta" is defined as the protein represented by GenBank entry No. BAG36664.1, or equivalent residues from a non-human species, such as a mouse, rodent, monkey, primate, etc., and a "zeta stimulating domain" or alternatively a "CD3 zeta stimulating domain" or "TCR zeta stimulating domain" is defined as amino acid residues of the cytoplasmic domain of the zeta chain that are sufficient for functional transmission of the primary signal required to activate T cells etc. In one aspect, the zeta cytoplasmic domain comprises residues 52 through 164 inclusive of a GenBank entry protein of BAG36664.1, or equivalent residues from a non-human species, for example, a mouse, rodent, monkey, primate, and the like, which are their functional orthologists.

It will be appreciated that one or more ITAMs of the intracellular signalling domain may be modified, for example by mutation. The modification may be used to increase or decrease the signalling function of the ITAM as compared to the native ITAM domain.

As mentioned above, the intracellular signalling domain may comprise a primary intracellular signalling domain by itself, or it may comprise a primary intracellular signalling domain in combination with one or more secondary intracellular signalling domains, such as one or more costimulatory signalling domains. Thus, the intracellular signalling domain of the CAR may comprise the CD3 zeta signalling domain by itself or in combination with one or more other intracellular signalling domains such as one or more costimulatory signalling domains.

The costimulatory signaling domain refers to a portion of the CAR comprising the intracellular domain of a costimulatory molecule.

The term "co-stimulating molecule" refers to a recognizable T-cell binding partner that specifically binds to a co-stimulating ligand, thereby mediating the co-stimulatory response exerted by the T-cell, such as, but not limited to, proliferation. Co-stimulating molecules are cell surface molecules other than antigen-specific receptors or their ligands, which are necessary for an effective immune response. A costimulatory molecule may be a cell surface molecule other than an antigen receptor or its ligands that is required for an efficient response of immune cells (eg lymphocytes) to an antigen. A costimulatory molecule can be represented in the following protein families: TNF receptor proteins, immunoglobulin-like proteins, cytokine receptors, integrins, lymphocyte activation signaling molecules (SLAM proteins) and NK cell activation receptors. Examples of such molecules include, but are not limited to 4-1 BB (CD137), OX40, ICOS, DAP10, CD27, CD28, CDS, CD30, CD137 (4-1BB), CD40, ICOS, lymphocyte function-associated antigen- 1 (LFA-1), CD2, CD7, LIGHT, NKG2C, GITR, NKG2C, SLAMF7, NKp80, BAFFR, HVEM, BTLA, ICAM-1, LFA-1 (CD11a/CD18), B7-H3, and a ligand that specifically binds with CD83, and the like. For example, CD27 co-stimulation has been demonstrated to enhance expansion, effector function, and survival of human CART cells in vitro and augments human T cell persistence and anti-tumour activity in vivo (Song et al. Blood. 2012; 1 19(3):696-706).

In a particular embodiment, the CAR of the invention comprises the intracellular domain of the costimulatory molecule CD28, and more particularly, the sequence RSKRSRLLHSDYMNMTPRRPGPTRKHYQPYAPPRDFAAYRS (SEQ ID NO: 115)

In a preferred embodiment, the at least one intracellular signaling domain comprises a costimulatory domain, a primary signaling domain or a combination thereof.

In another embodiment, the at least one intracellular signaling domain comprises the intracellular domain of the costimulatory molecules selected from OX40, CD70, CD27, CD28, CD5, ICAM-1, LFA-1 (CD11a/CD18), ICOS (CD278), DAP10, DAP 12, and 4-1BB (CD137), or any combination thereof.

In a particular embodiment, the at least one intracellular signaling domain further comprises a CD3-zeta intracellular domain.

In another embodiment, the at least one intracellular signaling domain is arranged on a N-terminal side relative to the CD3-zeta intracellular domain. In another embodiment, the at least one intracellular signaling domain is the intracellular domain of the costimulatory molecule CD28, and is arranged on a N-terminal side relative to the CD3-zeta intracellular domain.

The intracellular signaling sequences within the intracellular portion of the CAR of the invention may be linked to each other in a random or specified order. Optionally, a short oligo- or polypeptide linker, for example, between 2 and 10 amino acids (eg 2, 3, 4, 5, 6, 7, 8, 9, or 10 amino acids) in length may form the linkage between intracellular signaling sequences. In one embodiment, a glycine- serine doublet can be used as a suitable linker. In another embodiment, a single amino acid, such as an alanine or a glycine, can be used as a suitable linker.

In one embodiment, the intracellular signaling domain is designed to comprise two or more, for example 3, 4, 5, or more, costimulatory signalling domains. In an embodiment, the two or more, e.g., 2, 3, 4, 5, or more, costimulatory signaling domains, are separated by a linker molecule, such as one described herein. In one embodiment, the intracellular signaling domain comprises two costimulatory signaling domains. In some embodiments, the linker molecule is a glycine residue. In some embodiments, the linker is an alanine residue.

In preferred embodiments, the intracellular portion of the CAR comprises:
- the signalling domain of CD3 zeta and the signalling domain of CD28,
- the signaling domain of CD3-zeta and the signaling domain of 4-1 BB,
- the signaling domain of CD3-zeta and the signaling domain of OX40,
- the signaling domain of CD3-zeta and the signaling domain of ICOS,
- the signaling domain of CD3-zeta and the signaling domain of DAP10
- the signalling domain of CD3-zeta, the signalling domain of 4-1 BB and the signalling domain of OX40.
- the signaling domain of 4-1 BB and the signaling domain of CD28.

In another embodiment, the intracellular portion of the CAR comprises the signaling domain of CD3-zeta, the signaling domain of 4-1 BB and the signalling domain of CD28.

The intracellular signaling domain may include the entire intracellular portion, or the entire natural intracellular signaling domain, the molecule from which it originates, or a functional fragment thereof.

The antigen-binding domain of the CARs of the present invention is selected from an ScFv1 and two antigen-binding domains, the antigen-binding domain 1 and the antigen-binding domain 2.

The ScFv1 is characterized in that:
- the V_{L} and V_{H} regions are humanized,
- the V_{L} region is located N-terminally with respect to the V_{H} region, and
- the CDR1, CDR2 and CDR3 of the V_{H} region comprise, respectively, the sequences of SEQ ID NO: 1, 2 and 3 or functionally equivalent variants thereof or the sequences of SEQ ID NO: 1, 174 and 3 or functionally equivalent variants thereof, and
- the CDR1 CDR2 and CDR3 of the V_{L} region comprise respectively, the sequences of SEQ ID NO: 4, 5, and 6 or functionally equivalent variants thereof or the sequences of SEQ ID NO: 175, 5 and 6 or functionally equivalent variants thereof.

The antigen-binding domain 1, having at least one VH region and at least one VL region, is characterized in that:
- the CDR1, CDR2 and CDR3 of the at least one V_{H} region comprise, respectively, the sequences of SEQ ID NO: 7, 8 and 9 or SEQ ID NO: 176, 177 and DY or functionally equivalent variants thereof, and
- the CDR1, CDR2 and CDR3 of the at least V_{L} region comprise respectively, the sequences of SEQ ID NO: 10, 11, and 12 or functionally equivalent variants thereof.

The antigen-binding domain 2, having at least one VH region and at least one VL region, is characterized in that:
- the CDR1, CDR2 and CDR3 of the V_{H} region comprise, respectively, the sequences of SEQ ID NO: 13, 14 and 15 or functionally equivalent variants thereof, and
- the CDR1, CDR2 and CDR3 of the V_{L} region comprise respectively, the sequences of SEQ ID NO: 16, 17, and 18 or functionally equivalent variants thereof or the sequences of SEQ ID NO: 179, 17 and 18 or functionally equivalent variants thereof.

As it is used herein, the term "functionally equivalent variant of a CDR sequence" refers to a sequence variant of a particular CDR sequence having substantially similar sequence identity with it and substantially maintaining its capacity to bind to its cognate antigen when being part of an antibody, antibody fragment or antigen-binding domain as the ScFv described herein. For example, a functionally equivalent variant of a CDR sequence may be a polypeptide sequence derivative of said sequence comprising the addition, deletion or substitution of one or more amino acids. In one embodiment, the substitution of one amino acid by other in the functionally equivalent variant is a conservative substitution.

As used herein, the term "conservative substitution" refers to the replacement of an amino acid by another amino acid having similar chemical properties. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Functionally equivalent variants of a CDR sequence according to the invention include CDR sequences having at least 70% %, at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of the above reference sequences. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a CDR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 1 to 18 and 174-179 to bind to its cognate antigen when being part of an antibody fragment or antigen-binding domain such as the ScFv of the CAR of the invention. This capacity to bind to its cognate antigen may be determined as a value of affinity, avidity, specificity and/or selectivity of the antibody or antibody fragment to its cognate antigen.

In a particular embodiment, the FR1, FR2, FR3 and FR4 of the VH region of the ScFv1 comprise respectively the sequences of SEQ ID NO: 19, 20, 21 and 22 or functionally equivalent variants thereof and FR1, FR2, FR3 and FR4 of the VL region of the ScFv1 comprise respectively the sequences of SEQ ID NO: 23, 24, 25 and 26 or functionally equivalent variants thereof.

In another particular embodiment, the FR1, FR2, FR3 and FR4 of the at least one VH region of the antigen-binding domain 1 comprise respectively the sequences of SEQ ID NO: 31, 32, 33 and 34 or functionally equivalent variants thereof and FR1, FR2, FR3 and FR4 of the at least one VL region of the antigen-binding domain 1, comprise respectively the sequences of SEQ ID NO: 35, 36, 37 and 38 or functionally equivalent variants thereof.

In another embodiment, the FR1, FR2, FR3 and FR4 of the at least one VH region of the antigen-binding domain 1 comprise respectively the sequences of SEQ ID NO: 31, 32, 33 and 34, SEQ ID NO: 65, 66, 67 and 68, SEQ ID NO: 73, 74, 75 and 76 or SEQ ID NO: 81, 82, 83 and 84 or functionally equivalent variants thereof and the FR1, FR2, FR3, and FR4 of the at least one VL region of the antigen-binding domain 1 comprise respectively the sequences of SEQ ID NO: 35, 36, 37 and 38, SEQ ID NO: 69, 70, 71, and 72, SEQ ID NO: 77, 78, 79, and 80 or SEQ ID NO: 85, 86, 87 and 88 or functionally equivalent variants thereof.

In another particular embodiment, the FR1, FR2, FR3 and FR4 of the at least one VH region of the antigen-binding domain 2 comprise respectively the sequences of SEQ ID NO: 42, 43, 44 and 45 or functionally equivalent variants thereof and FR1, FR2, FR3 and FR4 of the at least VL region of the antigen-binding domain 2 comprise respectively the sequences of SEQ ID NO: 46, 47, 48 and 49 or functionally equivalent variants thereof.

In another embodiment, the FR1, FR2, FR3 and FR4 of the at least one VH region of the antigen binding domain 2 comprise respectively the sequences of SEQ ID NO: 42, 43, 44 and 45, SEQ ID NO: 89, 90, 91 and 92, SEQ ID NO: 97, 98, 99 and 100 or SE ID NO: 105, 106, 107 and 108 or functionally equivalent variants thereof and the FR1, FR2, FR3 and FR4 of the at least one VL region of the antigen comprise respectively the sequences of SEQ ID NO: 46, 47, 48 and 49, SEQ ID NO: 93, 94, 95 or 96, SEQ ID NO: 109, 110, 111 and 112 or functionally equivalent variants thereof.

As it is used herein, the term "functionally equivalent variant of a FR sequence" refers to a sequence variant of a particular FR sequence having substantially similar sequence identity with it and substantially maintaining its capacity to bind to its cognate antigen when being part of an antibody or antibody-binding domains described herein. For example, a functionally equivalent variant of a FR sequence may be a polypeptide sequence derivative of said sequence comprising the addition, deletion or substitution of one or more amino acids.

Functionally equivalent variants of a FR sequence according to the invention include FR sequences having at least approximately 70% , at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of the above reference sequences. It is also contemplated that functionally equivalent variants of a FR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a FR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 19-26, 21-38 and 42-49 to bind to its cognate antigen when being part of an antigen-binding domain of the invention. This capacity to bind to its cognate antigen may be determined as a value of affinity, avidity, specificity and/or selectivity of the antibody or antibody fragment to its cognate antigen In an embodiment the VL of the ScFv1 comprises the sequences of SEQ ID NO: 27 or 180 or a functionally equivalent variant thereof and the VH of the ScFc1 comprises the sequence of SEQ ID NO: 28 or 181 of a functionally equivalent variant thereof.

In an embodiment the at least one VL of the antigen-binding domain 1 comprises the sequence of SEQ ID NO: 39 or a functionally equivalent variant thereof and the VH of the antigen-binding domain 1 comprises the sequence of SEQ ID NO: 40 or 183 of a functionally equivalent variant thereof.

In another embodiment, the at least one VL region of the antigen-binding domain 1 comprises the sequence of SEQ ID NO: 39, 54, 56 or 58 or functionally equivalent variants thereof and the at least one VH region of the antigen-binding domain 1 comprises the sequence of SEQ ID NO: 40, 183, 53, 55 or 57 or functionally equivalent variants thereof,

In an embodiment the at least one VL of the antigen-binding domain 2 comprises the sequence of SEQ ID NO: 50 or 184 or a functionally equivalent variant thereof and the at least one VH of the antigen-binding domain 2 comprises the sequence of SEQ ID NO: 51 of a functionally equivalent variant thereof.

In another embodiment, the at least one VL region of the antigen-binding doiam 2 comprises the sequence of SEQ ID NO: 50 ,184, 60, 62 or 64 and the at least one VH regions of the antigen-binding domain 2 comprises the sequence of selected from SEQ ID NO: 51, 59, 61 and 63. or functionally equivalent variants thereof.

Preferred embodiments of the VL and VH regions of ScFv1 are as defined below:
1. In one embodiment, the VL of the ScFv1 according to the invention is characterized in that the CDR1 region:
   1.1. does not contain the sequence KASQNVGTAVA (SEQ ID NOs 10 or 16) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQSVGTAVA (SEQ ID NO: 4) or with the sequence RASQSVGTAVA (SEQ ID NO: 175).
   1.2. does not contain an Asn residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQSVGTAVA (SEQ ID NO: 4) or with the sequence RASQSVGTAVA (SEQ ID NO: 175).
   1.3. differs in at least 1, at least 2, at least 3, at least 4, at least 5, at least 6, at least 7, at least 8, at least 9 or at least 10 amino acids with respect to the sequence of KASQSVGTAVA (SEQ ID NO: 4) or with the sequence RASQSVGTAVA (SEQ ID NO: 175) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQSVGTAVA (SEQ ID NO: 4) or with the sequence RASQSVGTAVA (SEQ ID NO: 175).
   1.4. does not contain the sequence KASQNVGTAVA (SEQ ID NOs 10 or 16) and in that at least 1, at least 2, at least 3, at least 4, at least 5, at least 6, at least 7, at least 8, at least 9, at least 10 amino acids or all amino acids are conservative substitutions of the amino acids found in the corresponding positions in the sequence KASQSVGTAVA (SEQ ID NO: 4) or with the sequence RASQSVGTAVA (SEQ ID NO: 175).
   1.5. does not contain the sequence KASQNVGTAVA (SEQ ID NOs 10 or 16) and in that it contains at least 1, at least 2, at least 3, at least 4, at least 5, at least 6, at least 7, at least 8, at least 9 or at least 10 amino acids in common with the sequence of KASQSVGTAVA (SEQ ID NO: 4) or with the sequence RASQSVGTAVA (SEQ ID NO: 175).
2. In one embodiment, the VL of the ScFv1 according to the invention is characterized in that the CDR2 region
   2.1. does not contain the sequence SASNRYT (SEQ ID NOs: 11 or 17) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRFT (SEQ ID NO: 5).
   2.2. does not contain a Tyr residue at position 6 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRFT (SEQ ID NO: 5).
   2.3. differs in at least 1, at least 2, at least 3, at least 4 or at least 5 amino acids with respect to the sequence of SASNRFT (SEQ ID NO: 5) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRFT (SEQ ID NO: 5).
   2.4. does not contain the sequence SASNRYT (SEQ ID NOs: 11 or 17) and in that at least 1, at least 2, at least 3, at least 4, at least 5 or all amino acids are conservative substitutions of the amino acids found in the corresponding positions in the sequence SASNRFT (SEQ ID NO: 5).
   2.5. does not contain the sequence SASNRYT (SEQ ID NOs: 11 or 17) and in that it contains at least 1, at least 2, at least 3, at least 4 or at least 5 amino acids in common with the sequence of SASNRFT (SEQ ID NO: 5).
3. In one embodiment, the VL of the ScFv1 according to the invention is characterized in that the CDR3 region
   3.1. does not contain the sequence contain the sequence QQYSTYPLT (SEQ ID NO: 12) or the sequence QQYSSYPLT (SEQ ID NO: 18) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSTYPLA (SEQ ID NO: 6),
   3.2. does not contain a Thr residue at position 9 and/or a Ser residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSTYPLA (SEQ ID NO: 6),
   3.3. differs in at least 1, at least 2, at least 3, at least 4, at least 5, at least 6, at least 7 or at least 8 amino acids with respect to the sequence of QQYSTYPLA (SEQ ID NO: 6) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSTYPLA (SEQ ID NO: 6).
   3.4. does not contain the sequence QQYSTYPLT (SEQ ID NO: 12) or the sequence QQYSSYPLT (SEQ ID NO: 18) and in that at least 1, at least 2, at least 3, at least 4, at least 5, at least 6, at least 7, at least 8 or all amino acids are conservative substitutions of the amino acids found in the corresponding positions in the sequence QQYSTYPLA (SEQ ID NO: 6).
   3.5. does not contain the sequence QQYSTYPLT (SEQ ID NO: 12) or the sequence QQYSSYPLT (SEQ ID NO: 18)and in that it contains at least 1, at least 2, at least 3, at least 4, at least 5 amino acids, at least 6, at least 7 or at least 8 amino acids in common with the sequence of QQYSTYPLA (SEQ ID NO: 6).
4. In one embodiment, the VH of the ScFv1 according to the invention is characterized in that the CDR1 region
   4.1. does not contain the sequence TYGMA (SEQ ID NO: 7) or the sequence DYGMS (SEQ ID NO: 13) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DFGMS (SEQ ID NO: 1),
   4.2. does not contain a Thr residue at position 1, a Tyr residue at position 2 and/or an Ala residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DFGMS (SEQ ID NO: 1),
   4.3. differs in at least 1, at least 2, at least 3, at least 4 or at least 5 amino acids with respect to the sequence of DFGMS (SEQ ID NO: 1) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DFGMS (SEQ ID NO: 1).
   4.4. does not contain the sequence TYGMA (SEQ ID NO: 7) or the sequence DYGMS (SEQ ID NO: 13) and in that at least 1, at least 2, at least 3, at least 4 or at least 5 amino acids are conservative substitutions of the amino acids found in the corresponding positions in the sequence DFGMS (SEQ ID NO: 1).
   4.5. does not contain the sequence TYGMA (SEQ ID NO: 7) or the sequence DYGMS (SEQ ID NO: 13) and in that it contains at least 1, at least 2, at least 3 or at least 4 amino acids in common with the sequence of DFGMS (SEQ ID NO: 1).
5. In one embodiment, the VH of the ScFv1 according to the invention is characterized in that the CDR2 region
   5.1. does not contain the sequence TINSNGGKTYHPDSVKG (SEQ ID NO: 8) or the sequence TINGNGVKIYYVDSVKG (SEQ ID NO: 14) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINTNGGTTHYPDNVKG (SEQ ID NO: 2) or with the sequence TINTNGGTTHYPDSVKG (SEQ ID NO: 174),
   5.2. does not contain a Ser or Gly residue at position 4, a Val residue at position 7, a Lys residue at position 8, a IIe residue at position 9, a Tyr residue at position 10, at His residue at position 11, a Val reside at position 12 and and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINTNGGTTHYPDNVKG (SEQ ID NO: 2) or with the sequence TINTNGGTTHYPDSVKG (SEQ ID NO: 174),
   5.3. differs in at least 1, at least 2, at least 3, at least 4, at least 5, at least 6, at least 7, at least 8, at least 9, at least 10, at least 11, at least 12, at least 13, at least 14, at least 15 or at least 16 amino acids with respect to the sequence of TINTNGGTTHYPDNVKG (SEQ ID NO: 8) or with the sequence TINTNGGTTHYPDSVKG (SEQ ID NO: 14) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINTNGGTTHYPDNVKG (SEQ ID NO: 2) or with the sequence TINTNGGTTHYPDSVKG (SEQ ID NO: 174).
   5.4. does not contain the sequence TINSNGGKTYHPDSVKG (SEQ ID NO: 8) or the sequence TINGNGVKIYYVDSVKG (SEQ ID NO: 14) and at least 1, at least 2, at least 3, at least 4, at least 5, at least 6, at least 7, at least 8, at least 9, at least 10, at least 11, at least 12, at least 13, at least 14, at least 15, at least 16 amino acids or all are conservative substitutions of the amino acids found in the corresponding positions in the sequence TINTNGGTTHYPDNVKG (SEQ ID NO: 2) or with the sequence TINTNGGTTHYPDSVKG (SEQ ID NO: 174).
   5.5. does not contain the sequence TINSNGGKTYHPDSVKG (SEQ ID NO: 8) or the sequence TINGNGVKIYYVDSVKG (SEQ ID NO: 14) and in that it contains at least 1, at least 2, at least 3, at least 4, at least 5, at least 6, at least 7, at least 8, at least 9, at least 10, at least 11, at least 12, at least 13, at least 14, at least 15 or at least 16 amino acids contains at least 1, at least 2, at least 3, at least 4, at least 5 amino acids, at least 6, at least 7 or at least 8 amino acids in common with the sequence of TINTNGGTTHYPDNVKG (SEQ ID NO: 2) or with the sequence TINTNGGTTHYPDSVKG (SEQ ID NO: 174).,
6. In one embodiment, the VH of the ScFv1 according to the invention is characterized in that the CDR3 region
   6.1. does not contain the sequence EGFDY (SEQ ID NO: 9 or 15) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGLDY (SEQ ID NO: 3),
   6.2. does not contain a Phe residue at position 3 and, optionally, it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGLDY (SEQ ID NO: 3),
   6.3. differs in at least 1, at least 2, at least 3, at least 4 or at least 5 amino acids with respect to the sequence of EGLDY (SEQ ID NO: 3) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGLDY (SEQ ID NO: 3).
   6.4. does not contain the sequence EGFDY (SEQ ID NO: 9 or 15) and in that at least 1, at least 2, at least 3, at least 4 or at least 5 amino acids are conservative substitutions of the amino acids found in the corresponding positions in the sequence EGLDY (SEQ ID NO: 3).
   6.5. does not contain the sequence EGFDY (SEQ ID NO: 9 or 15) and in that it contains at least 1, at least 2, at least 3 or at least 4 amino acids in common with the sequence of EGLDY (SEQ ID NO: 3).

Preferred embodiments of the VL and VH regions of the antigen-binding domain 1 are as defined below:
1. In one embodiment, the VL of the antigen-binding domain 1 according to the invention is characterized in that the CDR1 region
   1.1. does not contain the sequence KASQSVGTAVA (SEQ ID NOs: 4) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNVGTAVA (SEQ ID NO: 10).
   1.2. does not contain an Ser residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNVGTAVA (SEQ ID NO: 10).
   1.3. differs in at least 1, at least 2, at least 3, at least 4, at least 5, at least 6, at least 7, at least 8, at least 9 or at least 10 amino acids with respect to the sequence of KASQNVGTAVA (SEQ ID NO: 10) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNVGTAVA (SEQ ID NO: 10).
   1.4. does not contain the sequence KASQSVGTAVA (SEQ ID NO: 4) and in that at least 1, at least 2, at least 3, at least 4, at least 5, at least 6, at least 7, at least 8, at least 9, at least 10 amino acids or all amino acids are conservative substitutions of the amino acids found in the corresponding positions in the sequence KASQNVGTAVA (SEQ ID NO: 10).
   1.5. does not contain the sequence KASQSVGTAVA (SEQ ID NO: 4) and in that it contains at least 1, at least 2, at least 3, at least 4, at least 5, at least 6, at least 7, at least 8, at least 9 or at least 10 amino acids in common with the sequence of KASQNVGTAVA (SEQ ID NO: 10).
2. In one embodiment, the VL of the antigen-binding domain 1 according to the invention is characterized in that the CDR2 region
   2.1. does not contain the sequence SASNRFT (SEQ ID NO: 5) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRYT (SEQ ID NO: 11).
   2.2. does not contain a Phe residue at position 6 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRYT (SEQ ID NO: 11).
   2.3. differs in at least 1, at least 2, at least 3, at least 4 or at least 5 amino acids with respect to the sequence of SASNRYT (SEQ ID NO: 11) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRYT (SEQ ID NO: 11).
   2.4. does not contain the sequence SASNRFT (SEQ ID NO: 5) and in that at least 1, at least 2, at least 3, at least 4, at least 5 or all amino acids are conservative substitutions of the amino acids found in the corresponding positions in the sequence SASNRYT (SEQ ID NO: 11).
   2.5. does not contain the sequence SASNRFT (SEQ ID NO: 5) and in that it contains at least 1, at least 2, at least 3, at least 4 or at least 5 amino acids in common with the sequence of SASNRYT (SEQ ID NO: 11).
3. In one embodiment, the VL of the antigen-binding domain 1 according to the invention is characterized in that the CDR3 region
   3.1. does not contain the sequence QQYSTYPLA (SEQ ID NO: 6) or the sequence QQYSSYPLT (SEQ ID NO: 18) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSTYPLT (SEQ ID NO: 12),
   3.2. does not contain a Ala residue at position 9 and/or a serine residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSTYPLT (SEQ ID NO: 12),
   3.3. differs in at least 1, at least 2, at least 3, at least 4, at least 5, at least 6, at least 7 or at least 8 amino acids with respect to the sequence of QQYSTYPLT (SEQ ID NO: 12) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSTYPLT (SEQ ID NO: 12).
   3.4. does not contain the sequence QQYSTYPLA (SEQ ID NO: 6) or the sequence QQYSSYPLT (SEQ ID NO: 18) and in that at least 1, at least 2, at least 3, at least 4, at least 5, at least 6, at least 7, at least 8 or all amino acids are conservative substitutions of the amino acids found in the corresponding positions in the sequence QQYSTYPLT (SEQ ID NO: 12).
   3.5. does not contain the sequence QQYSTYPLA (SEQ ID NO: 6) or the sequence QQYSSYPLT (SEQ ID NO: 18) and in that it contains at least 1, at least 2, at least 3, at least 4, at least 5 amino acids, at least 6, at least 7 or at least 8 amino acids in common with the sequence of QQYSTYPLT (SEQ ID NO: 12).
4. In one embodiment, the VH of the antigen-binding domain 1 according to the invention is characterized in that the CDR1 region
   4.1. does not contain the sequence DFGMS (SEQ ID NO: 1) or the sequence DYGMS (SEQ ID NO: 13) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TYGMA (SEQ ID NO: 7) or with the sequence SYGMA (SEQ ID NO: 176),
   4.2. does not contain a Asp residue at position 1, a Phe residue at position 2 and/or an Ser residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TYGMA (SEQ ID NO: 7) or with the sequence SYGMA (SEQ ID NO: 176),
   4.3. differs in at least 1, at least 2, at least 3, at least 4 or at least 5 amino acids with respect to the sequence of TYGMA (SEQ ID NO: 7) or with the sequence SYGMA (SEQ ID NO: 176) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TYGMA (SEQ ID NO: 7) or with the sequence SYGMA (SEQ ID NO: 176),
   4.4. does not contain the sequence DFGMS (SEQ ID NO: 1) or the sequence DYGMS (SEQ ID NO: 13) and in that at least 1, at least 2, at least 3, at least 4 or at least 5 amino acids are conservative substitutions of the amino acids found in the corresponding positions in the sequence TYGMA (SEQ ID NO: 7) or with the sequence SYGMA (SEQ ID NO: 176),
   4.5. does not contain the sequence DFGMS or the sequence DYGMS and in that it contains at least 1, at least 2, at least 3 or at least 4 amino acids in common with the sequence of TYGMA (SEQ ID NO: 7) or with the sequence SYGMA (SEQ ID NO: 176).
5. In one embodiment, the VH of the antigen-binding domain 1 according to the invention is characterized in that the CDR2 region
   5.1. does not contain the sequence TINTNGGTTHYPDNVKG (SEQ ID NO: 2) or the sequence TINGNGVKIYYVDSVKG (SEQ ID NO: 14) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINSNGGKTYHPDSVKG (SEQ ID NO: 8) or with the sequence AINSNGGKTYYADSVKG (SEQ ID NO: 177),
   5.2. does not contain a Thr or Gly residue at position 4, a Val residue at position 7, a Thr at position 8, a IIe residue at position 9, a His residue at position 10, a Val residue at position 12 and/or a Asn residue at position 14 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINSNGGKTYHPDSVKG (SEQ ID NO: 8) or with the sequence AINSNGGKTYYADSVKG (SEQ ID NO: 177),
   5.3. differs in at least 1, at least 2, at least 3, at least 4, at least 5, at least 6, at least 7, at least 8, at least 9, at least 10, at least 11, at least 12, at least 13, at least 14, at least 15 or at least 16 amino acids with respect to the sequence of TINSNGGKTYHPDSVKG (SEQ ID NO: 8) or with the sequence AINSNGGKTYYADSVKG (SEQ ID NO: 177) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINSNGGKTYHPDSVKG (SEQ ID NO: 8) or with the sequence AINSNGGKTYYADSVKG (SEQ ID NO: 177).
   5.4. does not contain the sequence TINTNGGTTHYPDNVKG (SEQ ID NO: 2) or the sequence TINGNGVKIYYVDSVKG (SEQ ID NO: 14) and at least 1, at least 2, at least 3, at least 4, at least 5, at least 6, at least 7, at least 8, at least 9, at least 10, at least 11, at least 12, at least 13, at least 14, at least 15, at least 16 amino acids or all are conservative substitutions of the amino acids found in the corresponding positions in the sequence TINSNGGKTYHPDSVKG (SEQ ID NO: 8) or with the sequence AINSNGGKTYYADSVKG (SEQ ID NO: 177).
   5.5. does not contain the sequence TINTNGGTTHYPDNVKG (SEQ ID NO: 2) or the sequence TINGNGVKIYYVDSVKG (SEQ ID NO: 14) and in that it contains at least 1, at least 2, at least 3, at least 4, at least 5, at least 6, at least 7, at least 8, at least 9, at least 10, at least 11, at least 12, at least 13, at least 14, at least 15 or at least 16 amino acids contains at least 1, at least 2, at least 3, at least 4, at least 5 amino acids, at least 6, at least 7 or at least 8 amino acids in common with the sequence of TINSNGGKTYHPDSVKG (SEQ ID NO: 8) or with the sequence AINSNGGKTYYADSVKG (SEQ ID NO: 177).,
6. In one embodiment, the VH of the antigen-binding domain 1 according to the invention is characterized in the CDR3 region
   6.1. does not contain the sequence EGLDY (SEQ ID NO: 3) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGFDY (SEQ ID NO: 9) or with the sequence DY,
   6.2. does not contain a Leu residue at position 3 and, optionally, it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGFDY (SEQ ID NO: 9) or with the sequence DY,
   6.3. differs in at least 1, at least 2, at least 3, at least 4 or at least 5 amino acids with respect to the sequence of EGFDY (SEQ ID NO: 9) or with the sequence DY and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGFDY (SEQ ID NO: 9) or with the sequence DY.
   6.4. does not contain the sequence EGLDY (SEQ ID NO: 3) and in that at least 1, at least 2, at least 3, at least 4 or at least 5 amino acids are conservative substitutions of the amino acids found in the corresponding positions in the sequence EGFDY (SEQ ID NO: 9) or with the sequence DY.
   6.5. does not contain the sequence EGLDY (SEQ ID NO: 3) and in that it contains at least 1, at least 2, at least 3 or at least 4 amino acids in common with the sequence of EGFDY (SEQ ID NO: 9) or with the sequence DY.

Preferred embodiments of the VL and VH regions of the antigen-binding domain 2 are as defined below.
1. In one embodiment, the VL of the antigen-binding domain 2 according to the invention is characterized in that the CDR1 region
   1.1. does not contain the sequence KASQSVGTAVA (SEQ ID NO: 4) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNVGTAVA (SEQ ID NO: 16) or the sequence RASQNVGTAVA (SEQ ID NO: 179)
   1.2. does not contain a Ser residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNVGTAVA (SEQ ID NO: 16) or the sequence RASQNVGTAVA (SEQ ID NO: 179).
   1.3. differs in at least 1, at least 2, at least 3, at least 4, at least 5, at least 6, at least 7, at least 8, at least 9 or at least 10 amino acids with respect to the sequence of KASQNVGTAVA (SEQ ID NO: 16) or the sequence RASQNVGTAVA (SEQ ID NO: 179) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence KASQNVGTAVA (SEQ ID NO: 16) or the sequence RASQNVGTAVA (SEQ ID NO: 179).
   1.4. does not contain the sequence KASQSVGTAVA (SEQ ID NO: 4) and in that at least 1, at least 2, at least 3, at least 4, at least 5, at least 6, at least 7, at least 8, at least 9, at least 10 amino acids or all amino acids are conservative substitutions of the amino acids found in the corresponding positions in the sequence KASQNVGTAVA (SEQ ID NO: 16) or the sequence RASQNVGTAVA (SEQ ID NO: 179).
   1.5. does not contain the sequence KASQSVGTAVA (SEQ ID NO: 4) and in that it contains at least 1, at least 2, at least 3, at least 4, at least 5, at least 6, at least 7, at least 8, at least 9 or at least 10 amino acids in common with the sequence of KASQNVGTAVA (SEQ ID NO: 16) or the sequence RASQNVGTAVA (SEQ ID NO: 179).
2. In one embodiment, the VL of the antigen-binding domain 2 according to the invention is characterized in that the CDR2 region
   2.1. does not contain the sequence SASNRFT (SEQ ID NO: 5) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRYT (SEQ ID NO: 17).
   2.2. does not contain a Phe residue at position 6 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRYT (SEQ ID NO: 17).
   2.3. differs in at least 1, at least 2, at least 3, at least 4 or at least 5 amino acids with respect to the sequence of SASNRYT (SEQ ID NO: 17) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence SASNRYT (SEQ ID NO: 17).
   2.4. does not contain the sequence SASNRFT (SEQ ID NO: 5) and in that at least 1, at least 2, at least 3, at least 4, at least 5 or all amino acids are conservative substitutions of the amino acids found in the corresponding positions in the sequence SASNRYT (SEQ ID NO: 17).
   2.5. does not contain the sequence SASNRFT (SEQ ID NO: 5) and in that it contains at least 1, at least 2, at least 3, at least 4 or at least 5 amino acids in common with the sequence of SASNRYT (SEQ ID NO: 17).
3. In one embodiment, the VL of the antigen-binding domain 2 according to the invention is characterized in that the CDR3 region
   3.1. does not contain the sequence contain the sequence QQYSTYPLA (SEQ ID NO: 6) or the sequence QQYSTYPLT (SEQ ID NO: 12) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSSYPLT (SEQ ID NO: 18),
   3.2. does not contain an Ala residue at position 9 and/or a Thr residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSSYPLT (SEQ ID NO: 18),
   3.3. differs in at least 1, at least 2, at least 3, at least 4, at least 5, at least 6, at least 7 or at least 8 amino acids with respect to the sequence of QQYSSYPLT (SEQ ID NO: 18) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQYSSYPLT (SEQ ID NO: 18).
   3.4. does not contain the sequence QQYSTYPLA (SEQ ID NO: 6) or the sequence QQYSTYPLT (SEQ ID NO: 12) and in that at least 1, at least 2, at least 3, at least 4, at least 5, at least 6, at least 7, at least 8 or all amino acids are conservative substitutions of the amino acids found in the corresponding positions in the sequence QQYSSYPLT (SEQ ID NO: 18).
   3.5. does not contain the sequence QQYSTYPLA (SEQ ID NO: 6)or the sequence QQYSTYPLT (SEQ ID NO: 12 and in that it contains at least 1, at least 2, at least 3, at least 4, at least 5 amino acids, at least 6, at least 7 or at least 8 amino acids in common with the sequence of QQYSSYPLT (SEQ ID NO: 18).
4. In one embodiment, the VH of the antigen-binding domain 2 according to the invention is characterized in that the CDR1 region
   4.1. does not contain the sequence TYGMA (SEQ ID NO: 7) or the sequence DFGMS (SEQ ID NO: 1) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DYGMS (SEQ ID NO: 13),
   4.2. does not contain a Thr residue at position 1, a Phe residue at position 2 and/or an Ala residue at position 5 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DYGMS (SEQ ID NO: 13),
   4.3. differs in at least 1, at least 2, at least 3, at least 4 or at least 5 amino acids with respect to the sequence of DYGMS (SEQ ID NO: 13) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DYGMS (SEQ ID NO: 13).
   4.4. does not contain the sequence TYGMA (SEQ ID NO: 7) or the sequence DFGMS (SEQ ID NO: 1) and in that at least 1, at least 2, at least 3, at least 4 or at least 5 amino acids are conservative substitutions of the amino acids found in the corresponding positions in the sequence DYGMS (SEQ ID NO: 13).
   4.5. does not contain the sequence TYGMA (SEQ ID NO: 7) or the sequence DFGMS (SEQ ID NO: 1) and in that it contains at least 1, at least 2, at least 3 or at least 4 amino acids in common with the sequence of DYGMS (SEQ ID NO: 13).
5. In one embodiment, the VH of the antigen-binding domain 2 according to the invention is characterized in that the CDR2 region
   5.1. does not contain the sequence TINSNGGKTYHPDSVKG (SEQ ID NO: 8) or the sequence TINTNGGTTHYPDNVKG (SEQ ID NO: 2) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINGNGVKIYYVDSVKG (SEQ ID NO: 14),
   5.2. does not contain a Ser or Thr residue at position 4, a Gly residue at position 7, a Thr residue at position 8, a Thr residue at position 9, a His residue at position 10, at His residue at position 11, a Pro reside at position 12 and/or an Asn residue at position 14 and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINGNGVKIYYVDSVKG (SEQ ID NO: 14),
   5.3. differs in at least 1, at least 2, at least 3, at least 4, at least 5, at least 6, at least 7, at least 8, at least 9, at least 10, at least 11, at least 12, at least 13, at least 14, at least 15 or at least 16 amino acids with respect to the sequence of TINGNGVKIYYVDSVKG (SEQ ID NO: 14) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence TINGNGVKIYYVDSVKG (SEQ ID NO: 14).
   5.4. does not contain the sequence TINSNGGKTYHPDSVKG (SEQ ID NO: 8) or the sequence TI NTNGGTTHYPDNVKG (SEQ ID NO: 2) and at least 1, at least 2, at least 3, at least 4, at least 5, at least 6, at least 7, at least 8, at least 9, at least 10, at least 11, at least 12, at least 13, at least 14, at least 15, at least 16 amino acids or all are conservative substitutions of the amino acids found in the corresponding positions in the sequence TINGNGVKIYYVDSVKG (SEQ ID NO: 14).
   5.5. does not contain the sequence TINSNGGKTYHPDSVKG (SEQ ID NO: 8) or the sequence TINTNGGTTHYPDNVKG (SEQ ID NO: 2) and in that it contains at least 1, at least 2, at least 3, at least 4, at least 5, at least 6, at least 7, at least 8, at least 9, at least 10, at least 11, at least 12, at least 13, at least 14, at least 15 or at least 16 amino acids contains at least 1, at least 2, at least 3, at least 4, at least 5 amino acids, at least 6, at least 7 or at least 8 amino acids in common with the sequence of TINGNGVKIYYVDSVKG (SEQ ID NO: 14).,
6. In one embodiment, the VH of the antigen-binding domain 2 according to the invention is characterized in that the CDR3 region
   6.1. does not contain the sequence EGLDY (SEQ ID NO: 3) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGFDY (SEQ ID NO: 15),
   6.2. does not contain a Leu residue at position 3 and, optionally, it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGFDY (SEQ ID NO: 15),
   6.3. differs in at least 1, at least 2, at least 3, at least 4 or at least 5 amino acids with respect to the sequence of EGFDY (SEQ ID NO: 15) and, optionally, in that it shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence EGFDY (SEQ ID NO: 15).
   6.4. does not contain the sequence EGLDY (SEQ ID NO: 3) and in that at least 1, at least 2, at least 3, at least 4 or at least 5 amino acids are conservative substitutions of the amino acids found in the corresponding positions in the sequence EGFDY (SEQ ID NO: 15).
   6.5. does not contain the sequence EGLDY (SEQ ID NO: 3) and in that it contains at least 1, at least 2, at least 3 or at least 4 amino acids in common with the sequence of EGFDY (SEQ ID NO: 15).

In another embodiment, the ScFv1 comprises the sequence of SEQ ID NO: 30 or 182 or a functionally equivalent thereof.

In another embodiment, the antigen-binding domain 1 comprises the sequence SEQ ID NO: 41 or 185 or a functionally equivalent thereof.

In another embodiment, the antigen-binding domain 1 comprises the sequence of SEQ ID NO: 41, 185, 187, 188 or 189 .or functionally equivalent variants thereof.

In another embodiment, the antigen-binding domain 2 comprises the sequence SEQ ID NO: 52 or 186 or a functionally equivalent thereof.

In another embodiment, the antigen-binding domain 2 comprises the sequence of SEQ ID NO: 52, 186, 190, 191 or 192. or functionally equivalent variants thereof.

In a particular embodiment, the VL of the ScFv1 comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 27 or 180. In a preferred embodiment, the VL of the ScFv1 comprises a sequence of SEQ ID NO: 27 or 180 or a functionally equivalent variant having at least 85% sequence identity with SEQ ID NO: 27 or 180.

In a particular embodiment, the VH of the ScFv1 comprises sequences having at least 67%, at least 68%, at least 69%, at least 70%, at least 71%, at least 72%, at least 73%, at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 28 or 181. In a preferred embodiment, the VH of the ScFv1 comprises a sequence of SEQ ID NO: 28 or 181 or a functionally equivalent variant having at least 67% sequence identity with SEQ ID NO: 28 or 181.

In a particular embodiment, the ScFv1 comprises the sequence of SEQ ID NO: 28 or a functionally equivalent variant thereof. In another embodiment, the ScFv1 comprises sequences having at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 30 or 182. In a preferred embodiment, the ScFv1 comprises a sequence of SEQ ID NO: 30 or 182 or a functionally equivalent variant having at least 76% sequence identity with SEQ ID NO: 30 or 182.

In another particular embodiment the at least one VL of the antigen-binding domain 1 comprises the sequence of SEQ ID NO: 39 and the at least one VH of the antigen-binding domain 1 comprises the sequence of SEQ ID NO: 40 or 183 or a functionally equivalent variant thereof.

In a particular embodiment, the at least one VL of the antigen-binding domain 1 comprises sequences having at least at least 89%, at least 90%, at least 91%, at least 92%, at least 93%, at least 94%, at least 95%, at least 96%, at least 97%, at least 98%, at least 99% sequence identity with SEQ ID NO: 39. In a preferred embodiment, the at least one VL of the antigen-binding domain 1 comprises a sequence of SEQ ID NO: 39 or a functionally equivalent variant having at least 39% sequence identity with SEQ ID NO: 39.

In a particular embodiment, the at least one VH of the antigen-binding domain 1 comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 40 or 183. In a preferred embodiment, the at least one VH of the antigen-binding domain 1 comprises a sequence of SEQ ID NO: 40 or 183 or a functionally equivalent variant having at least 74% sequence identity with SEQ ID NO: 40 or 183.

In a particular embodiment, the antigen-binding domain 1 comprises the sequence of SEQ ID NO: 41 or 185 or a functionally equivalent variant thereof. In another embodiment, the antigen-binding domain 1 comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 41 or 185. In a preferred embodiment, the antigen-binding domain 1 comprises a sequence of SEQ ID NO: 41 or 185 or a functionally equivalent variant having at least 85% sequence identity with SEQ ID NO: 41 or 185.

In another particular embodiment the at least one VL of the antigen-binding domain 2 comprises the sequence of SEQ ID NO: 50 or 184 or a functionally equivalent thereof and the at least one VH of the antigen-binding domain 2 comprises the sequence of SEQ ID NO: 51 or a functionally equivalent variant thereof.

In a particular embodiment, the at least one VL of the antigen-binding domain 2 comprises sequences having at least, at least 96%, at least 97%, at least 98%, at least 99% sequence identity with SEQ ID NO: 50 or 184. In a preferred embodiment, the at least one VL of the antigen-binding domain comprises a sequence of SEQ ID NO: 50 or 184 or a functionally equivalent variant having at least 89% sequence identity with SEQ ID NO: 50 or 184.

In a particular embodiment, the at least one VH of the antigen-binding domain 2 comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 51. In a preferred embodiment, the at least one VH of the antigen-binding domain 2 comprises a sequence of SEQ ID NO: 51 or a functionally equivalent variant having at least 67% sequence identity with SEQ ID NO: 51.

In a particular embodiment, the antigen-binding 2 comprises the sequence of SEQ ID NO: 52 or 186 or a functionally equivalent variant thereof. In another embodiment, antigen-binding domain 2 comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 52 or 186. In a preferred embodiment, the antigen-binding domain comprises a sequence of SEQ ID NO: 52 or 186 or a functionally equivalent variant having at least 78% sequence identity with SEQ ID NO: 52 or 186.

In a particular embodiment, the VH and/or VL regions of the antigen-binding domains 1 and 2 of the CAR of the invention are humanized.

Thus, in a particular embodiment, the at least one VH region of the antigen-binding domain 1 comprises a humanized sequence selected from SEQ ID NOs: 53, 55 and 57 or functionally equivalent variants thereof and the at least one VL region of the antigen-binding domain 1 comprises a humanized sequence selected from SEQ ID NOs: 54, 56 and 58 or functionally equivalent variants thereof.

In a particular embodiment, the antigen-binding domain 1 comprises the humanized sequence selected from SEQ ID NOs: 187, 188 and 189.

In another embodiment, the at least one VH and VL regions of the antigen-binding domain 1 comprise humanized FR1, FR2, FR3 and FR4 regions, wherein the FR1, FR2, FR3 and FR4 of the at least one VH region comprise respectively the sequences SEQ ID NOs: 65, 66, 67, and 68 or functionally equivalent variants thereof and, the FR1, FR2, FR3 and FR4 of the at least VL region comprise respectively the sequences SEQ ID NOs: 69, 70, 71 and 72 or functionally equivalent variants thereof.

In another embodiment, the at least one VH and VL regions of the antigen-binding domain 1 comprise humanized FR1, FR2, FR3 and FR4 regions, wherein the FR1, FR2, FR3 and FR4 of the at least one VH region comprise respectively the sequences SEQ ID NOs: 73, 74, 75 and 76 or functionally equivalent variants thereof and, the FR1, FR2, FR3 and FR4 of the at least one VL region comprise respectively the sequences SEQ ID NOs: 77, 78, 79 and 80 or functionally equivalent variants thereof.

In another embodiment, the at least one VH and VL regions of the antigen-binding domain 1 comprise humanized FR1, FR2, FR3 and FR4 regions, wherein the FR1, FR2, FR3 and FR4 of the at least one VH region comprise respectively the sequences SEQ ID NOs: 81, 82, 83, and 84 or functionally equivalent variants thereof and, the FR1, FR2, FR3 and FR4 of the at least one VL region comprise respectively the sequences SEQ ID NOs: 85, 86, 87 and 88 or functionally equivalent variants thereof.

In another embodiment, the at least one VH region of the antigen-binding domain 1 of the CAR of the invention comprises at least one humanized FR region, at least 2 humanized FR regions, at least 3 humanized FR regions or at least 4 humanized FR regions, and wherein said FR regions are selected from:
FR1: SEQ ID NOs 65, 73 and 81;
FR2: SEQ ID NOs 66, 74 and 82;
FR3: SEQ ID NOs 67, 75 and 83; and
FR4: SEQ ID NOs 68, 76, and 84
or functionally equivalent variants thereof.

In another embodiment the at least one VL region of the antigen-binding domain 1 of the CAR of the invention comprises at least one humanized FR region, at least 2 humanized FR regions, at least 3 humanized FR regions or at least 4 humanized FR regions, and wherein said FR regions are selected from:
FR1: SEQ ID NOs 69, 77 and 85;
FR2: SEQ ID NOs 70, 78 and 86;
FR3: SEQ ID NOs 71, 79 and 87; and
FR4: SEQ ID NOs 72, 80, and 88
or functionally equivalent variants thereof.

Likewise, in a particular embodiment, the at least one VH region of the antigen-binding domain 2 comprises a humanized sequence selected from SEQ ID NOs: 59, 61 and 63 or functionally equivalent variants thereof and the at least one VH region of the antigen-binding domain 1 comprises a humanized sequence selected from SEQ ID NOs: 60, 62 and 64 or functionally equivalent variants thereof.

In another embodiment, the antigen-binding domain 2 comprises a humanized sequence selected form SEQ ID NOs: 190, 191 and 192.

In another embodiment, the at least one VH and VL regions of the antigen-binding domain 2 comprise humanized FR1, FR2, FR3 and FR4 regions, wherein the FR1, FR2, FR3 and FR4 of the at least one VH region comprise respectively the sequences SEQ ID NOs: 89, 90, 91, and 92 or functionally equivalent variants thereof and, the FR1, FR2, FR3 and FR4 of the at least one VL region comprise respectively the sequences SEQ ID NOs: 93, 94, 95 and 96 or functionally equivalent variants thereof.

In another embodiment, the at least one VH and VL regions of the antigen-binding domain 2 comprise humanized FR1, FR2, FR3 and FR4 regions, wherein the FR1, FR2, FR3 and FR4 of the at least one VH region comprise respectively the sequences SEQ ID NOs: 97, 98, 99 and 100 or functionally equivalent variants thereof and, the FR1, FR2, FR3 and FR4 of the at least one VL region comprise respectively the sequences SEQ ID NOs: 101, 102, 103 and 104 or functionally equivalent variants thereof.

In another embodiment, the at least one VH and VL regions of the antigen-binding domain 2 comprise humanized FR1, FR2, FR3 and FR4 regions, wherein the FR1, FR2, FR3 and FR4 of the at least one VH region comprise respectively the sequences SEQ ID NOs: 105, 106, 107, and 108 or functionally equivalent variants thereof and, the FR1, FR2, FR3 and FR4 of the at least one VL region comprise respectively the sequences SEQ ID NOs: 109, 110, 111 and 112 or functionally equivalent variants thereof.

In another embodiment the at least one VH region of the antigen-binding domain 2 of the CAR of the invention comprises at least one humanized FR region, at least 2 humanized FR regions, at least 3 humanized FR regions or at least 4 humanized FR regions. In some embodiments, the humanized FR regions are selected from the group consisting of SEQ ID NOs 89, 97 and 105 for FR1, SEQ ID NOs 90, 98 and 106 for FR2, SEQ ID NOs 91, 99 and 107 for FR3 and SEQ ID NOs 92, 100, and 108 for FR4 or functionally equivalent variants thereof.

In another embodiment the at least one VL region of the antigen-binding domain 2 of the CAR of the invention comprises at least one humanized FR region, at least 2 humanized FR regions, at least 3 humanized FR regions or at least 4 humanized FR regions. In some embodiments, the humanized FR regions are selected from the group consisting of SEQ ID NOs 93, 101 and 109 for FR1, SEQ ID NOs 94, 102 and 110 for FR2, SEQ ID NOs 95, 103 and 111 for FR3 and SEQ ID NOs 96, 104, and 112 for FR4 or a functionally equivalent variant thereof.

In some cases, the CAR includes a linker between any two adjacent domains. For example, a linker can be disposed between the transmembrane domain and the co-stimulatory domain of the antigen binding domain. As another example, a linker can be disposed between the antigen-binding domain and the intracellular signaling domain.

In a particular embodiment, when the antigen-binding domain is an ScFv, the VH and VL regions of the antigen-binding domain are connected by a linker region comprising SEQ ID NO: 29.

In an embodiment, the antigen-binding domain 1 is an ScFv and the VH and VL regions of the ScFv are connected by a linker regions comprising SEQ ID NO: 29. In anotaher embodiment, the antigen-binding domain 2 is an ScFv and the VH and VL regions of the ScFv are connected by a linker regions comprising SEQ ID NO: 29

In a particular embodiment, the linker is located between the VH and the VL regions of the ScFv. In a more particular embodiment the linker between the VH and the VL comprises the sequence SEQ ID NO: 29. In an embodiment, when the ScFv of the CARs of the invention is ScFv1, the ScFv comprises the structure VL-linker-VH. In another embodiment, when the antigen-binding domain 1 or 2 of the CARs of the invention is an ScFv, the ScFv may have the structure VH-linker-VL or VL-linker-VH. In a particular embodiment, the linker is located C-terminally with respect to the VL region and N-terminally with respect to the VH region, that is, VL-linker-VH.

The term "flexible polypeptide linker" or "linker" refers to a peptide linker that consists of amino acids such as glycine and/or serine residues used alone or in combination, to link variable heavy and variable light chain regions together; or to link any or the regions of the CAR of the invention.

The linker peptide may have any of a variety of amino acid sequences. Proteins can be joined by a spacer peptide, generally of a flexible nature, although other chemical linkages are not excluded. A linker can be a peptide of between about 6 and about 40 amino acids in length, or between about 6 and about 25 amino acids in length. These linkers can be produced by using synthetic, linker-encoding oligonucleotides to couple the proteins. Peptide linkers with a degree of flexibility can be used. The linking peptides may have virtually any amino acid sequence, bearing in mind that suitable linkers will have a sequence that results in a generally flexible peptide. The use of small amino acids, such as glycine and alanine, are of use in creating a flexible peptide. The creation of such sequences is routine to those of skill in the art.

Suitable linkers can be readily selected and can be of any of a suitable of different lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids.

Exemplary flexible linkers include the linker having the sequence TGSTSGSGKPGSGEGS (SEQ ID NO 29). Suitable linkers include as glycine polymers (G) n, glycine-serine polymers (including, for example, (GS) n, GSGGS n (SEQ ID NO: 117) and GGGS n (SEQ ID NO:118), where n is an integer of at least one), glycine-alanine polymers, alanine-serine polymers, and other flexible linkers known in the art. In a particular embodiment the linker comprises a glycine polymer of formula (G₄S)₃. Glycine and glycine-serine polymers are of interest since both of these amino acids are relatively unstructured, and therefore may serve as a neutral tether between components. Glycine polymers are of particular interest since glycine accesses significantly more phi-psi space than even alanine, and is much less restricted than residues with longer side chains. Exemplary flexible linkers include, but are not limited to GGSG (SEQ ID NO:119), GGSGG (SEQ ID NO:120), GSGSG (SEQ ID NO:121), GSGGG (SEQ ID NO:122), GGGSG (SEQ ID NO:123), GSSSG (SEQ ID NO:124), and the like. The ordinarily skilled artisan will recognize that design of a peptide conjugated to any elements described above can include linkers that are all or partially flexible, such that the linker can include a flexible linker as well as one or more portions that confer less flexible structure.

In another embodiment, the CAR of the invention further comprises a hinge domain between the antigen-binding domain and the transmembrane domain.

As used herein, "hinge domain", "hinge region" or "spacer" refers to an amino acid region that allows for separation and flexibility of the binding moiety and the T cell membrane. The length of the flexible hinges also allow for better binding to relatively inaccessible epitopes, e.g., longer hinge domains are allow for optimal binding. One skilled in the art will be able to determine the appropriate hinge for the given CAR target.

In some cases, the first polypeptide of the CAR according to the invention comprises a hinge domain, where the hinge domain is interposed between the antigen-binding domain and the transmembrane domain. In some cases, the hinge domain is an immunoglobulin heavy chain hinge domain. In some cases, the hinge domain is a domain region polypeptide derived from a receptor (e.g., a CD8-derived hinge domain).

The hinge domain can have a length of from about 10 amino acids to about 200 amino acids, preferably, between 50 and 150 amino acids, more preferably between 75 and 125 amino acids.

Exemplary spacers include glycine polymers (G) n, glycine-serine polymers (including, for example, (GS) n, (GSGGS) n (SEQ ID NO:125) and (GGGS) n (SEQ ID NO: 126), where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains. Exemplary spacers can comprise amino acid sequences including, but not limited to, GGSG (SEQ ID NO: 127), GGSGG (SEQ ID NO: 128), GSGSG (SEQ ID NO: 129), GSGGG (SEQ ID NO: 130), GGGSG (SEQ ID NO: 131), GSSSG (SEQ ID NO: 132), and the like.

In a particular embodiment the spacer comprises the amino acid sequence GQPREPQVYTLPPSREEMTKNQVSLTCLVKGFYPSDISVEWESNGQPENNYKTTPP MLDSDGSFFLYSKLTVDKSRWQQGNVFSCSVMHEALHNHYTQKSLSLSPGK (SEQ ID NO: 116)

In some cases, the hinge domain in the first polypeptide of a CAR according to the invention includes at least one cysteine. For example, in some cases, the hinge domain can include the sequence Cys-Pro-Pro-Cys (SEQ ID NO: 133). If present, a cysteine in the hinge domain of a first CAR can be available to form a disulfide bond with a hinge domain in a second CAR.

Immunoglobulin hinge domain amino acid sequences are known in the art; see, e.g., Tan et al. (1990) Proc. Natl. Acad. Sci. USA 87:162; and Huck et al. (1986) Nucl. Acids Res. 14:1779. As non-limiting examples, an immunoglobulin hinge domain can include one of the following amino acid sequences: DKTHT (SEQ ID NO: 134); CPPC (SEQ ID NO: 133); CPEPKSCDTPPPCPR (SEQ ID NO: 136) (see, e.g., Glaser et al. (2005) J. Biol. Chem. 280:41494); ELKTPLGDTTHT (SEQ ID NO: 137); KSCDKTHTCP (SEQ ID NO: 138); KCCVDCP (SEQ ID NO: 139); KYGPPCP (SEQ ID NO: 140); EPKSCDKTHTCPPCP (SEQ ID NO: 141) (human IgG1 hinge); ERKCCVECPPCP (SEQ ID NO: 142 (human IgG2 hinge); ELKTPLGDTTHTCPRCP (SEQ ID NO: 143) (human IgG3 hinge); SPNMVPHAHHAQ (SEQ ID NO: 144) (human IgG4 hinge); and the like.

The hinge domain can comprise an amino acid sequence of a human IgG1, IgG2, IgG3, or IgG4, hinge domain. The hinge domain can include one or more amino acid substitutions and/or insertions and/or deletions compared to a wild-type (naturally-occurring) hinge domain. For example, His 229 of human IgG1 hinge can be substituted with Tyr, so that the hinge domain comprises the sequence EPKSCDKTYTCPPCP (SEQ ID NO: 145); see, e.g., Yan et al. (2012) J. Biol. Chem. 287:5891.

The hinge domain can comprise an amino acid sequence derived from human CD8; e.g., the hinge domain can comprise the amino acid sequence: TTTPAPRPPTPAPTIASQPLSLRPEACRPAAGGAVHTRGLDFACD (SEQ ID NO: 146), or a variant thereof.

In a particular embodiment, the hinge domain is the CD8 hinge domain.

In another embodiment, the CAR of the invention includes from the N-terminus to the C-terminus an anti-p95HER2 light chain variable domain, a linker domain, an anti-p95HER2 heavy chain variable domain, a CD8, a hinge domain, a CD28 transmembrane domain, a CD28 intracellular co-stimulatory signaling domain followed by a CD3 zeta intracellular signaling domain.

In a particular embodiment, the hinge domain is the CD8 hinge domain, the transmembrane domain is the CD28 transmembrane domain and the intracellular signaling domain is the CD28 costimulatory domain.

In a particular embodiment, the CAR of the invention comprises the CD8 hinge domain, the CD28 transmembrane domain and the CD3 zeta intracellular signaling domain and the CD28 costimulatory domain.

### Nucleic acids and host cells related to the CARs of the invention

In a second aspect the invention relates to a nucleic acid encoding the CAR of the invention.

The present disclosure provides a nucleic acid that comprises a nucleotide sequence encoding any of the CARs of the invention.

The term "nucleic acid" or "polynucleotide" refers to deoxyribonucleic acids (DNA) or ribonucleic acids (RNA) and their polymers in either single or double stranded form. Unless specifically limited, the term encompasses nucleic acids containing known analogs of natural nucleotides that have binding capabilities similar to those of a reference nucleic acid and which are metabolized similarly to naturally occurring nucleotides. Unless otherwise indicated, a specific nucleic acid sequence also implies conservatively modified variants (e.g., substitutions with degenerate codons), alleles, orthologs, SNPs and complementary sequences, as well as sequences indicated in direct form. In particular, substitutions with degenerate codons can be obtained by creating sequences in which the third position of one or more selected (or all) codons is replaced by residues with mixed bases and/or deoxyinosine residues.

In some cases, a subject nucleic acid provides for production of a CAR of the present disclosure, e.g., in a mammalian cell. In other cases, a subject nucleic acid provides for amplification of the CAR-encoding nucleic acid.

A nucleotide sequence encoding any of the CARs of the present invention can be operably linked to a transcriptional control element, e.g., a promoter, and enhancer, etc.

Suitable promoter and enhancer elements are known in the art. For expression in a bacterial cell, suitable promoters include, but are not limited to, lacI, lacZ, T3, T7, gpt, lambda P and trc. For expression in a eukaryotic cell, suitable promoters include, but are not limited to, light and/or heavy chain immunoglobulin gene promoter and enhancer elements; cytomegalovirus immediate early promoter; herpes simplex virus thymidine kinase promoter; early and late SV40 promoters; promoter present in long terminal repeats from a retrovirus; mouse metallothionein-I promoter; and various art-known tissue specific promoters.

Suitable reversible promoters, including reversible inducible promoters are known in the art. Such reversible promoters may be isolated and derived from many organisms, e.g., eukaryotes and prokaryotes. Modification of reversible promoters derived from a first organism for use in a second organism, e.g., a first prokaryote and a second a eukaryote, a first eukaryote and a second a prokaryote, etc., is well known in the art. Such reversible promoters, and systems based on such reversible promoters but also comprising additional control proteins, include, but are not limited to, alcohol regulated promoters (e.g., alcohol dehydrogenase I (alcA) gene promoter, promoters responsive to alcohol transactivator proteins (AlcR), etc.), tetracycline regulated promoters, (e.g., promoter systems including TetActivators, TetON, TetOFF, etc.), steroid regulated promoters (e.g., rat glucocorticoid receptor promoter systems, human estrogen receptor promoter systems, retinoid promoter systems, thyroid promoter systems, ecdysone promoter systems, mifepristone promoter systems, etc.), metal regulated promoters (e.g., metallothionein promoter systems, etc.), pathogenesis-related regulated promoters (e.g., salicylic acid regulated promoters, ethylene regulated promoters, benzothiadiazole regulated promoters, etc.), temperature regulated promoters (e.g., heat shock inducible promoters (e.g., HSP-70, HSP-90, soybean heat shock promoter, etc.), light regulated promoters, synthetic inducible promoters, and the like.

In some instances, the locus or construct or transgene containing the suitable promoter is irreversibly switched through the induction of an inducible system. Suitable systems for induction of an irreversible switch are well known in the art, e.g., induction of an irreversible switch may make use of a Cre-lox-mediated recombination. Any suitable combination of recombinase, endonuclease, ligase, recombination sites, etc. known to the art may be used in generating an irreversibly switchable promoter. Methods, mechanisms, and requirements for performing site-specific recombination, described elsewhere herein, find use in generating irreversibly switched promoters and are well known in the art.

In some cases, the promoter is a CD8 cell-specific promoter, a CD4 cell-specific promoter, a neutrophil-specific promoter, or an NK-specific promoter. For example, a CD4 gene promoter can be used. As another example, a CD8 gene promoter can be used. NK cell-specific expression can be achieved by use of an Ncr1 (p46) promoter; see, e.g., Eckelhart et al. (2011) Blood 117:1565.

In some embodiments, e.g., for expression in a yeast cell, a suitable promoter is a constitutive promoter such as an ADH1 promoter, a PGK1 promoter, an ENO promoter, a PYK1 promoter and the like; or a regulatable promoter such as a GAL1 promoter, a GAL10 promoter, an ADH2 promoter, a PHO5 promoter, a CUP1 promoter, a GAL7 promoter, a MET25 promoter, a MET3 promoter, a CYC1 promoter, a HIS3 promoter, an ADH1 promoter, a PGK promoter, a GAPDH promoter, an ADC1 promoter, a TRP1 promoter, a URA3 promoter, a LEU2 promoter, an ENO promoter, a TP1 promoter, and AOX1 (e.g., for use in Pichia). Selection of the appropriate vector and promoter is well within the level of ordinary skill in the art.

Suitable promoters for use in prokaryotic host cells include, but are not limited to, a bacteriophage T7 RNA polymerase promoter; a trp promoter; a lac operon promoter; a hybrid promoter, e.g., a lac/tac hybrid promoter, a tac/trc hybrid promoter, a trp/lac promoter, a T7/lac promoter; a trc promoter; a tac promoter, and the like; an araBAD promoter; in vivo regulated promoters, such as an ssaG promoter or a related promoter, a pagC promoter, a nirB promoter, and the like; a sigma70 promoter, e.g., a consensus sigma70 promoter; a stationary phase promoter, e.g., a dps promoter, an spv promoter, and the like; a promoter derived from the pathogenicity island SPI-2; an actA promoter; an rpsM promoter; a tet promoter; an SP6 promoter; and the like. Suitable strong promoters for use in prokaryotes such as Escherichia coli include, but are not limited to Trc, Tac, T5, T7, and P Lambda. Non-limiting examples of operators for use in bacterial host cells include a lactose promoter operator (LacI repressor protein changes conformation when contacted with lactose, thereby preventing the LacI repressor protein from binding to the operator), a tryptophan promoter operator (when complexed with tryptophan, TrpR repressor protein has a conformation that binds the operator; in the absence of tryptophan, the TrpR repressor protein has a conformation that does not bind to the operator), and a tac promoter operator.

In a particular embodiment, the nucleic acid encoding the CAR of the invention further comprises a sequence encoding leader sequence which, after expression of the nucleic acid, results in signal sequence which is located N-terminally with respect to the CAR.

The term "leader peptide" as referred to herein is used according to its ordinary meaning in the art and refers to a peptide having a length of about 5-30 amino acids. A leader peptide is present at the N-terminus of newly synthesized proteins that form part of the secretory pathway. Proteins of the secretory pathway include, but are not limited to proteins that reside either inside certain organelles (the endoplasmic reticulum, Golgi or endosomes), are secreted from the cell, or are inserted into a cellular membrane. In some embodiments, the leader peptide forms part of the transmembrane domain of a protein.

In some embodiments, the isolated nucleic acid encodes a protein from the N-terminus to the C-terminus: a leader peptide is present at the N-terminus of newly synthesized proteins that form part of the secretory pathway. Proteins of the secretory pathway include, but are not limited to proteins that reside either inside certain organelles (the endoplasmic reticulum, Golgi or endosomes), are secreted from the cell, or are inserted into a cellular membrane. In some embodiments, the leader peptide forms part of the transmembrane domain of a protein.

In some embodiments, the isolated nucleic acid encodes a protein from the N-terminus to the C-terminus: a leader peptide, an anti-p95HER2 light chain variable domain, a linker domain, an anti- p95HER2 heavy chain variable domain, a CD8 hinge domain, a CD28 transmembrane domain, a CD28 intracellular co-stimulatory signaling domain followed by a CD3 zeta intracellular signaling domain.

In another embodiment, the leader sequence is the CD8 leader sequence. In a particular embodiment, the leader peptide comprises the sequence SEQ ID NO: 147 (MALPVTALLLPLALLLHAARP).

In a third aspect the invention relates to an expression vector comprising the nucleic acid of the invention.

As used herein, "vector," "cloning vector," and "expression vector" are vehicles by which the host is transformed and expression of introduced sequences (eg, transcription and translation) Mean a vehicle in which a polynucleotide sequence (eg, a foreign gene) can be introduced into a host cell to facilitate Vectors include plasmids, phages, viruses and the like.

A nucleotide sequence encoding any of the CARs of the invention can be present in an expression vector and/or a cloning vector. An expression vector can include a selectable marker, an origin of replication, and other features that provide for replication and/or maintenance of the vector. Suitable expression vectors include, e.g., plasmids, viral vectors, and the like.

Large numbers of suitable vectors and promoters are known to those of skill in the art; many are commercially available for generating a subject recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

Expression vectors generally have convenient restriction sites located near the promoter sequence to provide for the insertion of nucleic acid sequences encoding heterologous proteins. A selectable marker operative in the expression host may be present. Suitable expression vectors include, but are not limited to, viral vectors (e.g. viral vectors based on vaccinia virus; poliovirus; adenovirus; adeno-associated virus; SV40; herpes simplex virus; human immunodeficiency virus; a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus); and the like.

As noted above, in some embodiments, a nucleic acid comprising any of the CARs of the invention will in some embodiments be RNA, e.g., in vitro synthesized RNA. Methods for in vitro synthesis of RNA are known in the art; any known method can be used to synthesize RNA comprising a nucleotide sequence encoding the first and/or the second polypeptide of a heterodimeric, conditionally active CAR of the present disclosure. Methods for introducing RNA into a host cell are known in the art. Introducing RNA comprising a nucleotide sequence encoding the first and/or the second polypeptide of a heterodimeric, conditionally active CAR of the present disclosure into a host cell can be carried out in vitro or ex vivo or in vivo. For example, a host cell (e.g., an NK cell, a cytotoxic T lymphocyte, etc.) can be electroporated in vitro or ex vivo with RNA comprising a nucleotide sequence encoding the first and/or the second polypeptide of a heterodimeric, conditionally active CAR of the present disclosure.

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors; in other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic -resistance genes, such as neo and the like. Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

In a fourth aspect, the invention relates to a host cell comprising the nucleic acid of the invention or the expression vector of the invention.

The terms "host cell" or "engineered cells" means any cell of any organism that is modified, transformed, or manipulated by addition or modification of a gene, a DNA or RNA sequence, or protein or polypeptide. It also refers to the progeny of such cells. Host cells or genetically engineered cells of the present invention include isolated immune cells, such as T, NK, or NKT cells that contain the DNA or RNA sequences encoding a chimeric antigen receptor or chimeric antigen receptor complex and express the chimeric receptor on the cell surface. Isolated host cells and engineered cells may be used, for example, for enhancing an NK or NKT cell activity or a T lymphocyte activity, treatment of cancer, and treatment of infectious diseases.

In an embodiment, the cell comprising any of the CAR polypeptides described herein; or a nucleic acid encoding any of the CAR polypeptides described herein is a mammalian cell.

Suitable mammalian cells include primary cells and immortalized cell lines. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, Hut-78, Jurkat, HL-60, NK cell lines (e.g., NKL, NK92, and YTS), and the like.

In one embodiment, the mammalian cell comprises any of the CAR polypeptides described herein. The mammalian cell or tissue can be of human, primate, hamster, rabbit, rodent, cow, pig, sheep, horse, goat, dog or cat origin, but any other mammalian cell may be used. In a preferred embodiment of any aspect, the mammalian cell is human.

In some instances, the cell is not an immortalized cell line, but is instead a cell (e.g., a primary cell) obtained from an individual. For example, in some cases, the cell is an immune cell obtained from an individual.

The engineered cells may be obtained from peripheral blood, cord blood, bone marrow, tumor infiltrating lymphocytes, lymph node tissue, or thymus tissue. The host cells may include placental cells, embryonic stem cells, induced pluripotent stem cells, or hematopoietic stem cells. The cells may be obtained from humans, monkeys, chimpanzees, dogs, cats, mice, rats, and transgenic species thereof. The cells may be obtained from established cell lines.

The above cells may be obtained by any known means. The cells may be autologous, syngeneic, allogeneic, or xenogeneic to the recipient of the engineered cells. The term "autologous" refer to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenic ally.

The term "xenogeneic" refers to a graft derived from an animal of a different species.

The term "syngeneic" refers to an extremely close genetic similarity or identity especially with respect to antigens or immunological reactions. Syngeneic systems include for example, models in which organs and cells (e.g. cancer cells and their non-cancerous counterparts) come from the same individual, and/or models in which the organs and cells come from different individual animals that are of the same inbred strain.

In one embodiment, the host cell is an immune cell.

As used herein, "immune cell" refers to a cell that plays a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes. In some embodiments, the cell is a T cell; a NK cell; a NKT cell; lymphocytes, such as B cells and T cells; and myeloid cells, such as monocytes, macrophages, eosinophils, mast cells, basophils, and granulocytes.

The immune cell can be obtained from a subject having or diagnosed as having cancer, a plasma cell disorder, or an autoimmune disease or disorder. For example, the immune cell can be obtained from a subject having a cancer, e.g., multiple myeloma, smoldering myeloma, or Waldenstrom's macroglobulenemia. In some embodiments, the immune cell is obtained from a subject resistant to anti-BCMA therapy. Immune cells can also be obtained from allogeneic donors, which are non-genetically identical individuals of the same species as the intended recipients of the cells.

Immune cells (e.g., human immune cells) that can be used in the invention include autologous cells, obtained from the subject to whom the cells are later to be administered, after ex vivo modification and expansion. For example, the immune cells can be obtained from an individual having or diagnosed as having cancer, a plasma cell disorder, or autoimmune disease or disorder. Immune cells can also be obtained from allogeneic donors, which are non-genetically identical individuals of the same species as the intended recipients of the cells. Immune cells useful for the invention include T cells and NK cells.

In another embodiment, the host cell is a T cell, a natural killer (NK) cell or a NKT cell.

The terms "T cell" and "T lymphocyte" are interchangeable and are used interchangeably herein. Examples include, but are not limited to, naive T cells, central memory T cells, effector memory T cells, or a combination thereof.

Natural killer cells or "NK cells" are well known in the art. In one embodiment, natural killer cells include cell lines, such as NK- 92 cells. Further examples of NK cell lines include NKG, YT, NK-YS, HANK-1, YTS cells, and NKL cells. NK cells can be detected by specific surface markers, such as CD16, CD56, and CD8 in humans. NK cells do not express T-cell antigen receptors, the pan T marker CD3, or surface immunoglobulin B cell receptors.

NK cells mediate anti-tumor effects without the risk of GvHD (graft-versus-host disease) and are short-lived relative to T-cells. Accordingly, NK cells would be exhausted shortly after destroying cancer cells, decreasing the need for an inducible suicide gene on CAR constructs that would ablate the modified cells.

Natural killer T (NKT) cells are a heterogeneous group of T cells that share properties of both T cells and natural killer cells. Thus, NKT cells are a subset of T cells that coexpress an αβ T-cell receptor, but also express a variety of molecular markers that are typically associated with NK cells, such as NK1. Many of these cells recognize the non-polymorphic CD1d molecule, an antigen-presenting molecule that binds self and foreign lipids and glycolipids. They constitute only approximately 0.1% of all peripheral blood T cells. Natural killer T cells should not be confused with natural killer cells.

In certain embodiments, T, NK and NKT cells are derived from human peripheral blood mononuclear cells (PBMC), leukapheresis products (PBSC), human embryonic stem cells (hESCs), induced pluripotent stem cells (iPSCs), bone marrow, or umbilical cord.

In an embodiment, immune cells (e.g., human immune cells) that can be used in the invention include autologous cells, obtained from the subject to whom the cells are later to be administered, after ex vivo modification and expansion. For example, the immune cells can be obtained from an individual having or diagnosed as having cancer. Immune cells can also be obtained from allogeneic donors, which are non-genetically identical individuals of the same species as the intended recipients of the cells. Immune cells useful for the invention include T, NK and NKT cells.

Methods for obtaining T, NK and NKT cells are known in the art and can be useful for the engineered immune cells described herein. T, NK and NKT cells are typically obtained from peripheral blood that is collected from a subject by, e.g., venipuncture or withdrawal through an implanted port or catheter. Optionally, the blood can be obtained by a process including leukapheresis, in which white cells are obtained from the blood of a subject, while other blood components are returned to the subject. Blood or leukapheresis product (fresh or cryopreserved) is processed to enrich for T, NK or NKT cells using methods known in the art. For example, density gradient centrifugation (using, e.g., Ficoll) and/or counter-flow centrifugal elutriation can be carried out to enrich for mononuclear cells (including T, NK or NKT cells). In one example, for T cells, a T cell stimulation step employing, e.g., CD3/CD28 antibodies coated on magnetic beads or artificial antigen presenting cells (aAPCs) expressing, e.g., cell surface-bound anti-CD3 and anti-CD28 antibody fragments (see below), can further be carried out in order to stimulate T cells and to deplete other cells, e.g., B cells. The T cells of enriched T cell preparations can then be subject to genetic modification.

As an alternative to peripheral blood, tissues including bone marrow, lymph nodes, spleen, and tumors can be used as a source for T cells and NK cells. The T cells and NK cells can be of human, primate, hamster, rabbit, rodent, cow, pig, sheep, horse, goat, dog, or cat origin, but any other mammalian cell may be used. In a certain embodiments of any aspect, the T or NK cell is human.

Immune cells such as T, NK or NKT cells can be obtained from a number of sources peripheral blood mononuclear cells, bone marrow, lymph node tissue, cord blood, thymus tissue, tissue from a site of infection, ascites, pleural effusion, spleen tissue, and tumors. Any number of cell lines (e.g. immune cell lines such as T cell lines) available in the art, may also be used.

In an embodiment, immune cells (e.g. T, NK or NKT cells) are obtained from a unit of blood collected from a subject using any suitable techniques known in the art such as Ficoll™ separation. In another embodiment, cells from the circulating blood of a subject are obtained by apheresis. The apheresis product typically contains lymphocytes, including T, NK or NKT cells, monocytes, granulocytes, B cells, other nucleated white blood cells, red blood cells, and platelets. It will be appreciated that the cells collected by apheresis may be washed to remove the plasma fraction and to place the cells in an appropriate buffer or media for subsequent processing steps. For example, the cells may be washed with phosphate buffered saline (PBS). Alternatively, the wash solution lacks calcium and may lack magnesium or may lack many if not all divalent cations. Initial activation steps in the absence of calcium can lead to magnified activation. A washing step may be accomplished by methods known to those in the art, such as by using a semi- automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

In an embodiment, T cells are isolated from peripheral blood lymphocytes by lysing the red blood cells and depleting the monocytes, for example, by centrifugation through a PERCOLLTM gradient or by counter-flow centrifugal elutriation. Specific subpopulations of T cells, such as CD3+, CD28+, CD4+, CD8+, CD45RA+, and CD45RO+T cells, may be further isolated by positive or negative selection techniques known in the art. For example, T cells may be isolated by incubation with anti-CD3/anti-CD28 (e.g., 3x28)-conjugated beads, such as DYNABEADS® M-450 CD3/CD28 T, for a time period sufficient for positive selection of the desired T cells. Additionally or alternatively, a population of T cells may be enriched by negative selection, for instance by a combination of antibodies directed to surface markers unique to the negatively selected cells. Cell sorting and/or selection via negative magnetic immunoadherence or flow cytometry may be used.

It will be understood that cells derived from subjects that are to be modified to express the CAR of the invention may be stored for a period of time prior to their use (see, for example, therapeutic methods below). For example, the cells may be frozen, optionally after they have been washed, or they may be incubated under suitable conditions for them to remain viable until needed (e.g. on a rotator at 2-10°C or at room temperature). In this way, the cells can be stored until such time as they might be needed. They may be stored in an unmodified state (i.e. wherein they do not express the CAR of the invention) or in a modified state (i.e. wherein they have been modified to express the CAR of the invention). Prior to use in the therapeutic applications described further below, the cells may be activated and expanded generally using methods known in the art. For example, T cells may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (eg bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. Examples of an anti-CD28 antibody include 9.3, B-T3, XR-CD28 (Diaclone, Besancon, France) can be used as can other methods commonly known in the art.

T cells that have been exposed to varied stimulation times may exhibit different characteristics. For example, typical blood or apherised peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

In a particular embodiment, the T cell is a CD8+ T cell.

Particularly, the host cells of the invention may be expanded prior to transduction with a polynucleotide or vector of the invention.

In a further aspect of the present invention, T cells are obtained from a patient immediately after treatment that leaves a subject with functional T cells. In this regard, it was noted that after some cancer treatments, in particular, treatments with drugs that damage the immune system, shortly after treatment during the period of time when patients should normally recover from treatment, the quality of the obtained T cells may be optimal or improved in relation to their ability to reproduce ex vivo. Also, after ex vivo manipulation using the methods described herein, these cells may be in a preferred condition for enhanced engraftment and in vivo propagation. Thus, in connection with the present invention provides for the production of blood cells, including T cells, dendritic cells or other cells of the hematopoietic line, during this phase of recovery. In addition, in some aspects, mobilization modes (e.g., mobilization using GM-CSF) and the establishment of a specific condition can be used to create a condition in a subject in which repopulation, recirculation, regeneration and / or reproduction of specific cell types is advantageous, especially in time of a certain time window after therapy. Illustrative cell types include T cells, B cells, dendritic cells, and other cells of the immune system.

The engineered cells of the present disclosure may also include a suicide system. Suicide systems provide a mechanism whereby the engineered cell, as described above, may be deactivated or destroyed. Such a feature allows precise therapeutic control of any treatments wherein the engineered cells are used. As used herein, a suicide system provides a mechanism by which the cell having the suicide system can be deactivated or destroyed. Suicide systems are well known in the art.

In one embodiment, a suicide system includes a gene that can be pharmacologically activated to eliminate the containing cells as required. In specific aspects, the suicide gene is not immunogenic to the host harboring the polynucleotide or cell. In one example, the suicide system includes a gene that causes CD20 to be expressed on the cell surface of the engineered cell. Accordingly, administration of rituximab may be used to destroy the engineered cell containing the gene.

In some embodiments, the suicide system includes an epitope tag. Examples of epitope tags include a c-myc tag, CD52 streptavidin-binding peptide (SBP), and truncated EGFR gene (EGFRt). In this embodiment, the epitope tag is expressed in the engineered cell. Accordingly, administration of an antibody against the epitope tag may be used to destroy the engineered cell containing the gene.

In another embodiment, the suicide system includes a gene that causes truncated epidermal growth factor receptor to be expressed on the surface of the engineered cell. Accordingly, administration of cetuximab may be used to destroy the engineered cell containing the gene.

In another embodiment, the suicide system includes CD52 to be expressed on the surface of the engineered cell. Accordingly, administration of anti-52 monoclonal antibody (CAMPATH, alemtuzumab) may be used to destroy the engineered cell containing the gene.

In another embodiment, the suicide system includes CAMPATH (alemtuzumab). Accordingly, administration of anti-52 monoclonal antibody (CAMPATH) may be used to destroy the engineered cell without expressing a tag or a gene as CAR T cells or T cells highly express CD52.

In another embodiment, the suicide gene may include caspase 8 gene, caspase 9 gene, thymidine kinase, cytosine deaminase (CD), or cytochrome P450.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyi phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyi phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol.

"Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self - rearrangement before the formation of closed structures and entrap water and dissolved solutes between the lipid bilayers. However, compositions that have different structures in solution than the normal vesicular structure are also encompassed. For example, the lipids may assume a micellar structure or merely exist as nonuniform aggregates of lipid molecules. Also contemplated are lipofectamine- nucleic acid complexes.

In some embodiments of the present disclosure, any of the engineered cells disclosed herein may be introduced by two vectors, each vector bearing a different CAR.

Regardless of the method used to introduce exogenous polynucleotides into a host cell or otherwise expose a cell to the polynucleotide of the present disclosure, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure.

### ScFv, antigen-binding domain and antibody of the invention

In a fifth aspect, the invention relates to an ScFv, characterized in that:
- the CDR1, CDR2 and CDR3 of the VH region comprise, respectively, the sequences of SEQ ID NO: 1, 2 and 3 or functionally equivalent variants thereof or the sequences of SEQ ID NO: 1, 174 and 3 or functionally equivalent variants thereof, and
- the CDR1, CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof.

In a particular embodiment, the FR1, FR2, FR3 and FR4 of the VH region of the ScFv of the invention comprise respectively the sequences of SEQ ID NO: 152, 153, 154 and 155 or functionally equivalent variants thereof and the FR1, FR2, FR3 and FR4 of the VL region of the ScFv or antigen binding domain of the invention comprise respectively the sequences of SEQ ID NO: 156, 157, 158 and 159 or functionally equivalent variants thereof.

In another embodiment, the FR1, FR2, FR3 and FR4 of the VH region of the ScFv of the invention comprise respectively the sequences of SEQ ID NO: 152, 153, 154 and 155, SEQ ID NO: 19, 20, 21 and 22 or SEQ ID NO: 163, 164, 165 and 166 or functionally equivalent variants thereof and FR1, FR2, FR3 and FR4 of the VL region comprise respectively the sequences of SEQ ID NO: 156, 157, 158 and 159, SEQ ID NO: 23, 24, 25 and 26 or SEQ ID NO: 167, 168, 169 or 170 or functionally equivalent variants thereof.

In another embodiment, the VL of the ScFv of the invention comprises the sequence SEQ ID NO: 160 or 193 or a functionally equivalent variant thereof and the VH of the ScFv of the invention comprises the sequence SEQ ID NO: 161 or 194 or a functionally equivalent variant thereof.

In another embodiment, the VL of the ScFv of the invention comprises the sequence of SEQ ID NO: 160, 193, 27, 171 or 180 or functionally equivalent variants thereof and the VH comprises the sequence of SEQ ID NO: 161,194, 28, 172 or 181 or functionally equivalent variants thereof.

In a particular embodiment, the VH and VL regions of the ScFv of the invention are connected by a linker region comprising SEQ ID NO: 29.

In a particular embodiment, the linker is located between the VH and the VL regions of the antigen-binding domain. In an embodiment, the ScFv may have the structure VH-linker-VL or VL-linker-VH. In a particular embodiment, the linker is located C-terminally with respect to the VL region and N-terminally with respect to the VH region, that is, VL-linker-VH.

In a further embodiment, the ScFv of the invention comprises the sequence SEQ ID NO: 162 or 195 or a functionally equivalent variant thereof.

In another embodiment, the ScFv of the invention comprises the sequence of SEQ ID NO: 162,195, 30, 173 or 182 or functionally equivalent variants thereof.

The definitions given within the context of the CARs of the invention apply equally to the ScFv of the invention. In a similar way, the possible functionally equivalent variants of the CDRs forming part of the ScFv of the invention which have been provided herewith have been defined previously and are equally applicable to the present case.

In a sixth aspect, the invention relates to an antigen-binding domain characterized in that:
- it has at least one VH region and at least one VL region,
- the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
- the CDR1, CDR2 and CDR3 of the at least one VL region comprise respectively, the sequences of SEQ ID NO: 10, 11, and 12 or functionally equivalent variants thereof.

In a particular embodiment, the FR1, FR2, FR3 and FR4 of the at least one VH region of the antigen-binding domain comprise respectively the sequences of SEQ ID NO: 31, 32, 33 and 34 or functionally equivalent variants thereof and FR1, FR2, FR3 and FR4 of the at least one VL region of the antigen-binding domain 1 comprise respectively the sequences of SEQ ID NO: 35, 36, 37 and 38 or functionally equivalent variants thereof.

In another embodiment, the FR1, FR2, FR3 and FR4 of the at least one VH region of the antigen-binding domain of the invention comprise respectively the sequences of SEQ ID NO: 31, 32, 33 and 34, SEQ ID NO: 65, 66, 67 and 68, SEQ ID NO: 73, 74, 75 and 76 or SEQ ID NO: 81, 82, 83 and 84 or functionally equivalent variants thereof and FR1, FR2, FR3 and FR4 of the at least one VL region of the antigen-binding domain of the invention comprise respectively the sequences of SEQ ID NO: 35, 36, 37 and 38, SEQ ID NO: 69, 70, 71, 72, SEQ ID NO: 77, 78, 79 and 80 or SEQ ID NO: 85, 86, 87 and 88 or functionally equivalent variants thereof.

In another embodiment, the at least one VL of the antigen-binding domain of the invention comprises the sequence SEQ ID NO: 39 or a functionally equivalent variant thereof and the at least one VH of the ScFv of the invention comprises the sequence SEQ ID NO: 40 or 183 or a functionally equivalent variant thereof.

In another embodiment, the at least one VL of the antigen-binding domain of the invention comprises the sequence of SEQ ID NO: 39, 54, 56 and 58 or functionally equivalent variants thereof and the at least one VH region comprises the sequence of SEQ ID NO: 40, 183, 53, 55 and 57 or functionally equivalent variants thereof.

In a particular embodiment, when the antigen binding domain is an ScFv, then, the VH and VL regions are connected by a linker region comprising SEQ ID NO: 29.

In a particular embodiment, when the antigen-binding domain is an ScFv, the linker is located between the VH and the VL regions. In an embodiment, when the antigen-binding domain 1 is an ScFv, the ScFv may have the structure VH-linker-VL or VL-linker-VH. In a particular embodiment, when the antigen-binding domain is an ScFv, the linker is located C-terminally with respect to the VL region and N-terminally with respect to the VH region, that is, VL-linker-VH.

In a further embodiment, the antigen-binding domain comprises the sequence SEQ ID NO: 41 or 185 or a functionally equivalent variant thereof.

In another embodiment, the antigen-binding domain of the invention comprises the sequence of SEQ ID NO: 41,185, 187, 188 or 189 or functionally equivalent variants thereof.

The definitions given within the context of the CARs of the invention apply equally to the antigen-binding domain of the invention. In a similar way, the possible functionally equivalent variants of the CDRs forming part of the antigen-binding domain of the invention which have been provided herewith have been defined previously and are equally applicable to the present case.

In a seventh aspect, the invention relates to an antibody or antibody fragment thereof characterized in that:
- it has at least one VH region and at least one VL region,
- the CDR1, CDR2 and CDR3 of the at least one VH region comprise, respectively, the sequences of SEQ ID NO: 13, 14 and 15 or functionally equivalent variants thereof, and
- the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.

In a particular embodiment, the FR1, FR2, FR3 and FR4 of the at least one VH region of the antibody or antibody fragment thereof comprise respectively the sequences of SEQ ID NO: 42, 43, 44 and 45 or functionally equivalent variants thereof and FR1, FR2, FR3 and FR4 of the at least one VL region of the antibody or antibody fragment thereof comprise respectively the sequences of SEQ ID NO: 46, 47, 48 and 49 or functionally equivalent variants thereof.

In another embodiment, the FR1, FR2, FR3 and FR4 of the at least one VH region of the antibody or antibody fragment of the invention comprise respectively the sequences of SEQ ID NO: 42, 43, 44 and 45, SEQ ID NO: 89, 90, 91 and 92, SEQ ID NO:97, 98, 99 and 100 or SEQ ID NO: 105, 106, 107 and 108 or functionally equivalent variants thereof and FR1, FR2, FR3 and FR4 of the at least one VL region of the antibody or antibody fragment of the invention comprise respectively the sequences of SEQ ID NO: 46, 47, 48 and 49, SEQ ID NO: 93, 94, 95 and 96, SEQ ID NO: 101, 102, 103 and 104 or SEQ ID NO: 109, 110, 11 and 112 or functionally equivalent variants thereof.

In another embodiment, the at least one VL of the antibody or antibody fragment thereof comprises the sequence SEQ ID NO: 50 or 184 or a functionally equivalent variant thereof and the at least one VH of the antibody or antibody fragment thereof comprises the sequence SEQ ID NO: 51 or a functionally equivalent variant thereof.

In another embodiment, the at least one VL region of the antibody of antibody fragment thereof comprises the sequence of SEQ ID NO: 50 or ,184, 60, 62 or 64 or functionally equivalent variants thereof and the at least one VH region of the antibody or antibody fragment thereof comprises the sequence of SEQ ID NO: 51, 59, 61 and 63 or functionally equivalent variants thereof.

In a particular embodiment, when the antibody or antibody fragment is an ScFv, then, the at least one VH and VL regions of the antibody or antibody fragment thereof are connected by a linker region comprising SEQ ID NO: 29.

In a particular embodiment, when the antibody or antibody fragment is an ScFv, the linker is located between the VH and the VL regions. In an embodiment, when the antibody or antibody fragment is an ScFv, the ScFv may have the structure VH-linker-VL or VL-linker-VH. In a particular embodiment, when the antibody or antibody fragment is an ScFv, the linker is located C-terminally with respect to the VL region and N-terminally with respect to the VH region, that is, VL-linker-VH.

In a further embodiment, the antibody or antibody fragment thereof comprises the sequence SEQ ID NO: 52 or 186 or a functionally equivalent variant thereof.

In another embodiment, the antibody of antibody fragment thereof comprises the the sequence of SEQ ID NO: 52,186, 190, 191 or 192 or functionally equivalent variants thereof.

The term "antibody", as used herein, refers to an immunoglobulin molecule or according to some embodiments of the invention, a fragment of an immunoglobulin molecule which has the ability to specifically bind to an epitope of a molecule ("antigen"). Naturally occurring antibodies typically comprise a tetramer which is usually composed of at least two heavy (H) chains and at least two light (L) chains. Each heavy chain is comprised of a heavy chain variable domain (abbreviated herein as VH) and a heavy chain constant domain, usually comprised of three domains (CH1, CH2 and CH3). Heavy chains can be of any isotype, including IgG (lgG1, IgG2, IgG3 and IgG4 subtypes). Each light chain is comprised of a light chain variable domain (abbreviated herein as VL) and a light chain constant domain (CL). Light chains include kappa chains and lambda chains. The heavy and light chain variable domain is typically responsible for antigen recognition, while the heavy and light chain constant domain may mediate the binding of the immunoglobulin to host tissues or factors, including various cells of the immune system (e.g., effector cells) and the first component (C1 q) of the classical complement system. The VH and VL domains can be further subdivided into domains of hypervariability, termed "complementarity determining regions," that are interspersed with domains of more conserved sequence, termed "framework regions" (FR). Each VH and VL is composed of three CDR Domains and four FR Domains arranged from amino-terminus to carboxy-terminus in the following order: FR1 -CDR1 -FR2- CDR2-FR3-CDR3-FR4. The variable domains of the heavy and light chains contain a binding domain that interacts with an antigen. Of particular relevance are antibodies and their epitope- binding fragments that have been "isolated" so as to exist in a physical milieu distinct from that in which it may occur in nature or that have been modified so as to differ from a naturally occurring antibody in amino acid sequence.

The term "antibody" comprises whole monoclonal antibodies or polyclonal antibodies, or fragments thereof, that retain one or more CDR regions, and includes human antibodies, humanized antibodies, chimeric antibodies and antibodies of a non-human origin.

"Monoclonal antibodies" are homogenous, highly specific antibody populations directed against a single site or antigenic "determinant". "Polyclonal antibodies" include heterogeneous antibody populations directed against different antigenic determinants.

In a particular embodiment, the antibody of the invention is an antibody of non-human origin, preferably of murine origin. In preferred embodiment, the antibody of the invention is a monoclonal antibody.

It is well known that the basic structural unit of an antibody comprises a tetramer. Each tetramer is constituted by two identical pairs of polypeptide chains, each of which is composed by a light chain (25 KDa) and by a heavy chain (50-75 KDa). The amino-terminal region of each chain includes a variable region of about 100-110 or more amino acids, which is involved in antigen recognition. The carboxy-terminal region of each chain comprises the constant region that mediates the effector function. The variable regions of each pair of light and heavy chains form the binding site of the antibody. Therefore, an intact antibody has two binding sites. Light chains are classified as K or λ. Heavy chains are classified as γ, µ, α, δ and ε, and they define the isotype of the antibody as respectively IgG, IgM, IgA, IgD or IgE.

The variable regions of each pair of light and heavy chains form the binding site of the antibody. They are characterized by the same general structure constituted by relatively preserved regions called frameworks (FR) joined by three hyper-variable regions called complementarity determining regions (CDR), as defined within the context of the extracellular domain or antigen-binding domain of the CAR of the invention.

Functionally equivalent variants of the CDRs and FRs sequences that defined the specificity of the antibody or antigen-binding domain of the invention are herewith contemplated. Thus, definitions of functionally equivalent variants of the sequences defining the CDRs and FRs of the antibodies of the invention, as well as the percentage identity with regard to said sequences that are within the scope of the present invention have already been defined within the context of the antigen-binding domain of the CAR of the invention and apply equally to the antibodies of the invention.

The skilled in the art will understand that the antibody or antibody fragment of the invention shares all the characteristics of the antigen-binding domain 2 of the CAR of the invention as it relates to the its capacity to bind to the specific antigen, that is, the p95HER2 peptide. Therefore, all the particulars of the antigen-binding domain 2 of the CAR of the invention related to the binding to the p95HER2 peptide, apply to the antibody or antibody fragment described here (as it refers to its variable region).

As used herein, the antibody of the invention encompasses not only full length antibodies (e.g., IgG), but also antigen-binding fragments thereof, for example, Fab, Fab', F(ab')2, Fv fragments, human antibodies, humanized antibodies, chimeric antibodies, antibodies of a non-human origin, recombinant antibodies, and polypeptides derived from immunoglobulins produced by means of genetic engineering techniques, for example, single chain Fv (scFv), diabodies, heavy chain or fragments thereof, light chain or fragment thereof, VH or dimers thereof, VL or dimers thereof, Fv fragments stabilized by means of disulfide bridges (dsFv), molecules with single chain variable region domains (Abs), minibodies, scFv-Fc, VL and VH domains and fusion proteins comprising an antibody, or any other modified configuration of the immunoglobulin molecule that comprises an antigen recognition site of a desired specificity. The antibody of the invention may also be a bispecific antibody. An antibody fragment may refer to an antigen binding fragment.

In particular embodiment the antibody is selected from the group consisting of a monoclonal antibody, a F(ab), a F(ab'), a Fv, a ScFv and a minibody.

As used herein a "recombinant antibody" is an antibody that comprises an amino acid sequence derived from two different species or, or two different sources, and includes synthetic molecules, for example, an antibody that comprises a non-human CDR and a human framework or constant region. In certain embodiments, recombinant antibodies of the present invention are produced from a recombinant DNA molecule or synthesized.

The person skilled in the art will understand that the amino acid sequences of the antibodies of the invention can include one or more amino acid substitutions such that, even though the primary sequence of the polypeptide is altered, the capacity of the antibody to bind to the p95HER antigen is maintained. Said substitution can be a conservative substitution and is generally applied to indicate that the substitution of one amino acid with another amino acid with similar properties (for example, the substitution of glutamic acid (negatively charged amino acid) with aspartic acid would be a conservative amino acid substitution).

Amino acid sequence modification(s) of the antibody described herein are contemplated. For example, it may be desirable to improve the binding affinity and/or other biological properties of the antibody. Amino acid sequence variants of the antibody are prepared by introducing appropriate nucleotide changes into the antibody encoding nucleic acid, or by peptide synthesis. Such modifications include, for example, deletions from, and/or insertions into and/or substitutions of, residues within the amino acid sequences of the antibody. Any combination of deletion, insertion, and substitution is made to achieve the final construct, provided that the final construct possesses the desired characteristics. The amino acid changes may also alter post-translational processes of the protein, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or the antibody polypeptidic chain fused to a cytotoxic polypeptide. Other insertional variants of the molecule include the fusion to the N- or C-terminus of an enzyme, or a polypeptide which increases its serum half-life.

Another type of variant is an amino acid substitution variant. These variants have at least one amino acid residue in the molecule replaced by a different residue. The sites of greatest interest for substitution mutagenesis of antibodies include the hypervariable In a particular embodiment, the ScFv, antigen-binding domain and antibody or antibody fragment thereof are humanized.

The term humanized has already been defined within the context of the CAR of the invention and applies equally to the present case. Similarly, suitable humanized sequences of the antigen-binding domain and antibody or antibody fragment (as it refers to its variable region and therefore equivalent to the antigen-binding domain 1 and antigen-binding domain 2 respectively) of the invention, have already been defined previously within the context of the CARs of the invention and apply equally to the antigen-binding domain or antibody or antibody fragment thereof. In a similar way, the ScFv of the invention may be humanized. Therefore, in a particular embodiment, the ScFv is humanized and, more particularly, the VH and/or VL regions of the ScFv are humanized.

In a particular embodiment, the VL region of the ScFv comprises the humanized sequence selected from SEQ ID NOs: 27,171 and 180 or functionally equivalent variants thereof and the VH regions comprises the humanized sequence selected from SEQ ID NOs: 28,172 and 181 or functionally equivalent variants thereof.

In another embodiment, the ScFv comprises the humanized sequence selected from SEQ ID NOs: 30, 173 and 182.

In another embodiment, the VH and VL regions of the ScFv comprise humanized FR1, FR2, FR3 and FR4 regions, wherein the FR1, FR2, FR3 and FR4 of the VH regions comprise respectively the sequences SEQ ID NOs: 19, 20, 21 and 22 or functionally equivalent variants thereof and, the FR1, FR2, FR3 and FR4 of the VL region comprise respectively the sequences SEQ ID NOs: 23, 24, 25 and 26 or functionally equivalent variants thereof.

In another embodiment, the VH and VL regions of the ScFv comprise humanized FR1, FR2, FR3 and FR4 regions, wherein the FR1, FR2, FR3 and FR4 of the VH regions comprise respectively the sequences SEQ ID NOs: 163,164, 165 and 166 or functionally equivalent variants thereof, and the FR1, FR2, FR3 and FR4 of the VL region comprise respectively the sequences SEQ ID NOs: 167, 168, 169 and 170 or functionally equivalent variants thereof.

In another embodiment, the VH region of the ScFv comprises at least one humanized FR region, at least 2 humanized FR regions, at least 3 humanized FR regions or at least 4 humanized FR regions. In other embodiments, the humanized FR1, FR2, FR3 and FR4 regions are selected from SEQ ID NO:19 or 163 for FR1, SEQ ID NO: 20 or 164 for FR2, SEQ ID NO:21 or 165 for FR3 and SEQ ID NO:22 and 166 for FR4 or a functionally equivalent variant of any of the above.

In another embodiment, the VL region of the ScFv comprises at least one humanized FR region, at least 2 humanized FR regions, at least 3 humanized FR regions or at least 4 humanized FR regions. In other embodiments, the humanized FR1, FR2, FR3 and FR4 regions are selected from SEQ ID NO:23 or 167 for FR1, SEQ ID NO: 24 or 168 for FR2, SEQ ID NO:25 or 169 for FR3 and SEQ ID NO:26 and 170 for FR4 or a functionally equivalent variant of any of the above.

The present invention also provides a derivative of the ScFv, antigen-binding domain or antibody disclosed herein. The derivatized ScFv, antigen-binding domain or antibody may comprise any molecule or material providing targeting properties, for example, an increased half-life in certain uses to the ScFv, antigen-binding domain. The derivatized ScFv, antigen-binding domain or antibody may comprise a detectable (or labeling) residue (e.g.: molecule binding to a radioactive, colorimetric, antigenic, or enzyme molecule, detectable bead (e.g.: magnetic or electron-dense (e.g.: gold) bead), or other molecules (e.g.: biotin or streptavidin)), a therapeutic or diagnostic residue (e.g.: radioactive, cytotoxic, or pharmaceutically active residue), or a molecule increasing suitability of the ScFv, antigen-binding domain or antibody for special uses (for example, administration to a subject, for example, a human subject, or other in vivo or in vitro uses). For examples of a molecule to be used for derivatizing an ScFv, antigen-binding domain or antibody, albumin (e.g.: human serum albumin) and polyethylene glycol (PEG) are included. The albumin-linked and pegylated derivatives of the ScFv, antigen-binding domain or antibody may be prepared by using techniques widely known in the art.

In some embodiments, the ScFv, antigen-binding domain or antibody may comprise one or more of labels. "Label" means any detectable material. For examples of appropriate label groups, a radioactive isotope or radioactive nuclide (e.g.: 3H, 14C, 15N, 35S, 90Y, 99Tc, 1251, 1311), a fluorescent group (e.g.: FITC, rhodamine, lanthanoid fluorescent substance), an enzyme group (e.g.: horse radish peroxidase, b-galactosidase, luciferase, alkaline phosphatase), a chemiluminescent group, a biotinyl group, or certain polypeptide epitope recognized by a secondary reporter (for example, leucine zipper pair sequence, secondary antibody binding site, metal binding domain, epitope tag) is included, but not limited thereto. In some embodiments, the labeling group is coupled to an antibody through various length of space arms to reduce potential steric hindrance. Various methods to label a protein are known in the art, and those skilled in the art will select an appropriate label and a proper method for a specific purpose.

Commonly, labels may be classified according to detection methods: a) radioactive or isotope label; b) magnetic label (e.g.: magnetic particle); c) oxidation- reduction active residue; d) optical dye; enzyme group (for example, horse radish peroxidase, b-galactosidase, luciferase, alkaline phosphatase); e) biotinyl group; and f) certain polypeptide epitope recognized by a secondary reporter (e.g.: leucine zipper pair sequence, binding site for a secondary antibody, metal binding domain, epitope tag, etc.). In some embodiments, the labeling group is coupled to an ScFv, antigen-binding domain or antibody through various length of spacer arms to reduce potential steric hindrance. Various methods for labeling a protein are known in the art.

In one embodiment, the label comprises an optical dye comprising a chromophore, a phosphor and a fluorescent substance, but not limited thereto. The fluorescent substance may be a small-molecular fluorescent material or protein fluorescent material.

"Fluorescent label" means any molecule to be detected by fluorescent properties which a material has. For examples of the fluorescent label, fluorescein, rhodamine, tetramethylrhodamine, eosin, erythrosine, coumarin, methyl-coumarin, pyrene, malachite green, stilbene, lucifer yellow, cascade blue J, texas red, IAEDANS, EDANS, BODIPY FL, LC red 640, Cy 5, Cy 5.5, LC red 705, Oregon green, alexa-fluor dye (alexa-fluor 350, alexa-fluor 430, alexa-fluor 488, alexa-fluor 546, alexa-fluor 568, alexa-fluor 594, alexa-fluor 633, alexa-fluor 647, alexa-fluor 660, alexa-fluor 680), cascade blue, cascade yellow and R-phycoerythrin (PE), FITC,), Cy5, Cy5.5, and Cy7 etc. are included, but not limited thereto.

The protein fluorescent label substances include green fluorescent proteins including Renilla, Ptilosarcus or Aequorea species of GFP, EGFP(Clontech Labs., Inc., Genbank Accession Number U55762), blue fluorescent proteins, enhanced yellow fluorescent proteins, b galactosidase, but not limited thereto.

In an eighth aspect, the invention relates to a nucleic acid encoding the ScFv, antigen binding domain or antibody according to the fifth, sixth and seventh aspects of the invention.

In a ninth aspect, the invention relates to an expression vector comprising the nucleic acid of the eighth aspect of the invention.

In a tenth aspect, the invention relates to a host cell comprising the nucleic acid of the eighth aspect of the invention or the expression vector of the ninth aspect of the invention.

The definitions and particularities regarding the nucleic acids, expression vectors and host cells related to the ScFv of the invention are the same as the ones defined within the context of the CARs of the invention.

### Antibody-drug conjugates (ADCs)

In further aspects of the invention, the ScFv, antigen-binding domain or antibody of the invention are conjugated with at least one therapeutic agent, which it may be a radioisotope, radionuclide, toxin, toxoid, a polypeptide or chemotherapeutic agent. When an ScFv, antigen-binding domain or antibody is conjugated to a drug it is referred to as an antibody-drug conjugates (ADCs) or, when an ScFv, antigen-binding domain or antibody are conjugated to a toxin, they are called immunotoxins. Both, ADCs and immunotoxins are included within the scope of the present invention. When administered to a patient, ADCs and immunotoxins bind to the target cell via their antibody portions and are internalized allowing drugs or toxins to exert their effects.

Thus, in an eleventh aspect the invention relates to an antibody-drug conjugate (ADC) comprising:
- a therapeutic agent
- an antibody or antigen-binding domain specific for p95HER2 selected from the group consisting of:
   (i) an antibody or antibody fragment thereof, characterized in that:
      - It has at least one VH region and one VL region,
      - the CDR1, CDR2 and CDR3 of the at least one VH region comprise, respectively, the sequences of SEQ ID NO: 1, 2 and 3 or functionally equivalent variants thereof or the sequences of SEQ ID NO: 1, 174 and 3 or functionally equivalent variants thereof, and
      - the CDR1, CDR2 and CDR3 of the at least one VL region comprise respectively, the sequences of SEQ ID NO: 4, 5, and 6 or functionally equivalent variants thereof or the sequences of SEQ ID NO: 175, 5 and 6 or functionally equivalent variants thereof;
   (ii) an antibody or antibody fragment thereof, characterized in that:
      - it has at least one VH region and at least one VL region characterized in that the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
      - the CDR1, CDR2 and CDR3 of the at least one VL region comprise respectively, the sequences of SEQ ID NO: 10, 11, and 12 or functionally equivalent variants thereof, and
   (iii) an antibody or antibody fragment thereof characterized in that:
      - the CDR1, CDR2 and CDR3 of the VH region comprise, respectively, the sequences of SEQ ID NO: 13, 14 and 15 or functionally equivalent variants thereof and
      - the CDR1, CDR2 and CDR3 of the VL region comprise respectively, the sequences of SEQ ID NO: 16, 17, and 18 or functionally equivalent variants thereof or the sequences of SEQ ID NO: 179, 17 and 18 or functionally equivalent variants thereof.

The terms "therapeutic agent" or "drug" refer to refers to a pharmacologically active substance or substances that are used to treat, or prevent diseases or conditions. Drugs act by altering the physiology of a living organism, tissue, cell, or in vitro system that they are exposed to.

As mentioned above, the term "antibody-drug conjugate" or "ADC" refers to antibodies, or antigen-binding domains as the ones previously defined within the context of the CARs and the antigen-binding domains and antibodies of the invention, also including antibody derivatives, that bind to p95HER2 and are conjugated to a drug such as a cytotoxic, cytostatic, and/or therapeutic agent.

Therefore, the antibody conjugates of the invention relate to an antibody or antibody fragment thereof recombinantly fused or chemically conjugated (covalent bonds and chemically) to heterologous agents to create fusion proteins. The heterologous agent is a polypeptide (or portion thereof, preferably a polypeptide of at least 10, at least 20, at least 30, at least 40, at least 50, at least 60, at least 70, at least 80, at least 90 or at least 100 amino acids), nucleic acid, small It may be a molecule (less than 1000 daltons) or an inorganic or organic compound. The fusion need not be direct and may occur through a linker sequence.

In an embodiment, when the antibody or antigen-binding domain is the antibody or antibody fragment thereof comprising the CDR sequences SEQ ID NOs: 1, 2,and 3 or functionally equivalent variants thereof or the sequences SEQ ID NO: 1, 174 and 3 or functionally equivalent variants thereof in the at least one VH region and the CDR sequences SEQ ID NOs 4, 5 and 6 or functionally equivalent variants thereof or the sequences SEQ ID NOs: 175, 5 and 6 or functionally equivalent variants thereof in the at least one VL region as described in the eleventh aspect of the invention, then, the FR1, FR2, FR3 and FR4 of the VH region comprise respectively the sequences of SEQ ID NO: 152, 153, 154 and 155 or functionally equivalent variants thereof and the FR1, FR2, FR3 and FR4 of the VL region comprise respectively the sequences of SEQ ID NO: 156, 157, 158 and 159 or functionally equivalent variants thereof.

In another embodiment, when the antibody or antigen-binding domain is the antibody or antibody fragment thereof comprising the CDR sequences SEQ ID NOs: 1, 2,and 3 or functionally equivalent variants thereof or the sequences SEQ ID NO: 1, 174 and 3 or functionally equivalent variants thereof in the at least one VH region and the CDR sequences SEQ ID NOs 4, 5 and 6 or functionally equivalent variants thereof or the sequences SEQ ID NOs: 175, 5 and 6 or functionally equivalent variants thereof in the at least one VL region as described in the eleventh aspect of the invention, then, the FR1, FR2, FR3 and FR4 of the at least one VH region comprise respectively the sequences SEQ ID NOs: 152, 153, 154 and 155, SEQ ID NO: 19, 20, 21 and 22 or SEQ ID NO: 163, 164, 165 and 166 or functionally equivalent variants thereof and the FR1, FR2, FR3 and FR4 of the at least one VL region comprise respectively the sequences SEQ ID NOs: 156, 157, 158 and 159, SEQ ID NO: 23, 24, 25 and 26 or SEQ ID NO: 167, 168, 169 or 170 or functionally equivalent variants thereof.

In another embodiment, when the antibody or antigen-binding domain is the antibody or antibody fragment thereof comprising the CDR sequences SEQ ID NOs: 7, 8 and 9 or sequences SEQ ID NOS: 176, 177 and DY or functionally equivalent variants thereof in the at least one VH region and the CDR sequences SEQ ID NOs: 10, 11 and 12 in the at least one VL region as described in the eleventh aspect of the invention, then, the FR1, FR2, FR3 and FR4 of the VH region comprise respectively the sequences of SEQ ID NO: 31, 32, 33 and 34 or functionally equivalent variants thereof and the FR1, FR2, FR3 and FR4 of the VL region comprise respectively the sequences of SEQ ID NO: 35, 36, 37 and 38 or functionally equivalent variants thereof.

In another embodiment, when the antibody or antigen-binding domain is the antibody or antibody fragment thereof comprising the CDR sequences SEQ ID NOs: 7, 8 and 9 or sequences SEQ ID NOS: 176, 177 and DY or functionally equivalent variants thereof in the at least one VH region and the CDR sequences SEQ ID NOs: 10, 11 and 12 in the at least one VL region as described in the eleventh aspect of the invention, then, the FR1, FR2, FR3 and FR4 or the at least one VH region comprise respectively the sequences SEQ ID NOs: 31, 32, 33 and 34, or SEQ ID NO: 65, 66, 67 and 68, SEQ ID NO: 73, 74, 75 and 76 or SEQ ID NO: 81, 82, 83 and 84 or functionally equivalent variants thereof and the FR1, FR2, FR3 and FR4 of the at least one VL region comprise respectively the sequences SEQ ID NOs: 35, 36, 37 and 98 38, SEQ ID NO: 69, 70, 71, 72, SEQ ID NO: 77, 78, 79 and 80 or SEQ ID NO: 85, 86, 87 and 88 or functionally equivalent variants thereof.

In another embodiment, when the antibody or antigen-binding domain is the antibody or antibody fragment thereof comprising the CDR sequences SEQ ID NOs: 13, 14 and 15 in the at least one VH region and the CDR sequences SEQ ID NOs: 16, 17 and 18 or functionally equivalent variants thereof or sequences SEQ ID NOs: 179, 17 and 18 or functionally equivalent variants thereof in the at least one VL region as described in the eleventh aspect of the invention, then, the FR1, FR2, FR3 and FR4 of the VH region comprise respectively the sequences of SEQ ID NO: 42, 43, 44 and 45 or functionally equivalent variants thereof and the FR1, FR2, FR3 and FR4 of the VL region comprise respectively the sequences of SEQ ID NO: 46, 47, 48 and 49 or functionally equivalent variants thereof.

In another embodiment, when the antibody or antigen-binding domain is the antibody or antibody fragment thereof comprising the CDR sequences SEQ ID NOs: 13, 14 and 15 in the at least one VH region and the CDR sequences SEQ ID NOs: 16, 17 and 18 or functionally equivalent variants thereof or sequences SEQ ID NOs: 179, 17 and 18 or functionally equivalent variants thereof in the at least one VL region as described in the eleventh aspect of the invention, then, the FR1, FR2, FR3 and FR4 or the at least one VH region comprise respectively the sequences SEQ ID NOs: 42, 43, 44 and 45, SEQ ID NO: 89, 90, 91 and 92, SEQ ID NO:97, 98, 99 and 100 or SEQ ID NO: 105, 106, 107 and 108 or functionally equivalent variants thereof and the FR1, FR2, FR3 and FR4 of the at least one VL region comprise respectively the sequences SEQ ID NOs: 46, 47, 48 and 49, SEQ ID NO: 93, 94, 95 and 96, SEQ ID NO: 101, 102, 103 and 104 or SEQ ID NO: 109, 110, 11 and 112 or functionally equivalent variants thereof.

In an embodiment, when the antibody or antigen-binding domain is the antibody or antibody fragment thereof comprising the CDR sequences SEQ ID NOs: 1, 2, 3 or functionally equivalent variants thereof or sequences SEQ ID NOs: 1, 174 and 3 or functionally equivalent variants thereof in the at least one VH region and the CDR sequences SEQ ID NOs 4, 5 and 6 or functionally equivalent variants thereof or sequences SEQ ID NOs: 175, 5 and 6 or functionally equivalent variants thereof in the at least one VL region as described in the eleventh aspect of the invention, the VL comprises the sequence of SEQ ID NO: 160 or 193 or a functionally equivalent variant thereof and the VH comprises the sequence of SEQ ID NO: 161 or 194 of a functionally equivalent variant thereof.

In an embodiment, when the antibody or antigen-binding domain is the antibody or antibody fragment thereof comprising the CDR sequences SEQ ID NOs: 1, 2, 3 or functionally equivalent variants thereof or sequences SEQ ID NOs: 1, 174 and 3 or functionally equivalent variants thereof in the at least one VH region and the CDR sequences SEQ ID NOs 4, 5 and 6 or functionally equivalent variants thereof or sequences SEQ ID NOs: 175, 5 and 6 or functionally equivalent variants thereof in the at least one VL region as described in the eleventh aspect of the invention, the VL comprises the sequence SEQ ID NO: 160,193, 27,171 or 180 or a functionally equivalent variant thereof and the VH comprises the sequence SEQ ID NO: 161, 194, 28, 172 or 181 of a functionally equivalent variants thereof.

In an embodiment, when the antibody or antigen-binding domain is the antibody or antibody fragment thereof comprising the CDR sequences SEQ ID NOs: 7, 8 and 9 or SEQ ID NOs: 176, 177 and DY or functionally equivalent variants thereof in the at least one VH region and the CDR sequences SEQ ID NOs: 10, 11 and 12 in the at least one VL region as described in the eleventh aspect of the invention, the VL comprises the sequence of SEQ ID NO: 39 or a functionally equivalent variant thereof and the VH comprises the sequence of SEQ ID NO: 40 or 183 or a functionally equivalent variant thereof.

In an embodiment, when the antibody or antigen-binding domain is the antibody or antibody fragment thereof comprising the CDR sequences SEQ ID NOs: 7, 8 and 9 or SEQ ID NOs: 176, 177 and DY or functionally equivalent variants thereof in the at least one VH region and the CDR sequences SEQ ID NOs: 10, 11 and 12 in the at least one VL region as described in the eleventh aspect of the invention, the VL comprises the sequence SEQ ID NO: 39, 54, 56 and 58 or a functionally equivalent variant thereof and the VH comprises the sequence SEQ ID NO: 40,183 53, 55 and 57 or a functionally equivalent equivalent variants thereof. In an embodiment, when the antibody or antigen-binding domain is the antibody or antibody fragment thereof comprising the CDR sequences SEQ ID NOs: 13, 14 and 15 in the at least one VH region and the CDR sequences SEQ ID NOs: 16, 17 and 18 or SEQ ID NO: 179, 17 and 18 in the at least one VL region as described in the eleventh aspect of the invention, the VL comprises the sequence of SEQ ID NO: 50 or 184 or a functionally equivalent variant thereof and the VH comprises the sequence of SEQ ID NO: 51 or a functionally equivalent variant thereof.

In an embodiment, when the antibody or antigen-binding domain is the antibody or antibody fragment thereof comprising the CDR sequences SEQ ID NOs: 13, 14 and 15 in the at least one VH region and the CDR sequences SEQ ID NOs: 16, 17 and 18 or SEQ ID NO: 179, 17 and 18 in the at least one VL region as described in the eleventh aspect of the invention, the VL comprises the sequence SEQ ID NO: 50,184, 60, 62 or 64 or a functionally equivalent variant thereof and the VH comprises the sequence SEQ ID NO: 51, 59, 61 and 63 or a functionally equivalent thereof.

In another embodiment, when the antibody or antigen-binding domain is the antibody or antibody fragment thereof comprising the CDR sequences SEQ ID NOs: 1, 2, 3 or functionally equivalent variants thereof or sequences SEQ ID NOs: 1, 174 and 3 or functionally equivalent variants thereof in the at least one VH region and the CDR sequences SEQ ID NOs 4, 5 and 6 of functionally equivalent variants thereof of sequences SEQ ID NOs: 175, 5 and 6 or functionally equivalent variants thereof in the at least one VL region as described in the eleventh aspect of the invention, it comprises the sequence SEQ ID NO: 162 or a functionally equivalent variant thereof.

In another embodiment, when the antibody or antigen-binding domain is the antibody or antibody fragment thereof comprising the CDR sequences SEQ ID NOs: 1, 2, 3 or functionally equivalent variants thereof or sequences SEQ ID NOs: 1, 174 and 3 or functionally equivalent variants thereof in the at least one VH region and the CDR sequences SEQ ID NOs 4, 5 and 6 of functionally equivalent variants thereof of sequences SEQ ID NOs: 175, 5 and 6 or functionally equivalent variants thereof in the at least one VL region as described in the eleventh aspect of the invention, it comprises the sequence SEQ ID NO: 162, 195, 30, 173 or 182 or a functionally equivalent variants thereof.

In another embodiment, when the antibody or antigen-binding domain is the antibody or antibody fragment thereof comprising the CDR sequences SEQ ID NOs: 7, 8 and 9 or SEQ ID NOs: 176, 177 and DY or functionally equivalent variants thereof in the at least one VH region and the CDR sequences SEQ ID NOs: 10, 11 and 12 in the at least one VL region as described in the eleventh aspect of the invention, it comprises the sequence SEQ ID NO: 41 or 185 or a functionally equivalent variant thereof.

In another embodiment, when the antibody or antigen-binding domain is the antibody or antibody fragment thereof comprising the CDR sequences SEQ ID NOs: 7, 8 and 9 or SEQ ID NOs: 176, 177 and DY or functionally equivalent variants thereof in the at least one VH region and the CDR sequences SEQ ID NOs: 10, 11 and 12 in the at least one VL region as described in the eleventh aspect of the invention, it comprises the sequence SEQ ID NO: 41,185, 187, 188 or 189 or a functionally equivalent variants thereof.

In another particular embodiment, when the antibody or antigen-binding domain is the antibody or antibody fragment thereof comprising the CDR sequences SEQ ID NOs: 13, 14 and 15 in the at least one VH region and the CDR sequences SQ Id NOs: 16, 17 and 18 or functionally equivalent variants thereof or sequences of SEQ ID NO: 179, 17 and 18 or functionally equivalent variants thereof in the at least one VL region as described in the eleventh aspect of the invention, it comprises the sequence SEQ ID NO: 52 or 186 or a functionally equivalent variant thereof.

In another particular embodiment, when the antibody or antigen-binding domain is the antibody or antibody fragment thereof comprising the CDR sequences SEQ ID NOs: 13, 14 and 15 in the at least one VH region and the CDR sequences SQ Id NOs: 16, 17 and 18 or functionally equivalent variants thereof or sequences of SEQ ID NO: 179, 17 and 18 or functionally equivalent variants thereof in the at least one VL region as described in the eleventh aspect of the invention, it comprises the sequence SEQ ID NO: 52,186, 190, 191 or 192 or a functionally equivalent variants thereof. Functionally equivalent variants of the CDR sequences have been defined and characterized within the context of the CARs of the invention when referring to the ScFv1, antigen-binding domain 1 and antigen-binding domain 2 and the same particulars apply equally to the antibody or antibody fragments thereof of the ADCs of the invention.

The skilled in the art will understand that the antibody or antibody fragment of the item (i) of the eleventh aspect of the invention shares all the characteristics of the ScFv1 as described within the context of the CARs of the invention as it relates to its capacity to bind to the specific antigen, that is, the p95HER2. Therefore, all the particulars of the ScFv1 of the CAR of the invention related to the binding to the p95HER2 peptide, apply to the antibody or antibody fragment of the item (i) of the eleventh aspect of the invention.

In a similar way, the skilled in the art will understand that the antibody or antibody fragment of the item (ii) of the eleventh aspect of the invention shares all the characteristics of the antigen-binding domain 1 as described within the context of the CARs of the invention as it relates to its capacity to bind to the specific antigen, that is, the p95HER2. Therefore, all the particulars of the antigen-binding domain 1 of the CAR of the invention related to the binding to the p95HER2 peptide, apply to the antibody or antibody fragment of the item (i) of the eleventh aspect of the invention.

In addition, the skilled in the art will understand that the antibody or antibody fragment of the item (ii) of the eleventh aspect of the invention shares all the characteristics of the antigen-binding domain 2 as described within the context of the CARs of the invention as it relates to its capacity to bind to the specific antigen, that is, the p95HER2. Therefore, all the particulars of the antigen-binding domain 2 of the CAR of the invention related to the binding to the p95HER2 peptide, apply to the antibody or antibody fragment of the item (i) of the eleventh aspect of the invention.

In an embodiment, the ADC of item (i) of the eleventh aspect of the invention comprises in the at least one VH region the FR1, FR2, FR3 and FR4 the sequences of SEQ ID NOs: 152, 153, 154 and 155 or functionally equivalent variants thereof respectively and in the at least one VL region the FR1, FR2, FR3 and FR4 the sequences SEQ ID NOs: 156, 157, 158 and 159 or functionally equivalent variants thereof respectively.

In an embodiment, the ADC of item (i) of the eleventh aspect of the invention comprises the sequence SEQ ID NO: 160 or a functionally equivalent variant thereof in the at least one VL region and the sequence SEQ ID NO: 161 in the at least one VH region.

In another embodiment, the antibody or antibody fragment of item (i) of the ADC of the eleventh aspect of the invention is an ScFv, wherein the VH and VL regions are connected by a linker regions comprising the sequence SEQ ID NO: 29.

In another embodiment, the ADC of item (i) of the eleventh aspect of the invention comprises the sequence of SEQ ID NO: 162 or 195 or a functionally equivalent variant thereof.

In an embodiment, the ADC of item (ii) of the eleventh aspect of the invention comprises in the at least one VH region the FR1, FR2, FR3 and FR4 the sequences of SEQ ID NOs: 31, 32, 33 and 34 or functionally equivalent variants thereof respectively and in the at least one VL region the FR1, FR2, FR3 and FR4 the sequences SEQ ID NOs: 35, 36, 37 and 98 or functionally equivalent variants thereof respectively..

In an embodiment, the ADC of item (ii) of the eleventh aspect of the invention comprises the sequence SEQ ID NO: 39 or a functionally equivalent variant thereof in the at least one VL region and the sequence SEQ ID NO: 40 or 183 or a functionally equivalent thereof in the at least one VH region.

In another embodiment, the antibody or antibody fragment of item (ii) of the ADC of the eleventh aspect of the invention is an ScFv, wherein the VH and VL regions of the ScFv are connected by a linker region comprising the sequence SEQ ID NO: 29.

In an embodiment, the ADC of item (ii) of the eleventh aspect of the invention comprises the sequence SEQ ID NO: 41 or 185 or a functionally equivalent variant thereof.

In another embodiment, the ADC of item (iii) of the eleventh aspect of the invention comprises in the at least one VH region the FR1, FR2, FR3 and FR4 the sequences SEQ ID NOs: 42, 43, 44 and 45 or functionally equivalent variants thereof respectively and in the at least one VL region the FR1, FR2, FR3 and FR4 the sequences SEQ ID NOs: 46, 47, 48 and 49 or functionally equivalent variants thereof respectively.

In an embodiment, the ADC of item (iii) of the eleventh aspect of the invention comprises the sequence SEQ ID NO: 50 or 184 or a functionally equivalent variant thereof in the at least one VL and the sequence SEQ ID NO: 51 or a functionally equivalent thereof in the at least one VH region.

In another embodiment, the antibody or antibody fragment of item (iii) of the ADC of the eleventh aspect of the invention is an ScFv wherein the VH and VL regions are connected by a linker region comprising the sequence SEQ ID NO: 29.

In an embodiment, the ADC of item (iii) of the eleventh aspect of the invention comprises the sequence SEQ ID NO: 52 or 186 or a functionally equivalent variant thereof.

In a particular embodiment the ADCs are humanized. The term humanized has already been defined within the context of the CAR of the invention and applies equally to the ADCs of the invention. Similarly, suitable humanized sequences of the antibody or antibody fragments thereof have already been defined within the context of the CARs of the invention and the ScFv of the invention. In this sense, as the humanization makes reference to the variable regions of the antibodies, it is understood that all the particulars regarding the humanization of the ScFv1, antigen-binding domain 1 and antigen-binding domain 2 of the CARs of the invention apply equally to the antibody or antibody fragments of items (i), (ii) and (ii) of the ADCs of the invention respectively. In addition, all the particulars regarding the humanization of the ScFv, antigen-binding domain and antibody or antibody fragment of the invention apply equally to the antibody or antibody fragments of items (i), (ii) and (ii) of the ADCs of the invention respectively.

In a particular embodiment, when the antibody or antibody fragment thereof of the ADCs of the invention is an ScFv, the VH and VL regions may be connected by a linker region comprising SEQ ID NO: 29.

In a particular embodiment, the linker is located between the VH and the VL regions of the ScFv.. In an embodiment, the ScFv may have the structure VH-linker-VL or VL-linker-VH. In a particular embodiment, the linker is located C-terminally with respect to the VL region and N-terminally with respect to the VH region, that is, VL-linker-VH.

In a particular embodiment, the therapeutic agent is a cytotoxic agent.

The term "cytotoxic agent" as used herein refers to a substance that inhibits or prevents the function of cells and/or causes destruction of cells. The term is intended to include radioactive isotopes (e.g., At, I, Y, Re, Re, Sm, TBi, ∼P, C, and radioactive isotopes of Lu), chemotherapeutic agents; and toxins such as small molecule toxins or enzymatically active toxins of bacterial, fungal, plant or animal origin, including synthetic analogs and derivatives thereof.

In another particular embodiment, the cytotoxic agent is a chemotherapeutic agent.

A "chemotherapeutic agent" is a chemical agent (e.g., compound or drug) useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors.

Chemotherapeutic agents include compounds used in targeted therapy and conventional chemotherapy. Examples of chemotherapeutic agents include, but are not limited to, Erlotinib (TARCEVA®, Genentech/OSI Pharm.), Bortezomib (VELCADE®, Millenium Pharm.), Fulvestrant (FASLODEX®, Astrazeneca), Sutent (SU11248, Pfizer), Letrozole (FEMARA®, Novartis), Imatinib mesylate (GLEEVEC®, Novartis), PTK787/Z 222584 (Novartis), Oxaliplatin (Eloxatin®, Sanofi), 5-FU (5-fluorouracil), Leucovorin, Rapamycin (Sirolimus, RAPAMUNE®, Wyeth), Lapatinib (GSK572016, GlaxoSmithKline), Lonafarnib (SCH 66336), Sorafenib (BAY43-9006, Bayer Labs.), and Gefitinib (IRESSA®, Astrazeneca), AG1478, AG1571 (SU 5271; Sugen), alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphorarnide and trimethylomelarnine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, -2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g calicheamicin, especially calicheamicin gammall and calicheamicin omegall (Angew Chem Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinoraysins, actinomycin, authramycin, azaserine, bleomycins, cactinomycin, carabicin, canninomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptpnigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as anunoglutetWmide, mitotane, trilostane; folic acid replenisher such as frolinic acid; aceglatone; aldophosphamide glycoside; aminolevulinic acid; eniluracil; amsacrine; bestrabucil; bisanrrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2^2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE® doxetaxel (Rhone- Poulenc Rorer, Antony, France); chloranbucil; GEMZAR® gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeioda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY 1 17018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RI VISor® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1 ,3-dioxolane nucleoside cytosine analog); (iv) protein kinase inhibitors such as ME inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE®, Genta Inc.); (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAX1 D®; PROLEUKIN® rIL-2; topoisomerase 1 inhibitors such as LURTOTECAN®; ABARELIX® rmRH; (ix) antiangiogenic agents such as bevacizumab (AVASTIN®, Genentech); and pharmaceutically acceptable salts, acids and derivatives of any of the above.

Protein kinase inhibitors include tyrosine kinase inhibitors which inhibit to some extent tyrosine kinase activity of a tyrosine kinase such as an ErbB receptor. Examples of tyrosine kinase inhibitors include EGFR- 10 targeted drugs such as: (i) antibodies which bind to EGFR, including MAb 579 (ATCC CRL HB 8506), MAb 455 (ATCC CRL HB8507), MAb 225 (ATCC CRL 8508), MAb 528 (ATCC CRL 8509) (see, US 4943533, Mendelsohn et al.) and variants thereof, such as chimerized 225 (C225 or Cetuximab; ERBITUX®, Imclone) and reshaped human 225 (H225) (WO 96/40210, Imclone Systems Inc.); antibodies that bind type Π mutant EGFR (US 5212290); humanized and chimeric antibodies that bind EGFR (US 5891996); and human antibodies that bind EGFR, such as ABX-EGF (WO 98/50433); (ii) anti-EGFR antibody conjugated with a cyotoxic agent (EP 659439A2); and small molecules that bind to EGFR including ZD1839 or Gefitinib QRESSA™; Astra Zeneca), Erlotinib HC1 (CP-358774, TARCEVA™; Genentech/OSI) and AG1478, AG1571 (SU 5271; Sugen), quinazolines such as PD 153035,4-(3-chloroanilino) quinazoline, pyridopyrimidines, pyrirnidopyrirnidines, pyrrolopyrimidines, such as CGP 59326, CGP 60261 and CGP 62706, and pyrazolopyrimidines, 4- 20 (phenylamino)-7H-pyrrolo[2,3-dJ pyrimidines, curcumin (diferuloyl methane, 4,5-bis (4- fluoroanilino)phthaiimide), tyrphostines containing nitrothiophene moieties; PD-0183805 (Warner-Lambert) and antisense molecules (e.g., those that bind to ErbB-encoding nucleic acid).

In yet another embodiment, the ADC cytotoxic or chemotherapeutic agent of the present invention is an anti-tubulin agent. In more specific embodiments, the cytotoxic agent is selected from the group consisting of vinca alkaloids, podophyllotoxins, taxanes, baccatin derivatives, cryptophycin, maytansinoids, combretastatins, and dolastatin. In a more specific embodiment, the cytotoxic agent is vincristine, vinblastine, vindesine, vinorelbine, VP-16, camptothecin, paclitaxel, docetaxel, episilon A, episilon B, nocodazole, colchicine, cortisimide, estramustine, semadotine, discodermolide, maytansine DM-1, auristatin or other dolastatin derivatives, such as auristatin E or auristatin F, AEB, AEVB, AEFP, MMAE (monomethyl auristatin E), MMAF (monomethyl auristatin F), eruterobin or netropsin.

In addition, powerful chemotherapeutic agents such as CC-1065 analogs, calikiamycin, maytansine, dolastatin 10 analogs, lysoxin, and palytoxin can be linked to ADCs using conditionally stable linkers to form potent immunoconjugates.

In a particular embodiment, the chemotherapeutic agent is selected from lapatinib, tucatinib and neratinib.

In other embodiments, an antibody of the invention or fragment or variant thereof is conjugated to a therapeutic agent or drug moiety that modifies a given biological response. The therapeutic agent or drug moiety is not limited to classic chemotherapeutic agents. For example, the drug moiety may be a protein or polypeptide that possesses the desired biological activity. Such proteins include, for example, toxins such as abrin, ricin A, Pseudomonas exotoxin, cholera toxin, or diphtheria toxin; proteins such as tumor necrosis factor, α-interferon, β-interferon, nerve growth factor, platelet-derived growth factor Tissue plasminogen activator, apoptotic agents such as TNF-α, TNF-β, AIMI, AIMII, Fas ligand, and VEGf, thrombotic or antiangiogenic agents such as angiostatin or endostatin; or biological response modifiers For example, lymphokines (eg, interleukin-1 (IL-1), interleukin-2 (IL-2), interleukin-4 (IL-4), interleukin Kin-6 (IL-6), Interleukin-7 (IL-7), Interleukin-9 (IL-9), Interleukin-15 (IL-15), Interleukin-12 (IL-12), Granules Sphere macrophage colony stimulating factor (GM-CSF), and granulocyte colony stimulating factor (G-CSF)), or growth factors (eg, growth hormone (GH)).

Techniques for conjugating therapeutic moieties to antibodies are well known. The therapeutic moiety may be conjugated to the antibody by any method known in the art including, but not limited to, aldehyde / Schiff bond, sulfhydryl bond, acid labile bond, cis-aconityl (Aconityl) bond, hydrazone bond, and enzyme-degradable bond. The fusion with the therapeutic portion of the antibody need not be direct, but may occur through a linker sequence. Such linker molecules are generally known in the art.

In some embodiments, the conjugation of the drug to the antibody is performed by a linker that is sensitive to the environment (eg, the lysosomal environment due to the endosomal environment or pH or protease sensitivity).

In one embodiment, the linker is an acid labile hydrazone or hydrazide group that is hydrolyzed within the lysosome. In other embodiments, the drug can be conjugated to the antibody via other acid labile linkers such as cis-aconitic amides, orthoesters, acetals and ketals. Such linkers are relatively stable under neutral pH conditions, such as blood conditions, but are unstable at pH 5 or lower, ie, at a pH close to lysosomes.

In other embodiments, the drug binds to the antibody of the invention using a peptide spacer that is cleaved by intracellular proteases. Target enzymes include cathepsins B and D and plasmin, all of which are known to hydrolyze dipeptide drug derivatives resulting in the release of active drugs inside the target cell

In certain non-limiting embodiments of the present invention, the linker region between the drug and antibody portions of the ADC can be cleaved under certain conditions, with linker cleavage or hydrolysis. The drug moiety is released from the antibody moiety. Preferably, this linker is sensitive to cleavage or hydrolysis under intracellular conditions.

In one embodiment, the linker region between the drug and antibody portions of the ADC can be hydrolyzed if the pH changes by a certain value or exceeds a certain value.

In yet another specific embodiment, the linker is a disulfide linker. Various disulfide linkers are known in the art, including but not limited to SATA (N-succinimidyl-S-acetylthioacetate), SPDP (N-succinimidyl-3- (2-pyridyldithio) propionate), SPDB (N-succinimidyl-3- (2-pyridyldithio) butyrate) and SMPT (N-succinimidyl-oxycarbonyl-α-methyl-α-(2-pyridyldithio) toluene) Is included. SPDB and SMPT.

Methods for Producing Antibodies Antibodies or fragments thereof can be produced by any method known in the art for antibody synthesis, particularly chemical synthesis, or preferably by recombinant expression techniques.

### Diagnostic method

In a twelfth aspect, the invention relates to a method of cancer diagnosis in a patient which comprises:
(i) contacting a sample of the patient containing tumor cells with the ScFv1, antigen-binding 1 domain or antibody of the invention and
(ii) detecting the binding of the ScFv, antigen-binding domain or antibody to cells in the sample,
wherein the presence of binding is indicative that the patient suffers from cancer.
The term "cancer" or "tumour" or "tumour disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

Examples of cancer or tumor include without limitation, breast, heart, lung, small intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukaemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelialtumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor.

In a particular embodiment, the cancer is breast cancer. In preferred embodiment, the cancer is a p95HER2 positive cancer.

The terms "p95HER2" and HER2 have already been defined within the context of the CARs of the invention and said definition applies equally to the present method of diagnosis.

A "cancer that is p95HER2 positive" refers to a cancer in which at least a portion of the cancer cells contain p95HER2, as determined by immunohistochemistry (IHC), Western blot, or VeraTag® assay (Monogram Biosciences). In some embodiments, a cancer is determined to be p95HER2 positive by IHC. In some such embodiments, a cancer is determined to be p95HER2 positive using the methods described in Sperinde et al., Clin. Canc. Res., 2010, 16(16): 4226-4235, such as methods using anti-p95 antibody clone D9 in a VeraTag assay. In some embodiments, a cancer is determined to be p95HER2 positive using the methods described in U.S. Pat. No. 8,389,227 B2, such as methods using an antibody produced by a hybridoma cell line deposited with the Deutschland Sammlung von Mikroorganismen and Zellen under accession number DSM ACC2904 or DSM ACC2980. In some embodiments, a cancer is determined to be p95HER2 positive according to the assay manufacturer's or assay laboratory's guidelines. p95HER2 refers to a collection of carboxy-terminal HER2 fragments, which, in some embodiments, may be divided into 95- to 100-kDa fragments and 100- to 115-kDa fragments. See, e.g., Arribas et al., Cancer Res., 2011, 71: 1515-1519. In some embodiments, a cancer that is p95HER2 positive contains 100- to 115-kDa fragments of HER2.

The terms "detection", "diagnosing", "diagnosis" or derivatives of the words, are used herein indistinctly and refer to the identification of the presence or characteristic of a pathological condition. It refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As the person skilled in the art will understand, such a diagnosis may not be correct for 100% of the subjects to diagnose, although preferred it is. The term, however, requires that a statistically significant part of the subjects can be identified as suffering from cancer in the context of the invention. The skilled in the art may determine whether a party is statistically significant using different statistical evaluation tools well known, for example, by determination of confidence intervals, the p-value determination, Student's-test, the Mann-Whitney, etc. Preferred confidence intervals are at least, 50%, at least 60%, at least 70%, at least 80%, at least 90% or at least 95%. The p-values are preferably, 0,015 , 0,001, 0,0005 or less.

In general, the method includes obtaining a sample suspected of expressing the p95HER2 antigen and contacting the sample with an ScFv, antigen binding domain or antibody capable of selectively binding or detecting p95HER2 antigen, under conditions effective to allow the formation of immunocomplexes.

The sample may be any sample that is suspected of containing the p95HER2 antigen, such as, for example, a tissue section or specimen, a homogenized tissue extract, a cell, an organelle, separated and/or purified forms of any of the above antigen-containing compositions, or any biological fluid, including blood, serum and plasma. In a preferred embodiment, the sample is a tumour sample. The sample is preferably a "tumor sample" which is a sample derived from, or comprising tumor cells from, a patient's tumor. Examples of tumor samples herein include, but are not limited to, tumor biopsies, circulating tumor cells, circulating plasma proteins, ascitic fluid, primary cell cultures or cell lines derived from tumors or exhibiting tumor-like properties, as well as preserved tumor samples, such as formalin-fixed, paraffin-embedded tumor samples or frozen tumor samples.

Contacting the chosen biological sample with the antibody of the invention under suitable and effective conditions and for a period of time sufficient to allow the formation of immune complexes is generally a matter of simply adding the ScFv1, antigen binding domain 1 or antibody of the invention to the sample and incubating the mixture for a period of time long enough for the antibodies to form immune complexes.

The effective conditions preferably include diluting the sample and/or ScFv1, antigen-binding domain 1 or antibody of the invention with solutions such as BSA, bovine gamma globulin (BGG) or phosphate buffered saline (PBS)/Tween. These added agents also tend to assist in the reduction of nonspecific background.

The "suitable" or "adequate" conditions also mean that the incubation is at a temperature or for a period of time sufficient to allow effective binding. Incubation steps are typically from about 1 to 2 to 4 hours or so, at temperatures preferably on the order of 25° C. to 27° C., or may be overnight at about 4° C. or so.

The determination of the amount of complex formed may be done in a number of ways. In a preferred embodiment, the antibody is labelled, and binding determined directly. For example, this may be done by attaching the p95HER2 antigen protein to a solid support, adding the labelled ScFv, antigen binding domain or antibody (for example a fluorescent label), washing off excess reagent, and determining whether the label is present on the solid support. Various blocking and washing steps may be utilized as is known in the art.

In general, the detection of immunocomplex formation is well known in the art and may be achieved through the application of numerous approaches. These methods are generally based upon the detection of a label or marker, such as any of those radioactive, fluorescent, biological and enzymatic tags. Of course, one may find additional advantages through the use of a secondary binding ligand such as a second antibody and/or a biotin/avidin ligand binding arrangement, as is known in the art.

In a particular embodiment, the ScFv1, antigen binding domain 1 or antibody of the invention are arranged on a solid support.

ScFvs or other polypeptides such as other antigen-binding domains or antibodies may be immobilized onto a variety of solid supports for use in assays. Solid phases that may be used to immobilize specific binding members include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the ScFv, antigen-binding domain or antibody or a plurality thereof in an array on solid support. This strip could then be dipped into the test sample and then processed quickly through washes and detection steps to generate a measurable signal, such as a coloured spot. ScFvs or other polypeptides, such as other antigen-binding domains or antibodies may be bound to specific zones of assay devices either by conjugating directly to an assay device surface, or by indirect binding.

As the person skilled in the art will understand, there is a wide range of conventional assays that can be used in the present invention which use an ScFv1, antigen binding domain 1 or antibody of the invention that is not labelled (primary antibody) and an antibody of the invention that is labelled (secondary antibody); these techniques include Western blot or immunoblot, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical and immunohistochemical techniques, flow cytometry or multiplex detection techniques based on using protein microspheres, biochips or microarrays which include the ScFv1, antigen-binding domain 1 or antibody of the invention. Other ways of detecting and quantifying p95HER2 antigen using the antibody of the invention include affinity chromatography techniques, ligand binding assays or lectin binding assays.

It will also be understood that ScFvs, antigen-binding domains or antibodies that are not labelled need to be detected with an additional reagent, for example, a secondary antibody that is labelled, which will be labelled. This is particularly useful in order to increase the sensibility of the detection method, since it allows the signal to be amplified.

In addition, the detection of the antibody can also be carried out by detecting changes in the physical properties in the sample that occur as a result of the binding of the antibody to its cognate antigen. These assays include determining a transmission-related parameter in a sample, which are known in the art. The term "transmission-related parameter", as used herein, relates to a parameter indicating or correlating with the ratio of transmitted light versus incident light of a sample or to a parameter derived therefrom.

In an embodiment, a transmission-related parameter is determined by turbidimetry or by nephelometry.

In another embodiment, the binding of the ScFv, antigen binding domain or antibody to its cognate antigen can be detected by Surface plasmon resonance (SPR).

As used herein, SPR refers to a phenomenon that the intensity of a reflected light decreases sharply at a particular angle of incidence (i.e., an angle of resonance) when a laser beam is irradiated to a metal thin film. SPR is a measurement method based on the phenomenon described above and is capable of assaying a substance adsorbed on the surface of the metal thin film, which is a sensor, with high sensitivity. According to the present invention, for example, the target substance in the sample can then be detected by immobilizing one or more ScFvs, antigen-binding domains or antibodies according to the present invention on the surface of the metal thin film beforehand, allowing the sample to pass through the surface of the metal thin film, and detecting the difference of the amount of the substance adsorbed on the surface of the metal thin film resulting from the binding of the ScFv, antigen-binding domain or antibody and the target antigen, between before and after the sample passes therethrough.

In an embodiment, the presence of binding, measured by any of the above related techniques or any other known in the art, is indicative that the patient suffers from cancer.

In another embodiment, the diagnostic method of the invention comprises comparing the levels obtained in the subject under study with a reference value, whereby, increased levels of p95HER2 with respect to a reference value are indicative that the patient suffers from cancer.

The term "increased", in relation to the levels of p95HER2 relates to any level of expression of p95HER2 detected using the ScFv1, antigen binding domain 1 or antibody according to the invention in a sample lower than the reference value. Thus, p95HER2 expression levels are considered to be decreased or to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value

The term "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. In one embodiment, the reference value corresponds to the level of p95HER2 expression determined in a healthy subject, whereby a healthy subject is understood as a subject that shows no proliferative disease at the moment the levels p95HER2 expression are determined and that, preferably, shows no history of cancer.

In another embodiment, the reference value corresponds to an average or mean level of the p95HER2 expression determined from a pool of samples obtained from a group of patients who are well documented from the clinical point of view, and who present no disease, particularly who are not suffering from cancer, particularly not suffering from a p95HER2 positive cancer. In said samples, the expression levels can be determined, for example by means of the determination of the average expression level in a reference population. In the determination of the reference value, it is necessary to take into consideration some characteristics of the type of sample, such as age, gender, the physical state or other characteristics of the patient. For example, the reference sample can be obtained from identical amounts of a group of at least 2, at least 10, at least 100 to more than 1000 individuals, such that the population is statistically significant.

The term "expression" or "expression level", as used herein, refers to a measurable quantity of a protein or an antigen. As understood by the person skilled in the art, the expression level can be quantified by measuring the protein or antigen. Thus, in the present case the expression level of the p95HER2 is measured by determining the amount of immunocomplex formed between the p95HER2 antigen and the ScFv1, antigen binding domain 1 or antibody of the invention and can be done in a number of ways related above and known by the skilled person.

### Pharmaceutical compositions

In a thirteenth aspect, the invention relates to a pharmaceutical composition comprising any of the host cells of the fourth aspect the invention, and/or an ScFv1, antigen binding domain 1 or antibody according to the fifth, sixth and seventh aspects of the invention and/or the ADC of the invention and at least one pharmaceutically acceptable excipient.

The term "pharmaceutical composition" is such a form that allows the biological activity of the active ingredient contained therein to be effective and has unacceptable toxicity for the subject to which the composition is administered. Refers to a preparation that does not contain additional ingredients.

"Pharmaceutically acceptable carrier" refers to an ingredient of a pharmaceutical composition other than an active ingredient that is non-toxic to a subject. Pharmaceutically acceptable carriers include but are not limited to buffers, excipients, stabilizers or preservatives.

In a particular embodiment, the pharmaceutical composition comprises the host cells of the invention, more particularly, the immune cells (e.g. T, NK or NKT cells) that have been genetically engineered to express any of the CARs of the invention, that is the CAR comprising the ScFv1, antigen-binding domain 1, antigen-binding domain 2 or any combination thereof. In another embodiment, the pharmaceutical composition of the invention comprises the ScFv1, antigen binding domain 1 or antibody of the invention. In another embodiment, the pharmaceutical composition comprises both, the host cells and the ScFv1, antigen binding domain 1 or antibody of the invention. In another embodiment, the pharmaceutical composition comprises the ADCs of the invention.

Pharmaceutical compositions and formulations as described herein can be prepared by mixing the active ingredients having the desired degree of purity with one or more optional pharmaceutically acceptable carriers (Remington's Pharmaceutical Sciences 22nd edition, 2012), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn- protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.).

### Method of treatment

The present invention provides methods for immunotherapy comprising administering a therapeutic effective amount of the ScFv1, antigen-binding domain 1, antibody , ADC or immune cells of the present invention. In one embodiments, a medical disease or disorder is treated by transfer of an immune cell population that elicits an immune response.

Thus, in a fourteenth aspect, the invention relates to any of the host of the fourth aspect of the invention and/or the ScFv1, antigen binding domain 1 or antibody of the fifth, sixth and seventh aspects of the invention and/or the ADC of the eleventh aspect of the invention for use in medicine.

In final aspect, the invention relates to any of the host cell of the fourth aspect of the invention and/or the ScFv1, antigen binding fragment 1 or antibody of the fifth, sixth and seventh aspects of the invention and/or the ADC of the eleventh aspect of the invention for use in a method of preventing or treating cancer.

In a particular embodiment, the cancer is breast cancer. In a preferred embodiment, the cancer is p95HER2 positive.

As used herein, the terms "treat", "treatment", "treatment", or "amelioration". The term refers to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (ie, not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment). In some embodiments, treating cancer includes reducing tumor volume, reducing the number of cancer cells, suppressing cancer metastasis, prolonging life, reducing cancer cell growth, reducing cell survival, or reducing cancerous status It involves amelioration of the various physiological symptoms involved.

In certain embodiments of the present disclosure, immune cells are delivered to an individual in need thereof, such as an individual that has cancer. The cells then enhance the individual's immune system to attack the respective cancer cells. In some cases, the individual is provided with one or more doses of the immune cells. In cases where the individual is provided with two or more doses of the immune cells, the duration between the administrations should be sufficient to allow time for propagation in the individual, and in specific embodiments the duration between doses is 1, 2, 3, 4, 5, 6, 7, or more days.

In some embodiments, the subject can be administered nonmyeloablative lymphodepleting chemotherapy prior to the immune cell therapy. The nonmyeloablative lymphodepleting chemotherapy can be any suitable such therapy, which can be administered by any suitable route. The nonmyeloablative lymphodepleting chemotherapy can comprise, for example, the administration of cyclophosphamide and fludarabine, particularly if the cancer is melanoma, which can be metastatic. An exemplary route of administering cyclophosphamide and fludarabine is intravenously. Likewise, any suitable dose of cyclophosphamide and fludarabine can be administered. In particular aspects, around 60 mg/kg of cyclophosphamide is administered for two days after which around 25 mg/m2 fludarabine is administered for five days.

In certain embodiments, a growth factor that promotes the growth and activation of the immune cells is administered to the subject either concomitantly with the immune cells or subsequently to the immune cells. The immune cell growth factor can be any suitable growth factor that promotes the growth and activation of the immune cells. Examples of suitable immune cell growth factors include interleukin (IL)-2, IL-7, IL-15, and IL-12, which can be used alone or in various combinations, such as IL-2 and IL-7, IL-2 and IL-15, IL-7 and IL-15, IL-2, IL-7 and IL-15, IL-12 and IL-7, IL-12 and IL-15, or IL-12 and IL2.

Therapeutically effective amounts of immune cells can be administered by a number of routes, including parenteral administration, for example, intravenous, intraperitoneal, intramuscular, intrasternal, or intraarticular injection, or infusion.

The immune cell population can be administered in treatment regimens consistent with the disease, for example a single or a few doses over one to several days to ameliorate a disease state or periodic doses over an extended time to inhibit disease progression and prevent disease recurrence. The precise dose to be employed in the formulation will also depend on the route of administration, and the seriousness of the disease or disorder, and should be decided according to the judgment of the practitioner and each patient's circumstances. The therapeutically effective number of immune cells will be dependent on the subject being treated, the severity and type of the affliction, and the manner of administration. In some embodiments, a therapeutically effective number of immune cells can vary from about 5 x 10⁶ cells per kg body weight to about 7.5 x 10⁸ cells per kg body weight, such as about 2x 10⁷ cells to about 5x 10⁸ cells per kg body weight, or about 5 x 10⁷ cells to about 2x 10⁸ cells per kg body weight. The exact number of immune cells is readily determined by one of skill in the art based on the age, weight, sex, and physiological condition of the subject. Effective doses can be extrapolated from dose-response curves derived from in vitro or animal model test systems.

In certain embodiments, the compositions and methods of the present embodiments involve an immune cell population or ScFv in combination with at least one additional therapy. The additional therapy may be radiation therapy, surgery (e.g. , lumpectomy and a mastectomy), chemotherapy, gene therapy, DNA therapy, viral therapy, RNA therapy, immunotherapy, bone marrow transplantation, nanotherapy, monoclonal antibody therapy, or a combination of the foregoing. The additional therapy may be in the form of adjuvant or neoadjuvant therapy.

In some embodiments, the additional therapy is the administration of small molecule enzymatic inhibitor or anti-metastatic agent. In some embodiments, the additional therapy is the administration of side- effect limiting agents (e.g. agents intended to lessen the occurrence and/or severity of side effects of treatment, such as anti-nausea agents, etc.). In some embodiments, the additional therapy is radiation therapy. In some embodiments, the additional therapy is surgery. In some embodiments, the additional therapy is a combination of radiation therapy and surgery. In some embodiments, the additional therapy is gamma irradiation. In some embodiments, the additional therapy is therapy targeting PBK/AKT/mTOR pathway, HSP90 inhibitor, tubulin inhibitor, apoptosis inhibitor, and/or chemopreventative agent. The additional therapy may be one or more of the chemotherapeutic agents known in the art.

The pharmaceutical composition of the invention or immune cell therapy of the invention may be administered before, during, after, or in various combinations relative to an additional cancer therapy, such as immune checkpoint therapy. The administrations may be in intervals ranging from concurrently to minutes to days to weeks. In some embodiments where the immune cell therapy is provided to a patient separately from an additional therapeutic agent, one would generally ensure that a significant period of time did not expire between the time of each delivery, such that the two compounds would still be able to exert an advantageously combined effect on the patient. In such instances, it is contemplated that one may provide a patient with the antibody therapy and the anti-cancer therapy within about 12 to 24 or 72 h of each other and, more particularly, within about 6-12 h of each other. In some situations it may be desirable to extend the time period for treatment significantly where several days (2, 3, 4, 5, 6, or 7) to several weeks (1, 2, 3, 4, 5, 6, 7, or 8) lapse between respective administrations.

Various combinations may be employed. For the example below the pharmaceutical composition of the invention or the immune cell therapy is "A" and an anti-cancer therapy is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of any compound or therapy of the present embodiments to a patient will follow general protocols for the administration of such compounds, taking into account the toxicity, if any, of the agents. Therefore, in some embodiments there is a step of monitoring toxicity that is attributable to combination therapy.

A wide variety of chemotherapeutic agents may be used in in combination with the pharmaceutical composition of the invention or immune cell therapy. The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis.

Examples of chemotherapeutic agents include alkylating agents, such as thiotepa and cyclosphosphamide; alkyl sulfonates, such as busulfan, improsulfan, and piposulfan; aziridines, such as benzodopa, carboquone, meturedopa, and uredopa; ethylenimines and methylamelamines, including altretamine, triethylenemelamine, trietylenephosphoramide, triethiylenethiophosphoramide, and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards, such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, and uracil mustard; nitrosureas, such as carmustine, chlorozotocin, fotemustine, lomustine, nimustine, and ranimnustine; antibiotics, such as the enediyne antibiotics (e.g., calicheamicin, especially calicheamicin gammall and calicheamicin omegall); dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, carminomycin, carzinophilin, chromomycinis, dactinomycin, daunorubicin, detorubicin, 6- diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, such as mitomycin C, mycophenolic acid, nogalarnycin, olivomycins, peplomycin, potfiromycin, puromycin, quelamycin, rodorubicin, streptonigrin, streptozocin, tubercidin, ubenimex, zinostatin, and zorubicin; anti -metabolites, such as methotrexate and 5-fiuorouracil (5-FU); folic acid analogues, such as denopterin, pteropterin, and trimetrexate; purine analogs, such as fludarabine, 6-mercaptopurine, thiamiprine, and thioguanine; pyrimidine analogs, such as ancitabine, azacitidine, 6-azauridine, carmofur, cytarabine, dideoxyuridine, doxifluridine, enocitabine, and floxuridine; androgens, such as calusterone, dromostanolone propionate, epitiostanol, mepitiostane, and testolactone; anti-adrenals, such as mitotane and trilostane; folic acid replenisher, such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids, such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSKpolysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, e.g., paclitaxel and docetaxel gemcitabine; 6-thioguanine; mercaptopurine; platinum coordination complexes, such as cisplatin, oxaliplatin, and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-11); topoisomerase inhibitor RFS 2000; difluorometlhylornithine (DMFO); retinoids, such as retinoic acid; capecitabine; carboplatin, procarbazine,plicomycin, gemcitabien, navelbine, farnesyl-protein tansferase inhibitors, transplatinum, and pharmaceutically acceptable salts, acids, or derivatives of any of the above.

The invention is defined below by the following aspects:
1. A chimeric antigen receptor (CAR) comprising:
   (i) an antigen-binding domain specific for p95HER2,
   (ii) a transmembrane domain and
   (iii) at least one intracellular signaling domain and/or costimulatory domain
   wherein the antigen-binding domain is selected from the group consisting of:
   (i) an ScFv (ScFv1) characterized in that:
      - the framework regions of the VL and VH regions are humanized,
      - the VL region is located N-terminally with respect to the VH region,
      - the CDR1, CDR2 and CDR3 of the VH region comprise, respectively, the sequences of SEQ ID NO:1, 2 and 3 or functionally equivalent variants thereof or the sequences of SEQ ID NO: 1, 174 and 3 or functionally equivalent variants thereof, and
      - the CDR1, CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof,
   (ii) an antigen-binding domain (antigen-binding domain 1), characterized in that:
      - it has at least one VH region and at least one VL region,
      - the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
      - the CDR1, CDR2 and CDR3 of the at least one VL region comprise respectively, the sequences of SEQ ID NO: 10, 11, and 12 or functionally equivalent variants thereof,
   (iii) an antigen-binding domain (antigen-binding domain 2), characterized in that:
      - it has at least one VH region and at least one VL region,
      - the CDR1, CDR2 and CDR3 of the at least one VH region comprise, respectively, the sequences of SEQ ID NO: 13, 14 and 15 or functionally equivalent variants thereof,
      - and the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.
2. The CAR of aspect 1 wherein the FR1, FR2, FR2 and FR4 of the VH region of the ScFv1 comprise respectively the sequences of SEQ ID NO: 19, 20, 21 and 22 or functionally equivalent variants thereof and FR1, FR2, FR3 and FR4 of the VL region of the ScFv1 comprise respectively the sequences of SEQ ID NO: 23, 24, 25 and 26 or functionally equivalent variants thereof.
3. The CAR of aspect 2 wherein the VL of ScFv1 comprises the sequence of SEQ ID NO: 27 or 180 or functionally equivalent variants thereof and the VH comprises the sequence of SEQ ID NO: 28 or. 181 or functionally equivalent vatriants thereof.
4. The CAR of aspect 1 or 2 wherein the VH and VL regions of the ScFv1 are connected by a linker region comprising the sequence SEQ ID NO: 29.
5. The CAR of aspect 4 wherein the ScFv1 comprises the sequence of SEQ ID NO: 30 or 182 or functionally equivalent variants thereof.
6. The CAR of aspect 1 wherein the FR1, FR2, FR3 and FR4 of the at least one VH region of the antigen-binding domain 1 comprise respectively the sequences of SEQ ID NO: 31, 32, 33 and 34, SEQ ID NO: 65, 66, 67 and 68, SEQ ID NO: 73, 74, 75 and 76 or SEQ ID NO: 81, 82, 83 and 84 or functionally equivalent variants thereof and the FR1, FR2, FR3, and FR4 of the at least one VL region of the antigen-binding domain 1 comprise respectively the sequences of SEQ ID NO: 35, 36, 37 and 38, SEQ ID NO: 69, 70, 71, and 72, SEQ ID NO: 77, 78, 79, and 80 or SEQ ID NO: 85, 86, 87 and 88 or functionally equivalent variants thereof.
7. The CAR of aspect 6 wherein the at least one VL region comprises the sequence of SEQ ID NO: 39, 54, 56 or 58 or functionally equivalent variants thereof and the at least one VH region comprises the sequence of SEQ ID NO: 40, 183, 53, 55 or 57 or functionally equivalent variants thereof,.
8. The CAR of aspects 6 or 7 wherein, the antigen-binding domain 1 is an ScFv, and wherein the VH and VL regions of the ScFv are connected by a linker region comprising SEQ ID NO: 29.
9. The CAR of aspect 8 wherein the ScFv comprises the sequence of SEQ ID NO: 41, 185, 187, 188 or 189 .or functionally equivalent variants thereof.
10. The CAR of aspect 1, wherein the FR1, FR2, FR3 and FR4 of the at least one VH region of the antigen binding domain 2 comprise respectively the sequences of SEQ ID NO: 42, 43, 44 and 45, SEQ ID NO: 89, 90, 91 and 92, SEQ ID NO: 97, 98, 99 and 100 or SE ID NO: 105, 106, 107 and 108 or functionally equivalent variants thereof and the FR1, FR2, FR3 and FR4 of the at least one VL region of the antigen comprise respectively the sequences of SEQ ID NO: 46, 47, 48 and 49, SEQ ID NO: 93, 94, 95 or 96, SEQ ID NO: 109, 110, 111 and 112 or functionally equivalent variants thereof.
11. The CAR of aspect 10, wherein the at least one VL region comprises the sequence of SEQ ID NO: 50 ,184, 60, 62 or 64 and the at least one VH comprises the sequence of selected from SEQ ID NO: 51, 59, 61 and 63. or functionally equivalent variants thereof.
12. The CAR of aspects 10 or 11, wherein the antigen-binding domain 2 is an ScFv, and wherein the VH and VL regions of the ScFv are connected by a linker region comprising SEQ ID NO: 29.
13. The CAR of aspect 12, wherein the ScFv comprises the sequence of SEQ ID NO: 52, 186, 190, 191 or 192. or functionally equivalent variants thereof.
14. The CAR of any of aspects 1 to 13 further comprising a hinge domain between the antigen binding domain and the transmembrane domain.
15. The CAR of aspect 14 wherein the hinge domain is the CD8 hinge domain.
16. The CAR according to any of aspects 1 to 15 wherein the transmembrane domain is selected from the group consisting of the CD4 transmembrane domain, the CD8 transmembrane domain, the CD28 transmembrane domain, the 4-1BB transmembrane domain, the CTLA4 transmembrane domain, the CD27 transmembrane domain and the CD3 zeta transmembrane domain.
17. The CAR according to any of aspects 1 to 16 wherein the at least one intracellular signaling domain comprises a costimulatory domain, a primary signaling domain, or any combination thereof.
18. The CAR according to aspect 17 wherein the at least one intracellular signaling domain comprises intracellular domain of the costimulatory molecules selected from OX40, CD70, CD27, CD28, CD5, ICAM-1, LFA-1 (CD11a/CD18), ICOS (CD278), DAP10, DAP 12, and 4-1BB (CD137), or any combination thereof.
19. The CAR according to any of aspects 1 to 18 wherein the at least one intracellular signaling domain further comprises a CD3 zeta intracellular domain.
20. The CAR according to aspect 19 wherein the at least one intracellular signaling domain is arranged on a N-terminal side relative to the CD3 zeta intracellular domain.
21 The CAR according to aspect 20 wherein the hinge region is the CD8 hinge domain, the transmembrane domain is the CD28 transmembrane domain and the intracellular signaling domain is the CD28 costimulatory domain.
22. A nucleic acid encoding a CAR according to any of aspects 1 to 21.
23. The nucleic acid of aspect 22 wherein the encoded CAR further comprises a leader sequence.
24. The nucleic acid of aspect 23 wherein the leader sequence is the CD8 leader sequence.
25. An expression vector comprising the nucleic acid of aspects 22 to 24.
26. A host cell comprising the chimeric antigen receptor (CAR) of any of aspects 1 to 21, the nucleic acid of any of aspects 22 to 24 or the expression vector of aspect 25.
27. The host cell of aspect 26 wherein the cell is an immune cell.
28. The host cell of aspect 27 wherein the immune cells is a T cell, NK cell, or NKT cell.
29. The host cell of aspect 28 wherein the T cell is a CD8+ T cell.
30. An ScFv characterized in that:
   - the CDR1, CDR2 and CDR3 of the VH region comprise, respectively, the sequences of SEQ ID NO: 1, 2 and 3 or functionally equivalent variants thereof or the sequences of SEQ ID NO: 1, 174 and 3 or functionally equivalent variants thereof, and
   - the CDR1, CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof.
31. The ScFv according to aspect 30 wherein the FR1, FR2, FR3 and FR4 of the VH region comprise respectively the sequences of SEQ ID NO: 152, 153, 154 and 155, SEQ ID NO: 19, 20, 21 and 22 or SEQ ID NO: 163, 164,165 and 166 or functionally equivalent variants thereof and FR1, FR2, FR3 and FR4 of the VL region comprise respectively the sequences of SEQ ID NO: 156, 157, 158 and 159, SEQ ID NO: 23, 24, 25 and 26 or SEQ ID NO: 167, 168, 169 or 170 or functionally equivalent variants thereof.
32. The ScFv according to aspect 31 wherein the VL comprises the sequence of SEQ ID NO: 160, 193, 27, 171 or 180 or functionally equivalent variants thereof and the VH comprises the sequence of SEQ ID NO: 161,194, 28, 172 or 181 or functionally equivalent variants thereof.
33. The ScFv according to aspect 32 wherein the VH and VL regions of the ScFv1 are connected by a linker region comprising SEQ ID NO: 29.
34. The ScFv according to aspect 33 wherein the ScFv comprises the sequence of SEQ ID NO: 162,195, 30, 173 or 182 or functionally equivalent variants thereof.
35. An antigen-binding domain characterized in that:
   - it has at least one VH region and at least one VL region,
   - the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
   - the CDR1, CDR2 and CDR3 of the at least one VL region comprise respectively, the sequences of SEQ ID NO: 10, 11, and 12 or functionally equivalent variants thereof.
36. The antigen-binding domain according to aspect 35 wherein the FR1, FR2, FR3 and FR4 of the at least one VH region comprise respectively the sequences of SEQ ID NO: 31, 32, 33 and 34, SEQ ID NO: 65, 66, 67 and 68, SEQ ID NO: 73, 74, 75 and 76 or SEQ ID NO: 81, 82, 83 and 84 or functionally equivalent variants thereof and FR1, FR2, FR3 and FR4 of the at least one VL region comprise respectively the sequences of SEQ ID NO: 35, 36, 37 and 38, SEQ ID NO: 69, 70, 71, 72, SEQ ID NO: 77, 78, 79 and 80 or SEQ ID NO: 85, 86, 87 and 88 or functionally equivalent variants thereof.
37. The antigen-binding domain according to aspect 36 wherein the at least one VL region comprises the sequence of SEQ ID NO: 39, 54, 56 and 58 or functionally equivalent variants thereof and the at least one VH region comprises the sequence of SEQ ID NO: 40, 183, 53, 55 and 57 or functionally equivalent variants thereof.
38. The antigen-binding domain according to aspect 37 wherein, the antigen-binding domain 1 is an ScFv, and the VH and VL regions of the antigen-binding domain are connected by a linker region comprising SEQ ID NO: 29.
39. The antigen-binding domain according to aspect 38, wherein the ScFv comprises the sequence of SEQ ID NO: 41,185, 187, 188 or 189 or functionally equivalent variants thereof.
40. An antibody or an antibody fragment thereof characterized in that,
   - at least one VH region and at least one VL region,
   - the CDR1, CDR2 and CDR3 of the at least one VH region comprise, respectively, the sequences of SEQ ID NO: 13, 14 and 15 or functionally equivalent variants thereof, and
   - the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.
41. The antibody or antibody fragment thereof according to aspect 40 wherein the FR1, FR2, FR3 and FR4 of the at least one VH region comprise respectively the sequences of SEQ ID NO: 42, 43, 44 and 45, SEQ ID NO: 89, 90, 91 and 92, SEQ ID NO:97, 98, 99 and 100 or SEQ ID NO: 105, 106, 107 and 108 or functionally equivalent variants thereof and FR1, FR2, FR3 and FR4 of the at least one VL region comprise respectively the sequences of SEQ ID NO: 46, 47, 48 and 49, SEQ ID NO: 93, 94, 95 and 96, SEQ ID NO: 101, 102, 103 and 104 or SEQ ID NO: 109, 110, 11 and 112 or functionally equivalent variants thereof.
42. The antibody or antibody fragment thereof according to aspect 41 wherein the at least one VL region comprises the sequence of SEQ ID NO: 50 or ,184, 60, 62 or 64 or functionally equivalent variants thereof and the at least one VH region comprises the sequence of SEQ ID NO: 51, 59, 61 and 63 or functionally equivalent variants thereof.
43. The antibody or antibody fragment thereof according to aspect 42 wherein, the antibody fragment is an ScFv, and the VH and VL regions are connected by a linker region comprising SEQ ID NO: 29.
44. The antibody or antibody fragment thereof according to aspect 43, wherein the ScFv comprises the sequence of SEQ ID NO: 52,186, 190, 191 or 192 or functionally equivalent variants thereof.
45. A nucleic acid encoding an ScFv, antigen-binding domain or antibody according to any of aspects 30 to 44.
46. An expression vector comprising the nucleic acid of aspect 45.
47. A host cell comprising the nucleic acid of aspect 45 or the expression vector of aspect 46.
48. An antibody-drug conjugate (ADC) comprising:
   - a therapeutic agent, and
   - an antibody or antigen-binding domain specific for p95HER2 selected from the group consisting of:
      (i) an antibody or antibody fragment thereof, characterized in that:
         - it has at least one VH region and one VL region,
         - the CDR1, CDR2 and CDR3 of the at least one VH region comprise, respectively, the sequences of SEQ ID NO: 1, 2 and 3 or functionally equivalent variants thereof or the sequences SEQ ID NO: 1, 174 and 3 or functionally equivalent variants thereof, and
         - the CDR1, CDR2 and CDR3 of the at least one VL region comprise respectively, the sequences of SEQ ID NO: 4, 5, and 6 or functionally equivalent variants thereof or the sequences of SEQ ID NO: 175, 5 and 6 or functionally equivalent variants thereof,
      (ii) an antibody or antibody fragment thereof, characterized in that,
         - it has at least one VH region and one VL region,
         - the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
         - the CDR1, CDR2 and CDR3 of the at least one VL region comprise respectively, the sequences of SEQ ID NO: 10, 11, and 12 or functionally equivalent variants thereof, and
      (iii) an antibody or antibody fragment thereof, characterized in that:
         - it has at least one VH region and at least one VL region,
         - the CDR1, CDR2 and CDR3 of the at least one VH region comprise, respectively, the sequences of SEQ ID NO: 13, 14 and 15 or functionally equivalent variants thereof, and
         - the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.
49. The ADC according to aspect 48(i), wherein FR1, FR2, FR3 and FR4 of the at least one VH region comprise respectively the sequences SEQ ID NOs: 152, 153, 154 and 155, SEQ ID NO: 19, 20, 21 and 22 or SEQ ID NO: 163, 164, 165 and 166 or functionally equivalent variants thereof and the FR1, FR2, FR3 and FR4 of the at least one VL region comprise respectively the sequences SEQ ID NOs: 156, 157, 158 and 159, SEQ ID NO: 23, 24, 25 and 26 or SEQ ID NO: 167, 168, 169 or 170 or functionally equivalent variants thereof.
50. The ADC according to aspect 49, wherein the VL comprises the sequence SEQ ID NO: 160,193, 27, 171 or 180 or a functionally equivalent variant thereof and the VH comprises the sequence SEQ ID NO: 161, 194, 28, 172 or 181 of a functionally equivalent variants thereof.
51. The ADC according to aspect 50, wherein the antibody or antibody fragment thereof is an ScFv, and wherein VH and VL regions of the ScFv are connected by a linker regions comprising the sequence SEQ ID NO: 29.
52. The ADC according to aspect 51, wherein the ScFv1 comprises the sequence of SEQ ID NO: 162,195, 30, 173 or 182 or a functionally equivalent variants thereof.
53. The ADC according to aspect 48 (ii), wherein the FR1, FR2, FR3 and FR4 or the at least one VH region comprise respectively the sequences SEQ ID NOs: 31, 32, 33 and 34, or SEQ ID NO: 65, 66, 67 and 68, SEQ ID NO: 73, 74, 75 and 76 or SEQ ID NO: 81, 82, 83 and 84 or functionally equivalent variants thereof and the FR1, FR2, FR3 and FR4 of the at least one VL region comprise respectively the sequences SEQ ID NOs: 35, 36, 37 and 98 38, SEQ ID NO: 69, 70, 71, 72, SEQ ID NO: 77, 78, 79 and 80 or SEQ ID NO: 85, 86, 87 and 88 or functionally equivalent variants thereof.
54. The ADC according to aspect 53, wherein the VL comprises the sequence SEQ ID NO: 39, 54, 56 and 58 or a functionally equivalent variant thereof and the VH comprises the sequence SEQ ID NO: 40,183 53, 55 and 57 or a functionally equivalent equivalent variants thereof.
55. The ADC according to aspect 54, wherein the antibody or antibody fragment is an ScFv, and wherein VH and VL regions of the ScFv are connected by a linker region comprising the sequence SEQ ID NO: 29.
56. The ADC according to aspect 55, wherein the ScFv comprises the sequence of SEQ ID NO: 41,185, 187, 188 or 189 or a functionally equivalent variants thereof.
57. The ADC according to aspect 48(iii), wherein the FR1, FR2, FR3 and FR4 or the at least one VH region comprise respectively the sequences SEQ ID NOs: 42, 43, 44 and 45, SEQ ID NO: 89, 90, 91 and 92, SEQ ID NO:97, 98, 99 and 100 or SEQ ID NO: 105, 106, 107 and 108 or functionally equivalent variants thereof and the FR1, FR2, FR3 and FR4 of the at least one VL region comprise respectively the sequences SEQ ID NOs: 46, 47, 48 and 49, SEQ ID NO: 93, 94, 95 and 96, SEQ ID NO: 101, 102, 103 and 104 or SEQ ID NO: 109, 110, 11 and 112 or functionally equivalent variants thereof.
58. The ADC according to aspect 56, wherein the VL comprises the sequence SEQ ID NO: 50,184, 60, 62 or 64 or a functionally equivalent variant thereof and the VH comprises the sequence SEQ ID NO: 51, 59, 61 and 63 or a functionally equivalent thereof.
59. The ADC according to aspect 58, wherein the antibody or antibody fragment is an ScFv, and wherein VH and VL regions of the ScFv are connected by a linker region comprising the sequence SEQ ID NO: 29.
60. The ADC according to aspect 59, wherein the ScFv comprises the sequence of SEQ ID NO: 52,186, 190, 191 or 192 or a functionally equivalent variants thereof.
61. The ADC according to any of aspects 48 to 60, wherein the therapeutic agent is a cytotoxic agent.
62. A method of cancer diagnosis in a patient which comprises:
   (i) contacting a sample of the patient containing tumor cells with an ScFv, and antigen-binding domain or antibody according to any of aspects 30 to 44 and
   (ii) detecting the binding of the ScFv, antigen-binding domain or antibody to cells in the sample
   wherein the presence of binding is indicative that the patient suffers from cancer.
63. The method according to aspect 62 wherein the ScFv1, antigen-binding domain or antibody are arranged on a solid support.
64. A pharmaceutical composition comprising the host cell of any of aspects 26 to 29, an ScFv, an antigen-binding domain or antibody according to any of aspects 30 to 44 or the ADC according to aspect 48 and at least one pharmaceutically acceptable excipient and/or vehicle.
65. The host cell of any of aspects 26 to 29, an ScFv, antigen-binding domain or antibody according to any of aspects 30 to 44, or the ADC according to aspect 48 for use in medicine.
66. The host cell of any of aspects 26 to 29, an ScFv, antigen-binding domain or antibody according to any of aspects 30 to 44 or ADC according to aspect 48 for use in a method of preventing or treating cancer.
67. The host cell, ScFv, antigen-binding domain, antibody or ADC for use of aspect 66 wherein the cancer is p95HER2 positive.

The invention will be described by way of the following examples which are to be considered as merely illustrative and not limitative of the scope of the invention.

### EXAMPLES

### Methodology

### CAR vector design and production

Vector plasmids coding for p95HER2 CARs (h32H2, 214D8, H1 214D8, H2 214D8, H3 214D8, 215C2, H1 215C2, H2 215C2) were synthesized and cloned into pMSGV-1 retroviral vector (Genscript, Netherlands). Then, stocks of p95HER2 CARs, HER2 CAR and Empty (UTD) CAR retrovirus were produced. Briefly, 0,7 µg of envelope plasmid (RD-114) and 1.5 µg of transfer plasmid (p95HER2, HER2, Empty CARs in pMSGV-1) were co-transfected in GP-293 cells (#631458, Clontech). After 2 and 3 days, cell supernatant containing retrovirus particles was collected and store at -80C for future transductions.

### Transduction and expansion of CAR T cells

PBMCs were stimulated with 10 ng/ul of α-CD3 (OKT3) (#16-0037-85, Thermo-Fisher) and 300 U/ml IL-2 (#703892-4, Novartis) for 48 hours before transduction. Then, cell supernatant containing retroviral particles was thawed and centrifuge in retronectin (#T100A, Takara)-coated 6-well plates for 2 hours at 2000g. Next, 2 x 10⁶ stimulated PBMCs were added on top and centrifuged for 10 minutes at 400g. After 5 days, CAR expression and cytotoxic assays were performed. Untransduced T cells (UTD) were transduced with empty CAR retrovirus.

### CAR expression analysis

0,2 x 10⁶ CAR Ts were washed twice with 1xPBS and re-suspended in 1xPBS, 2.5 mM EDTA, 1% BSA, and 5% horse serum for 20 minutes. Then, cells were stained with 1/20 Biotin anti-IgG (#115-065-072, Jackson ImmunoResearch) for 30 minutes and washed twice with 1xPBS. APC-Streptavidin antibody (#405207, Biolegend) at 1/150 and 1/300 anti-CD3-PE (#300408, Biolegend) were added for 30 minutes. Zombie Aqua (#423101, Biolegend) was used as a viability marker at 1:1000 dilution. CAR expression was measured on FACSCelesta (BD Bioscience) and analyzed with FlowJo software.

### CAR T cytotoxic assay

CFSE-labelled MCF10A p95HER2/empty cells were co-cultured with CAR T cells at the indicated E: CAR T ratio in 96-well flat bottom plates. After 48 hours of incubation, the mixture of cells was washed with 1xPBS and re-suspended in 1xPBS, 2.5 mM EDTA, 1% BSA, and 5% horse serum for 20 minutes. Then, cells were stained with zombie Aqua (#423101, Biolegend) at 1:1000 dilution as a viability marker. CFSE positive cells were counted on LSR Fortessa (BD Bioscience) and analysed with FlowJo software.

### In vivo models

NSG mice were injected orthotopically with 3 x 10⁶ MCF7p95HER2/parental cells. Once tumour volume reached 300 mm³, animals were intravenously (i.v.) treated with 3 x 10⁶ CAR+ T cells every 7-10 days a maximum of 4 doses. In the case of MCF7p95HER2 cells, mice were maintained in the presence of doxycycline (1g/L) in the drinking water.

### Results

### Example 1: Humanized 32H2 p95HER2 CAR

The anti-p95HER2 antibody 32H2 has been disclosed in PCT application published as WO/2010/000565. Initially, the single chain fragment variable (scFv) of the anti-p95HER2 antibody 32H2 was used to generate two versions of 32H2-devided p95HER2 CAR.

The two versions of 32H2 p95HER2 CAR differed in the order of arrangement of the light variable region (VL) and the heavy variable region (VH) of the single chain fragment variable (scFv) of the 32H2 antibody (Figure 2A). Both 32H2 p95HER2 CARs contained a CD8 leader sequence (MALPVTALLLPLALLLHAARP SEQ ID NO: 147) at the beginning of the CAR sequence, a linker (TGSTSGSGKPGSGEGS SEQ ID NO: 29) between the variable regions, a CD8 hinge domain, a CD28 transmembrane and costimulatory domain and a CD3 zeta domain. A trastuzumab-based CAR targeting full-length HER2 was used as a positive control (Figure 2A).

None of the 32H2 p95HER2 CARs generated were detected in the cell surface (Figure 2B). Accordingly, both 32H2 p95HER2 CAR Ts were not functional, showed by the lack of killing of MCF10A cells expressing p95HER2 (Figure 2C).

The scFv of 32H2 was humanized resulting in two humanized versions of VH and VL, depending on the grade of humanization (Table 1).

**Table 1: Amino acid sequence of the heavy and light variable regions of different humanized 32H2 versions H1: Humanized version 1; H2: Humanized version 2.**

| | **V_{H}** | **V_{L}** |
|---|---|---|
| **H1 32H2** | EVQLVESGGGLVQPGGSLRLSCAASGFTF NDFGMSWVRQAPGKGLEWVSTINTNGGTT HYPDSVKGRFTISRDNSKNTLYLQMNSLRA EDTAVYYCAKEGLDYWGQGTLVTVSS (SEQ ID NO: 28) | EIVLTQSPATLSLSPGERATLSCRASQSVGTA VAWYQQKPGQAPRLLIYSASNRFTGIPARFS GSGSGTDFTLTISSLEPEDFAVYYCQQYSTYP LAFGQGTKLEIK (SEQ ID NO: 27) |
| **H2 32H2** | EVQLVESGGGLVQPGGSLRLSCAASGFTF NDFGMSWVRQAPGKGLEWVSTINTNGGTT HYPDSVKGRFTISRDNSKKFVYLQMNSLRA EDTAVYYCPREGLDYWGQGTLVTVSS (SEQ ID NO: 171) | EIVLTQSPATLSLSPGERATLSCRASQSVGTA VAWYQLKPGQAPRLLIYSASNRFTGIPARFSG SGSGTDFTLTISSLEPEDFAVYYCQQYSTYPL AFGQGTKLEIK (SEQ ID NO: 172) |
| **32H2** | EVQLVESGGGIVQPGGSLKLSCAASGFTFN DFGMSWIRQTPDKRLELVATINTNGGTTHY PDNVKGRFSISRDNAKKFVYLQMSSLKSDD TAIYYCPREGLDYWGQGTTLTVSS (SEQ ID NO: 161) | DIVLTQSQKFMSTSVGDRVSIICKASQSVGTA VAWYQLKAGQSPKLLIYSASNRFTGVPDRFT GSGSGTDFTLTISNVQSEDLADYFCQQYSTY PLAFGAGTKLELK (SEQ ID NO: 160) |

Four versions of humanized 32H2 p95HER2 CAR were generated, differing in the order of arrangement of the light variable region (VL) and the heavy variable region (VH) and the humanized version used (H1 or H2). The four humanized 32H2 p95HER2 CARs contained a CD8 leader sequence at the beginning of the CAR sequence, a linker between the variable regions, a CD8 hinge domain, a CD28 transmembrane and costimulatory domain and a CD3 zeta domain. (Figure 3A). VL-VH H1 32H2 p95HER2 CAR was expressed at the cell surface (Figure 3B, 4B) in contrast to the rest of humanized 32H2 p95HER2 CARs versions (Figure 3B). Thus, VL-VH H1 32H2 p95HER2 CAR was used for further experiments and named as humanized 32H2 (h32H2) p95HER2 CAR.

In additional experiments, h32H2 p95HER2 could be expressed at the cell surface at similar levels as trastuzumab-based CAR (Figure 4B). Moreover, h32H2 p95HER2 CAR Ts co-cultured with MCF10A cells expressing p95HER2 induced a specific cytotoxic effect (Figure 4C) although the efficacy was evident at high ratios of Target: CAR T cells. In contrast, h32H2 p95HER2 CAR Ts did not have any effect on MCF10A cells (Figure 4D), suggesting its specificity for p95HER2.

### Example 2: 214D8 p95HER2 CAR

214D8 p95HER2 CARs were generated from the scFv of the anti-p95HER2 antibody 214D8 which has been disclosed in US patent application published as US2011/0135653, the contents of which are hereby incorporated by reference in their entirety.

Two versions of 214D8 p95HER2 CAR were developed, differing in the order of arrangement of the light variable region (VL) and the heavy variable region (VH) of 214D8 antibody (Table 2, Figure 5A). Both 214D8 p95HER2 CARs contained a CD8 leader sequence, a linker, a CD8 hinge domain, a CD28 transmembrane and costimulatory domain and a CD3 zeta domain.

Both 214D8 p95HER2 CARs were expressed at the cell surface, being VL-VH 214D8 p95HER2 CAR expressed at higher levels (Figure 5B). VL-VH 214D8 p95HER2 CAR Ts co-cultured with MCF10A cells expressing p95HER2 induced a high cytotoxic effect even at low ratios of Target: CAR T cells (Figure 5C).

**Table 2: Amino acid sequence of the heavy and light variable regions of 214D8 anti-p95HER2 antibody.**

| | **V_{H}** | **V_{L}** |
|---|---|---|
| **214D8** | EVQLVESGGGLVQPGGSLKLSCAASGFT FSTYGMAWVRQTPDRRLELVATINSNGG KTYHPDSVKGRFTISRDNAKNTLYLQMSS LKSEDTAMYYCTREGFDYWGQGTTLTVS S (SEQ ID NO: 40) | DIVMTQSQKFMSTSVGDRVSITCKASQNVG TAVAWYQQKPGQSPKILIYSASNRYTGVPD RFTGSGSGTDFTLTISNMKSEDLADYFCQQ YSTYPLTFGAGTKLELK (SEQ ID NO: 39) |

Humanized version of the heavy and light variable regions of the 214 anti-p95HER2 have also been obtained, as shown in Table 3.

**Table 3: Amino acid sequence of the heavy and light variable regions of different humanized 214 anti-p95HER2 versions.**

| | **V_{H}** | **V_{L}** |
|---|---|---|
| **H1 214D8** | EVQLVESGGGLVQPGGSLRLSCAASGF TFSTYGMAWVRQTPDRRLELVATINSN GGKTYHPDSVKGRFTISRDNSKNTLYL QMNSLRAEDTAVYYCTREGFDYWGQG TLVTVSS (SEQ ID NO: 53 | DIQMTQSPSSLSASVGDRVTITCKASQNVGTAVAWF QQKPGKAPKILIYSASNRYTGVPSRFSGSGSGTDFTL TISSLQPEDFATYYCQQYSTYPLTFGQGTKLEIK (SEQ ID NO: 54) |
| **H2 214D8** | EVQLVESGGGLVQPGGSLRLSCAASGF TFSTYGMAWVRQAPGKGLEWVSTINS NGGKTYHPDSVKGRFTISRDNSKNTLY LQMNSLRAEDTAVYYCTREGFDYWGQ GTLVTVSS (SEQ ID NO: 55) | DIQMTQSPSSLSASVGDRVTITCKASQNVGTAVAWF QQKPGKAPKILIYSASNRYTGVPSRFSGSGSGTDFTL TISSLQPEDFATYYCQQYSTYPLTFGQGTKLEIK (SEQ ID NO: 56) |
| **H3 214D8** | EVQLVESGGGLVQPGGSLRLSCAASGF TFSSYGMAWVRQAPGKGLEWVSAINS NGGKTYYADSVKGRFTISRDNSKNTLYL QMNSLRAEDTAVYYCAKDYWGQGTLV TVSS (SEQ ID NO: 57) | DIQMTQSPSSLSASVGDRVTITCKASQNVGTAVAWF QQKPGKAPKILIYSASNRYTGVPSRFSGSGSGTDFTL TISSLQPEDFATYYCQQYSTYPLTFGQGTKLEIK (SEQ ID NO: 58) |

Humanized 214 anti-p95HER2 CAR versions were expressed at the cell surface (Figure 6B), and at least H1 214 and H2 214 humanized CAR versions induced a high cytotoxic effect even at low ratios of Target: CAR T cells (Figure 6D). In addition, as shown in figure 6C, the use of humanized ScFv versions generates CAR Ts more specific for p95HER2 due to the decrease in the killing of cells expressing normal levels of HER2, compared with the non-humanized versions..

### Example 3: 215C2 p95HER2 CAR

215C2 p95HER2 CARs were generated from the ScFv of the anti-p95HER2 antibody 215C2.

Two versions of 215C2 p95HER2 CAR were developed, differing in the order of arrangement of the light variable region (VL) and the heavy variable region (VH) of 215C2 antibody (Table 4, Figure 7A). Both 215C2 p95HER2 CARs contained a CD8 leader sequence, a linker, a CD8 hinge domain, a CD28 transmembrane and costimulatory domain and a CD3 zeta domain.

Both 215C2 p95HER2 CARs were expressed at the cell surface, being VL-VH 215C2 p95HER2 CAR expressed at higher levels (Figure 7B). Furthermore, VL-VH 215C2 p95HER2 CAR Ts co-cultured with MCF10A cells expressing p95HER2 induced a high cytotoxic effect at low ratios of Target: CAR T cells (Figure 7C).

**Table 4: Amino acid sequence of the heavy and light variable regions of 215C2 anti-p95HER2 antibody**

| | **V_{H}** | **V_{L}** |
|---|---|---|
| **215C2** | EVHLVESGGGLVQPGGSLKLSCAASGFTF SDYGMSWVRQTPDKRLELVATINGNGVKI YYVDSVKGRFTISRDNAKNTLYLQMNSLK SEDTAIYYCPREGFDYWGQGTTLTVSS (SEQ ID NO: 51) | DIVMTQSQKFMSTSEGDRVSITCKASQNVGTAV AWYQQKPGQSPKVLIYSASNRYTGVPDRFTGS GSGTDFTLTISNMQSEDLADYFCQQYSSYPLTF GAGTTLELK (SEQ ID NO: 50) |

Humanized version of the heavy and light variable regions of the 215 anti-p95HER2 have also been obtained, as shown in Table 5

**Table 5: Amino acid sequence of the heavy and light variable regions of different humanized 215 anti-p95HER2 versions.**

| | V_{H} | V_{L} |
|---|---|---|
| **H1 215C2** | EVQLVESGGGLVQPGGSLRLSCAASG FTFSDYGMSWVRQAPGKRLELVATING NGVKIYYVDSVKGRFTISRDNSKNTLYL QMNSLRAEDTAVYYCPREGFDYWGQ GTLVTVSS (SEQ ID NO: 59) | DIQMTQSPSSLSASVGDRVTITCKASQNVGTAV AWFQQKPGKAPKVLIYSASNRYTGVPSRFSGS GSGTDFTLTISSLQPEDFATYYCQQYSSYPLTF GQGTKLEIK (SEQ ID NO: 60) |
| **H2 215C2** | EVQLVESGGGLVQPGGSLRLSCAASG FTFSDYGMSWVRQAPGKGLEWVATIN GNGVKIYYVDSVKGRFTISRDNSKNTLY LQMNSLRAEDTAVYYCPREGFDYWGQ GTLVTVSS (SEQ ID NO: 61 ) | DIQMTQSPSSLSASVGDRVTITCRASQNVGTAV AWFQQKPGKAPKSLIYSASNRYTGVPSRFSGS GSGTDFTLTISSLQPEDFATYYCQQYSSYPLTF GQGTKLEIK SEQ ID NO: 62) |
| **H3 215C2** | EVQLVESGGGLVQPGGSLRLSCAASG FTFSDYGMSWVRQAPGKGLEWVATIN GNGVKTYYADSVKGRFTISRDNSKNTL YLQMNSLRAEDTAVYYCPR (SEQ ID NO: 63) | DIQMTQSPSSLSASVGDRVTITCRASQNVGTAV AWFQQKPGKAPKSLIYSASNRQSGVPSRFSGS GSGTDFTLTISSLQPEDFATYYCQQYSSYPLTF GQGTKLEIK (SEQ ID NO: 64) |

Humanized 215 anti-p95HER2 CAR versions H1 and H2 were expressed at the cell surface (Figure 8B), which aslo induced a high cytotoxic effect even at low ratios of Target: CAR T cells (Figure 8C). In addition, as shown in figure 8D, the use of humanized ScFv versions generates CAR Ts more specific for p95HER2 due to the decrease in the killing of cells expressing normal levels of HER2, compared with the non-humanized versions..

### Example 4: Effect of m215-derived p95HER2 CAR T on the growth of p95HER2-positive tumours in vivo.

NSG mice were orthotopic implanted with MCF7p95HER2 cells. When tumors reached approximately 300 mm³, animals were treated with 3 x 10⁶ m215-derived p95HER2 CAR+ T cells or UTD T cells. A complete remission of the tumor was achieved after three rounds of CAR + T cell treatment (Figure 9A). Moreover, circulating human CD3+ cells were detected after thirty-five days of treatment, suggesting a proper CAR T persistence.

## Claims

1. A chimeric antigen receptor (CAR) comprising:
(i) an antigen-binding domain specific for p95HER2,
(ii) a transmembrane domain and
(iii) at least one intracellular signaling domain and/or costimulatory domain
wherein the antigen-binding domain is selected from the group consisting of:
(i) an ScFv (ScFv1) **characterized in that**:
- the framework regions of the VL and VH regions are humanized,
- the VL region is located N-terminally with respect to the VH region,
- the CDR1, CDR2 and CDR3 of the VH region comprise, respectively, the sequences of SEQ ID NO:1, 2 and 3 or functionally equivalent variants thereof or the sequences of SEQ ID NO: 1, 174 and 3 or functionally equivalent variants thereof, and
- the CDR1, CDR2 and CDR3 of the VL region comprise respectively, the sequences of SEQ ID NO: 4, 5, and 6 or functionally equivalent variants thereof or the sequences of SEQ ID NO: 175, 5 and 6 or functionally equivalent variants thereof,
(ii) an antigen-binding domain (antigen-binding domain 1), **characterized in that**:
- it has at least one VH region and at least one VL region,
- the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
- the CDR1, CDR2 and CDR3 of the at least one VL region comprise respectively, the sequences of SEQ ID NO: 10, 11, and 12 or functionally equivalent variants thereof,
(iii) an antigen-binding domain (antigen-binding domain 2), **characterized in that**:
- it has at least one VH region and at least one VL region,
- the CDR1, CDR2 and CDR3 of the at least one VH region comprise, respectively, the sequences of SEQ ID NO: 13, 14 and 15 or functionally equivalent variants thereof,
- and the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.

2. The CAR of claim 1 wherein the FR1, FR2, FR2 and FR4 of the VH region of the ScFv1 comprise respectively the sequences of SEQ ID NO: 19, 20, 21 and 22 or functionally equivalent variants thereof and FR1, FR2, FR3 and FR4 of the VL region of the ScFv1 comprise respectively the sequences of SEQ ID NO: 23, 24, 25 and 26 or functionally equivalent variants thereof.

3. The CAR of claim 2 wherein the VL of ScFv1 comprises the sequence of SEQ ID NO: 27 or 180 or functionally equivalent variants thereof and the VH comprises the sequence of SEQ ID NO: 28 or 181 or functionally equivalent vatriants thereof.

4. The CAR of claim 3 wherein the ScFv1 comprises the sequence of SEQ ID NO: 30 or 182 or functionally equivalent variants thereof.

5. The CAR of claim 1 wherein the at least one VL region of the antigen-binding domain 1 comprises the sequence of SEQ ID NO: 39, 54, 56 or 58 or functionally equivalent variants thereof and the at least one VH region of the antigen-binding domain 1 comprises the sequence of SEQ ID NO: 40, 183, 53, 55 or 57 or functionally equivalent variants thereof,

6. The CAR of claim 5 wherein the antigen-binding domain 1 is an ScFv, and wherein the ScFv comprises the sequence of SEQ ID NO: 41, 185, 187, 188 or 189 or functionally equivalent variants thereof.

7. The CAR of claim 1, wherein the at least one VL region of the antigen-binding domain 2 comprises the sequence of SEQ ID NO: 50,184, 60, 62 or 64 or functionally equivalent variants thereof and the at least one VH of the antigen-binding domain 2 comprises the sequence of SEQ ID NO: 51, 59, 61 and 63. or functionally equivalent variants thereof.

8. The CAR of claim 7, wherein the antigen-binding domain 2 is an ScFv, and wherein the ScFv comprises the sequence of SEQ ID NO: 52, 186, 190, 191 or 192 or functionally equivalent variants thereof.

9. A nucleic acid encoding a CAR according to any of claims 1 to 8.

10. A host cell comprising the chimeric antigen receptor (CAR) of any of claims 1 to 8 or the nucleic acid of any of claim 9.

11. An antibody or an antibody fragment thereof **characterized in that**,
- at least one VH region and at least one VL region,
- the CDR1, CDR2 and CDR3 of the at least one VH region comprise, respectively, the sequences of SEQ ID NO: 13, 14 and 15 or functionally equivalent variants thereof, and
- the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.

12. The antibody or antibody fragment thereof according to claim 11 wherein the at least one VL region comprises the sequence of SEQ ID NO: 50,184, 60, 62 or 64 or functionally equivalent variants thereof and the at least one VH region comprises the sequence of SEQ ID NO: 51, 59, 61 and 63 or functionally equivalent variants thereof.

13. The antibody or antibody fragment thereof according to claim 11, wherein the antibody fragment is an ScFv, and wherein the ScFv comprises the sequence of SEQ ID NO: 52,186, 190, 191 or 192 or functionally equivalent variants thereof.

14. An antibody-drug conjugate (ADC) comprising:
- a therapeutic agent, and
- an antibody or antigen-binding domain specific for p95HER2 selected from the group consisting of:
(i) an antibody or antibody fragment thereof, **characterized in that**:
- it has at least one VH region and one VL region,
- the CDR1, CDR2 and CDR3 of the at least one VH region comprise, respectively, the sequences of SEQ ID NO: 1, 2 and 3 or functionally equivalent variants thereof or the sequences SEQ ID NO: 1, 174 and 3 or functionally equivalent variants thereof, and
- the CDR1, CDR2 and CDR3 of the at least one VL region comprise respectively, the sequences of SEQ ID NO: 4, 5, and 6 or functionally equivalent variants thereof or the sequences of SEQ ID NO: 175, 5 and 6 or functionally equivalent variants thereof,
(ii) an antibody or antibody fragment thereof, **characterized in that**,
- it has at least one VH region and one VL region,
- the CDR1, CDR2 and CDR3 of the at least one VH region comprise, respectively, the sequences of SEQ ID NO: 7, 8 and 9 or SEQ ID NO: 176, 177 and DY or functionally equivalent variants thereof, and
- the CDR1, CDR2 and CDR3 of the at least one VL region comprise respectively, the sequences of SEQ ID NO: 10, 11, and 12 or functionally equivalent variants thereof, and
(iii) an antibody or antibody fragment thereof, **characterized in that**:
- it has at least one VH region and at least one VL region,
- the CDR1, CDR2 and CDR3 of the at least one VH region comprise, respectively, the sequences of SEQ ID NO: 13, 14 and 15 or functionally equivalent variants thereof, and
- the CDR1, CDR2 and CDR3 of the at least one VL region comprise respectively, the sequences of SEQ ID NO: 16, 17, and 18 or functionally equivalent variants thereof or the sequences of SEQ ID NO: 179, 17 and 18 or functionally equivalent variants thereof.

15. A pharmaceutical composition comprising the host cell of claim 10, an antibody or antibody fragment according to claim 11 or the ADC according to claim 14 and at least one pharmaceutically acceptable excipient and/or vehicle.

16. The host cell of claim 10, the antibody fragment according to claim 11 or the ADC according to claim 14 for use in a method of preventing or treating cancer.
